# EUROPEAN PATENT SPECIFICATION

(11) **EP 4 373 805 B1**
(45) Date of publication and mention of the grant of the patent: **09.04.2025**
(21) Application number: 23725150.9
(22) Date of filing: 04.05.2023
(51) Int. Cl.: C07C 323/22, C07D 307/82, C07D 309/04, C07D 319/06, C11B 9/00

(54) **PRECURSORS FOR THE CONTROLLED RELEASE OF ALPHA, BETA-UNSATURATED ALDEHYDES OR KETONES AND OTHER HIGH IMPACT FRAGRANCE SUBSTANCES**
VORLÄUFER ZUR KONTROLLIERTEN FREISETZUNG VON ALPHA,BETA-UNGESÄTTIGTEN ALDEHYDEN ODER KETONEN UND ANDEREN HOCHSCHLAGFESTEN DUFTSTOFFEN
PRÉCURSEURS POUR LA LIBÉRATION CONTRÔLÉE D'ALDÉHYDES OU DE CÉTONES ALPHA,BETA-INSATURÉS ET D'AUTRES SUBSTANCES DE PARFUM À IMPACT ÉLEVÉ

(30) Priority: 04.05.2022 WO PCT/EP2022/062021
(43) Date of publication of application: 29.05.2024
(73) Proprietor: Symrise AG, 37603 Holzminden (DE)
(72) Inventor: GERSTMANN, Frank, 60599 Frankfurt am Main (DE); HÖLSCHER, Bernd, 37620 Halle (DE); JUNGE, Thorsten, 37603 Holzminden (DE); EILERS, Jörg, 37603 Holzminden (DE); KEPLER-BOZKURT, Lucie, 37603 Holzminden (DE); KORTE, Stephanie, 37671 Höxter (DE); VOM ENDE, Marc, 13585 Berlin (DE); ELTERLEIN, Franziska, 37603 Holzminden (DE); KRAFT, Philip, 37603 Holzminden (DE)
(74) Representative: Eisenführ Speiser
(86) International application number: PCT/EP2023/061751
(87) International publication number: WO 2023/213922

(56) References cited:
- EP-A1- 3 533 786
- WO-A1-2015/032885
- WO-A1-2019/037862
- WO-A1-2021/123144
- US-A1- 2009 298 807
- SELL C S: "On the Unpredictability of Odor", ANGEWANDTE CHEMIE INTERNATIONAL EDITION, VERLAG CHEMIE, HOBOKEN, USA, vol. 45, no. 38, 25 September 2006 (2006-09-25), pages 6254 - 6261, XP007911257, ISSN: 1433-7851, [retrieved on 20060919], DOI: 10.1002/ANIE.200600782
- BÄTTIG SARAH ET AL: "Cassis and Green Tea: Spontaneous Release of Natural Aroma Compounds from [beta]-Alkylthioalkanones", vol. 104, no. 11, 1 November 2021 (2021-11-01), Hoboken, USA, pages 1 - 10, XP093013525, ISSN: 0018-019X, Retrieved from the Internet <URL:https://onlinelibrary.wiley.com/doi/full-xml/10.1002/hlca.202100135> DOI: 10.1002/hlca.202100135
- UMBERTO MADDALENA ET AL: "Thioether Profragrances: Parameters Influencing the Performance of Precursor-Based Fragrance Delivery in Functional Perfumery", CHEMISTRY & BIODIVERSITY, vol. 11, no. 11, 1 November 2014 (2014-11-01), CH, pages 1700 - 1733, XP055502263, ISSN: 1612-1872, DOI: 10.1002/cbdv.201400023

## Description

The present invention primarily relates to compounds of formula (I) as defined herein. It also relates to fragrance substance mixtures as defined herein and methods for producing the same and to perfumed products as defined herein and methods for producing the same. The present invention further relates to the use of a compound of formula (I) as defined herein as a precursor for the release of two or more different pleasantly smelling fragrance substances, to methods for the release of two or more different pleasantly smelling fragrance substances and to thiols as defined herein.

Further aspects and preferred embodiments of the present invention are apparent from the following description, the attached examples and, in particular, the attached patent claims.

On the part of the perfume industry, there is a particular interest in a controlled and long-lasting release of certain fragrances. This applies in particular to fragrances with a high volatility or low substantivity. The washing of textiles, for example, is an area where there is a great desire to be able to perceive the pleasant odours of the fragrance substances or perfume oils used in detergents or fabric softeners on the laundry for a long period of time after washing and drying of the textiles.

Precursors are known in the perfume industry and are formed, for example, from α,β-unsaturated carbonyl compounds and thiols by way of Michael additions. They usually release a pleasantly smelling α,β-unsaturated carbonyl compound, such as an α,β-unsaturated aldehyde or ketone. This release takes place in a controlled manner and thus enables a sensorial perception of highly volatile fragrance substances that would not be achievable if the aldehyde or ketone compounds were used directly in a perfume oil.

There is a great demand in the perfume industry for precursors that are capable of releasing several fragrance substances simultaneously or over time. This would allow an expansion of the perfumery palette. There is also a particularly high demand for fragrance substances that have a strong olfactory effect even at low dosages (i.e. a low odorant threshold), so-called "high impact" fragrance substances.

The known precursors are limited to the release of fragrant α,β-unsaturated carbonyl compounds. Due to the unpleasant odour impression of common, especially short-chain, thiol compounds, the prior art teaches the use of thiol compounds that are as odourless as possible for the formation of precursors. In patent application WO 2004/105713 A1, for example, 3-mercaptopropyltriethoxysilanes and 3-mercaptopropyltrimethoxysilanes are reacted with α,β-unsaturated carbonyl compounds to form precursors. Only the released α,β-unsaturated carbonyl compounds serve as fragrance substances in the teaching described therein.

In patent application WO 2015/032885 A1, short-chain thiol compounds are used as thiol compounds to form the precursors. Also in this publication, only the α,β-unsaturated carbonyl compounds released from the precursors serve as fragrance substances. It is described that the unpleasant odour of the short-chain thiol compounds, which are also released from the precursors, surprisingly does not affect the positive olfactory effect of the α,β-unsaturated carbonyl compounds too negatively, so that overall, unexpectedly, the positive olfactory impression of the released α,β-unsaturated carbonyl compounds dominates.

EP 3 533 786 A1 discloses the use of a compound of formula (I), a composition of matter comprising a compound of formula (I) and a fragrance ingredient comprising a compound of formula (I) 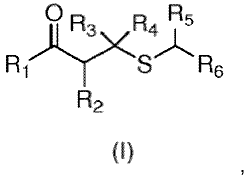 wherein *inter alia* R₅ is defined as H or a residue comprising 1 to 20 carbon atoms and R₆ is defined as a residue comprising 3 to 20 carbon atoms.

WO 2021/123144 A1 relates to a perfuming composition comprising at least two pro-perfume compounds. It further concerns a perfumed consumer product comprising the perfuming composition described therein, as well as the use of the perfuming composition for improving, enhancing, conferring and/or modifying the fragrance impression and/or fragrance intensity of a consumer product.

S. Bättig et al., Helv. Chim. Acta 2021, 104, e2100135 relates to the spontaneous release of natural aroma compounds from specific β-alkylthioalkanones.

U. Maddalena et al., Chemistry & Biodiversity 2014, 11, 1700 relates to specific thioether profragrances and the parameters influencing the performance of precursor-based fragrance delivery in functional perfumery.

The primary task was to provide improved precursors that meet the above requirements, i.e. are capable of releasing pleasantly smelling, high impact fragrances in a controlled manner and over a long period of time. This release should preferably be possible from surfaces treated with the precursors, especially from textiles.

Surprisingly, this task is solved by a compound of formula (I) 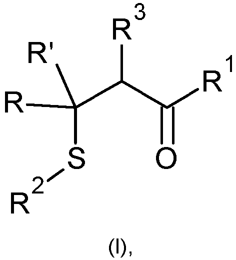
(i) wherein R is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
   wherein R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
   wherein R¹ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
   wherein R² is selected from the group consisting of 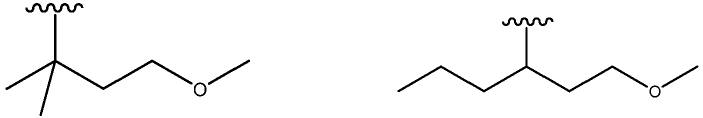 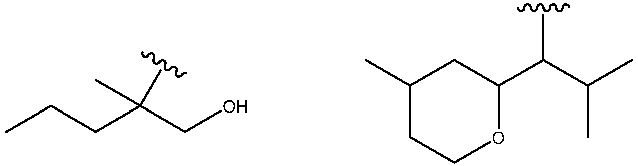 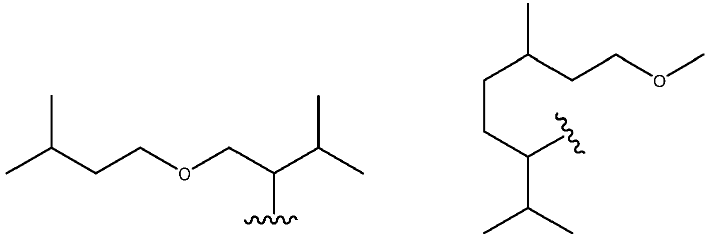 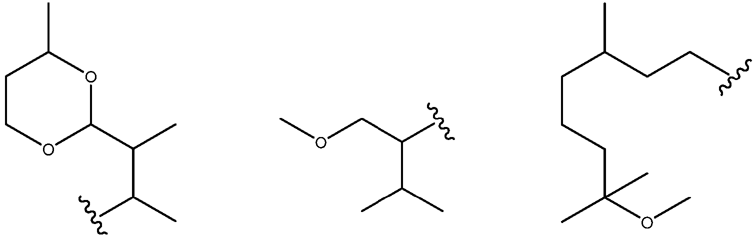 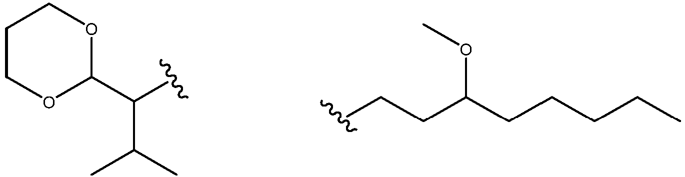 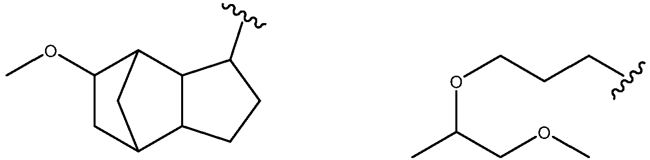 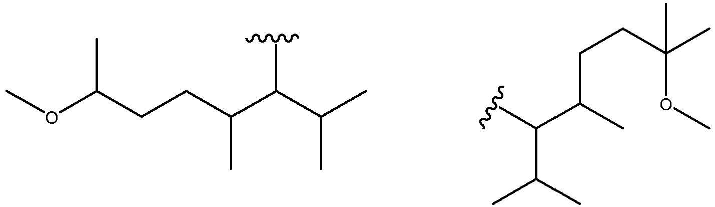 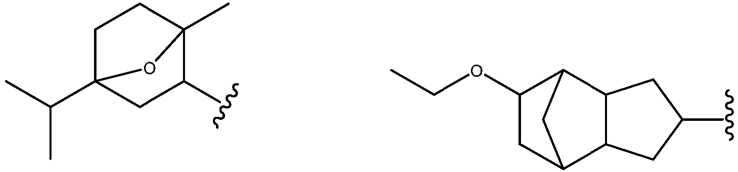 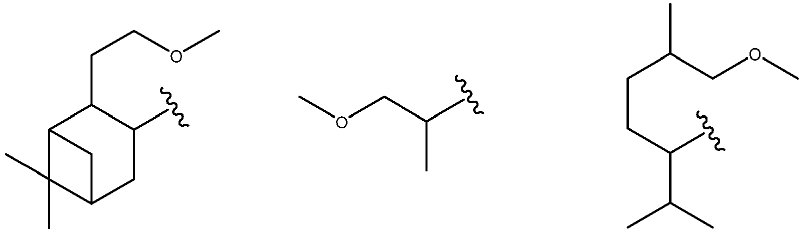 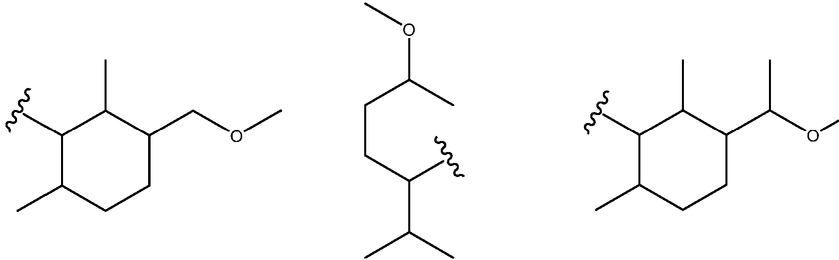 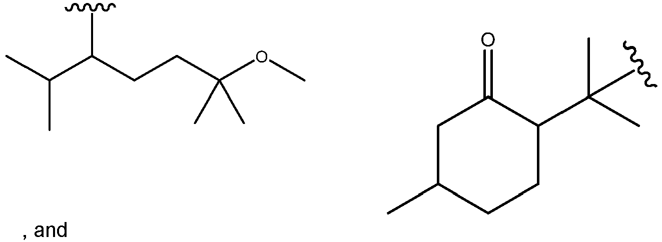
   wherein R³ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another),
   or
(ii) wherein R', R² and R³ are independently selected from the groups as defined in (i), respectively, and R and R¹ are bonded together to form a saturated or unsaturated ring system comprising 5 to 10 carbon atoms, preferably 6 to 8 carbon atoms, most preferably 6 carbon atoms, preferably wherein the ring system additionally carries one, two, three, four or more C₁ to C₅ linear, branched or cyclic alkyl, alkenyl or alkadienyl substituents (and wherein the substituents can be the same or different from one another),
   or
(iii) wherein R', R¹ and R² are independently selected from the groups as defined in (i), respectively, and R and R³ are bonded together to form a saturated or unsaturated, mono or bicyclic, ring system comprising 5 to 10 carbon atoms and optionally 1 or 2 ether functional groups, preferably comprising 6 to 8 carbon atoms and optionally 1 ether functional group, most preferably comprising 6 carbon atoms, preferably wherein the ring system additionally carries one, two, three, four or more C₁ to C₅ linear, branched or cyclic alkyl, alkenyl or alkadienyl substituents, preferably one, two or more methyl groups.

The ring system as defined in (ii) above includes the carbon atoms to which R and R¹ are bonded and the ring system as defined in (iii) above includes the carbon atoms to which R and R³ are bonded.

The compound of formula (I) (i.e. the precursor) may be in the form of a pure diastereomer or stereoisomer or in the form of any mixture of said isomers.

The compounds of formula (I) as defined herein have not been previously known in the perfume industry. The suitability of the compounds of formula (I) as precursors for the release of fragrance substances was therefore unexpected. In the course of the studies underlying this application, and contrary to the teaching in the prior art with respect to the negative olfactory influence of the released thiol compound, it was surprisingly found that the compounds of formula (I) according to the present invention release both a pleasantly smelling (high impact) α,β-unsaturated aldehyde or ketone and a pleasantly smelling (high impact) thiol compound. Said release of the two (or more) different fragrance substances, and thus their sensorial perception, advantageously occurs in a controlled way, preferably at different points in time, which was particularly surprising. The sensory odour impression of the released thiol compound (e.g. sulphurous-fruity notes), for instance, is very intensely perceptible on damp laundry, especially directly after washing, while the released α,β-unsaturated aldehyde or ketone provides a long-lasting, fresh odour impression on dried laundry (e.g. fresh, green and/or flowery notes).

Further advantages of the present invention are:
- Due to the controlled release of two different pleasantly smelling, high impact fragrance substances with low odorant thresholds (α,β-unsaturated aldehyde or ketone compound, thiol compound), a distinct odour perception occurs when even small amounts of compounds of formula (I) according to the present invention are used.
- A higher atomic economy is achieved compared to the precursors of the prior art, since, unexpectedly, both compounds released by the compounds of formula (I) according to the present invention (α,β-unsaturated aldehyde or ketone compound, thiol compound) contribute to a pleasant odour profile.
- A positive effect of the compounds of formula (I) according to the present invention on the odour impression is surprisingly achieved both on wet laundry (thiol compound) as well as on dried laundry (α,β-unsaturated aldehyde or ketone compound), as the different fragrance substances are preferably released from the compounds of formula (I) according to the present invention and/or sensorially perceived at different points in time.
- The thiol compound released by the compounds of formula (I) according to the present invention not only improves the substantivity and/or deposition of the compounds of formula (I) according to the present invention on e.g. textiles, hair or skin, but it also serves as a pleasantly smelling fragrance substance itself.

Also described herein is a compound of formula (I), wherein
R is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three, four or more C₁ to C₄ linear, cyclic or branched alkyl, alkenyl or alkadienyl substituents (and wherein the substituents can be the same or different from one another), and
R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three, four or more C₁ to C₄ linear, cyclic or branched alkyl, alkenyl or alkadienyl substituents (and wherein the substituents can be the same or different from one another), and
R¹ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three, four or more C₁ to C₄ linear, cyclic or branched alkyl, alkenyl or alkadienyl substituents (and wherein the substituents can be the same or different from one another), and
R² is selected from the group consisting of C₆ to C₁₂, more preferably C₇ to C₁₂, most preferably C₈ to C₁₂, linear, cyclic or branched alkyl residues, preferably additionally comprising one, two or more functional groups selected from the group consisting of ether, alcohol, and ketone, more preferably additionally comprising one ether group or two ether groups or one alcohol group or one ketone group, and
R³ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three, four or more C₁ to C₄ linear, cyclic or branched alkyl, alkenyl or alkadienyl substituents (and wherein the substituents can be the same or different from one another).

Also described herein is a compound of formula (I), wherein
R is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R¹ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R² is selected from the group consisting of C₆ to C₁₂, more preferably C₇ to C₁₂, most preferably C₈ to C₁₂, linear, cyclic or branched alkyl residues, preferably additionally comprising one, two or more functional groups selected from the group consisting of ether, alcohol, and ketone, more preferably additionally comprising one ether group or two ether groups or one alcohol group or ketone group, and
R³ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another).

Also described herein is a compound of formula (I), wherein
R is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl or alkenyl residues, preferably wherein the residues additionally carry one, two, three or more substituents selected from the group consisting of methyl and ethenyl (and wherein the substituents can be the same or different from one another), and
R² is selected from the group consisting of C₆ to C₁₂, more preferably C₇ to C₁₂, most preferably C₈ to C₁₂, linear, cyclic or branched alkyl residues, preferably additionally comprising one, two or more functional groups selected from the group consisting of ether, alcohol, and ketone, more preferably additionally comprising one ether group or two ether groups or one alcohol group or one ketone group, and
R³ is hydrogen.

Residue R² of the compounds of formula (I) according to the invention does not comprise any Si atoms.

According to another preferred embodiment of the compound of formula (I) according to the invention, R² is not an n-butyl, n-pentyl, or n-hexyl (or 2-propyl) residue.

According to another preferred embodiment of the compound of formula (I) according to the invention, R² is not a residue of the following formula (4-hydroxybutyl):

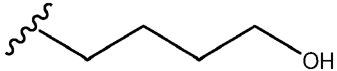

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one, two or more ether functional groups.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one ether functional group.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising two ether functional groups.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one, two or more alcohol functional groups.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one alcohol functional group.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising two alcohol functional groups.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one, two or more ketone functional groups.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising one ketone functional group.

Also described herein is a compound of formula (I), wherein R² is selected from the group consisting of C₄ to C₁₅, preferably C₄ to C₁₂, more preferably C₅ to C₁₂, more preferably C₆ to C₁₂, more preferably C₇ to C₁₂, most preferably C₈ to C₁₂, linear, cyclic or branched alkyl residues, additionally comprising two ketone functional groups.

According to a preferred embodiment of the compound of formula (I) according to the invention, R is selected from the group consisting of

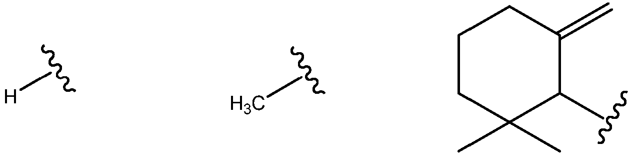

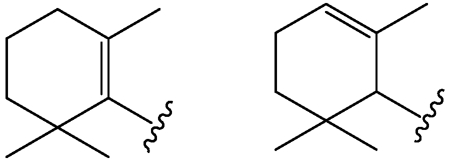

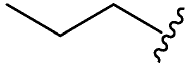

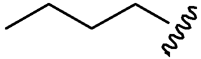

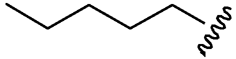

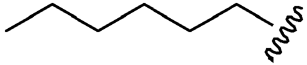

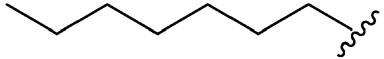

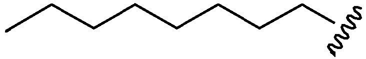

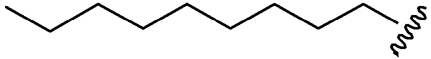

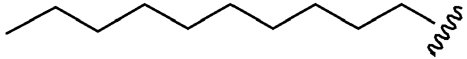

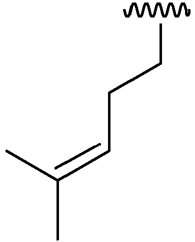

According to another preferred embodiment of the compound of formula (I) according to the invention, R' is selected from the group consisting of

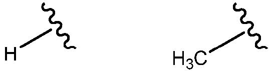

According to another preferred embodiment of the compound of formula (I) according to the invention, R¹ is selected from the group consisting of

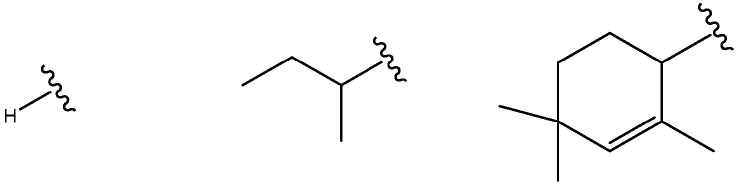

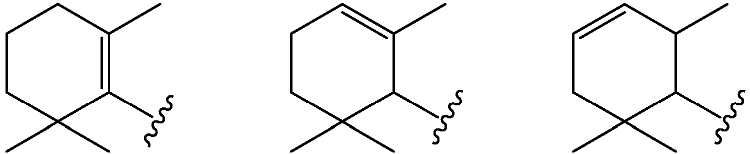

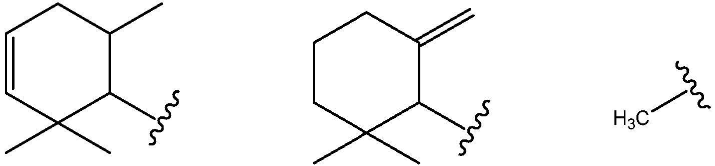

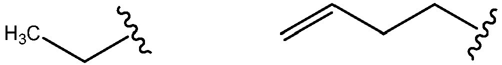

According to another preferred embodiment of the compound of formula (I) according to invention, R² is selected from the group consisting of

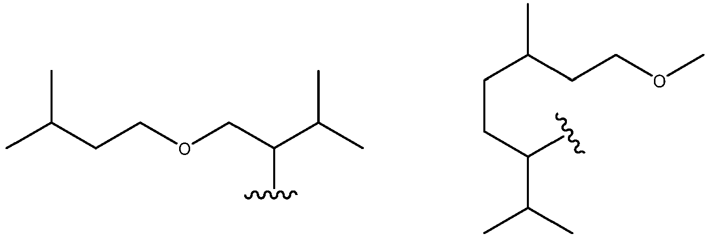

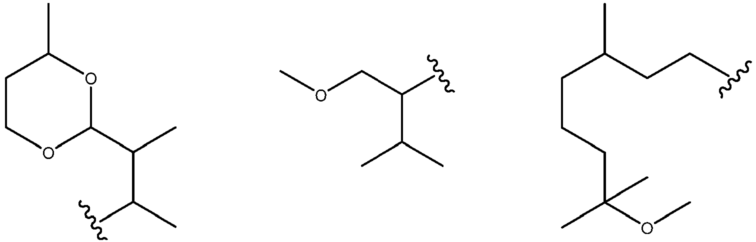

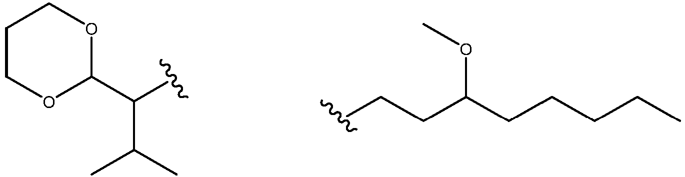

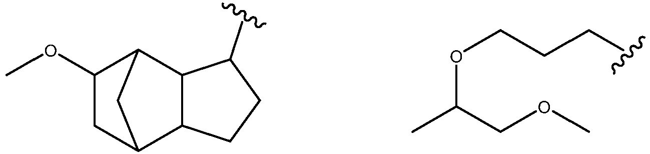

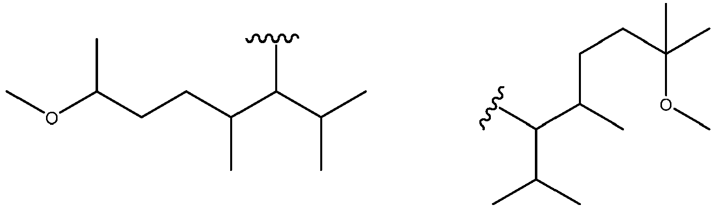

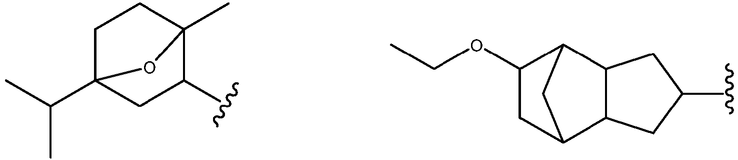

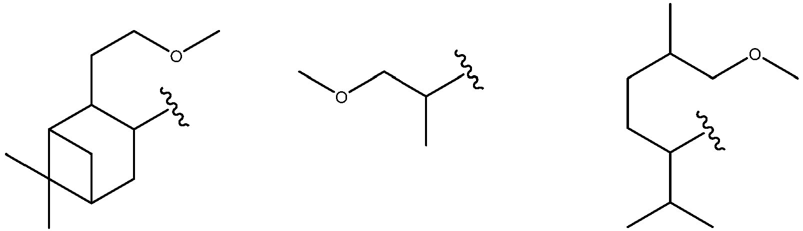

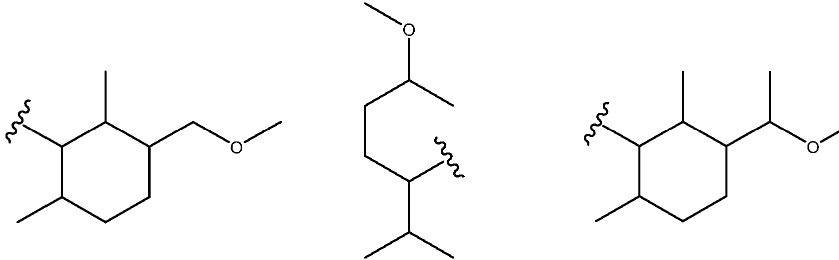

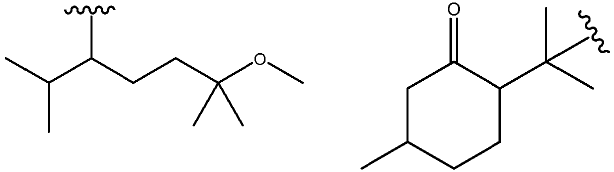

According to a particularly preferred embodiment of the compound of formula (I) according to the invention,
R is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
R' is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
R¹ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another), and
R² is selected from the group consisting of 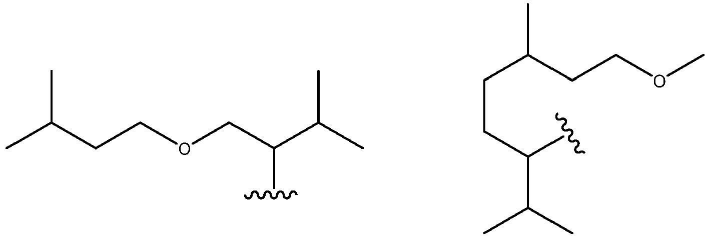 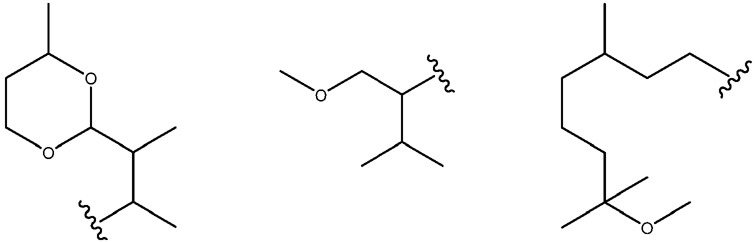 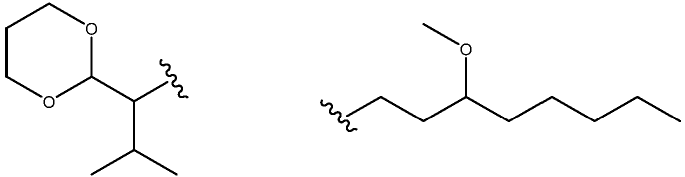 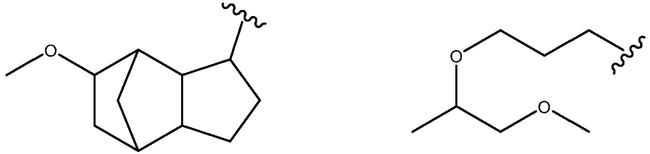 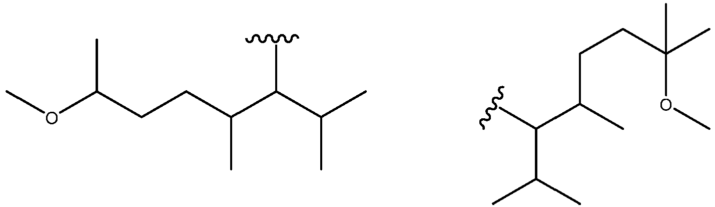 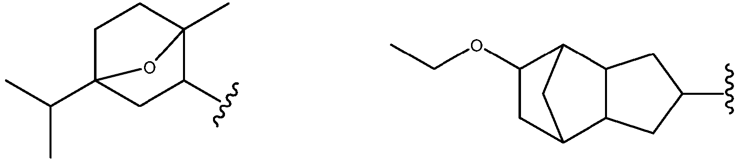 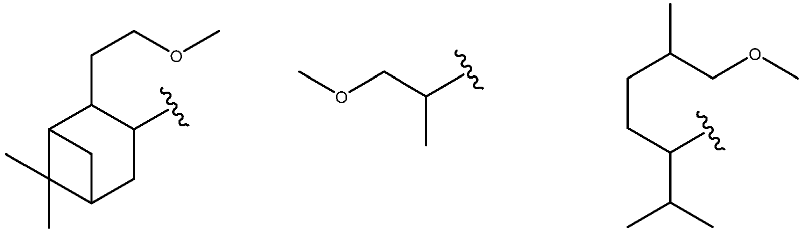 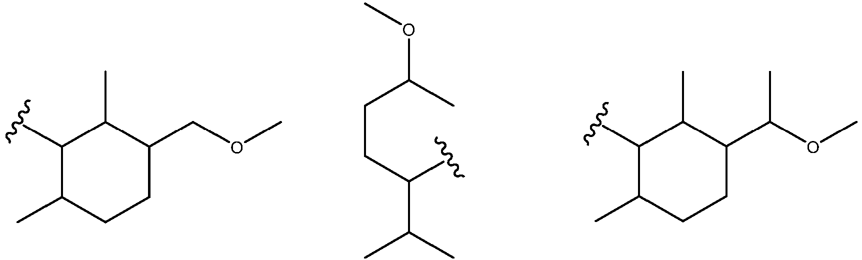 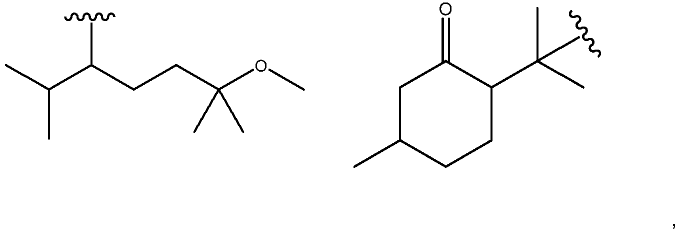 and
R³ is selected from the group consisting of hydrogen and C₁ to C₁₀, preferably C₁ to C₉, more preferably C₁ to C₈, more preferably C₁ to C₇, more preferably C₁ to C₆, most preferably C₁ to C₅, linear or cyclic alkyl, alkenyl or alkadienyl residues, preferably wherein the residues additionally carry one, two, three, four or more C₁ to C₄ linear, cyclic or branched alkyl, alkenyl or alkadienyl substituents (and wherein the substituents can be the same or different from one another).

According to another particularly preferred embodiment of the compound of formula (I) according to the invention,
residue 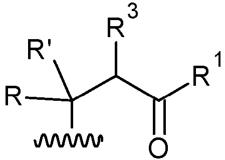
of the compound of formula (I) is selected from the group consisting of 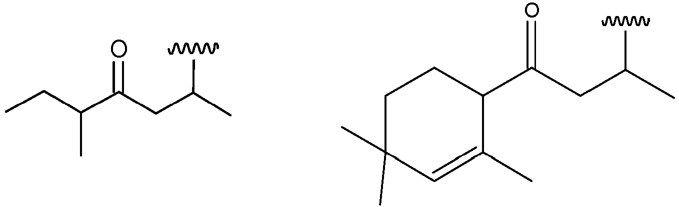 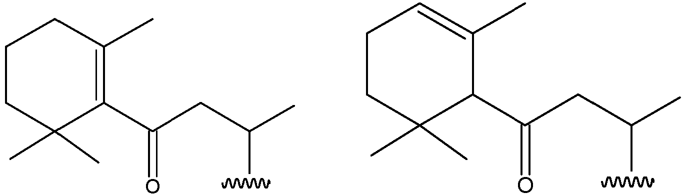 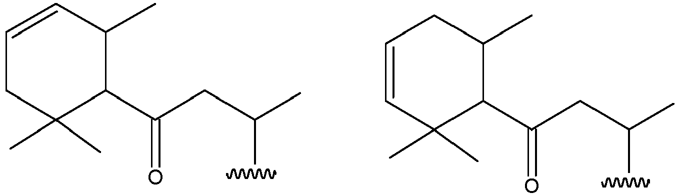 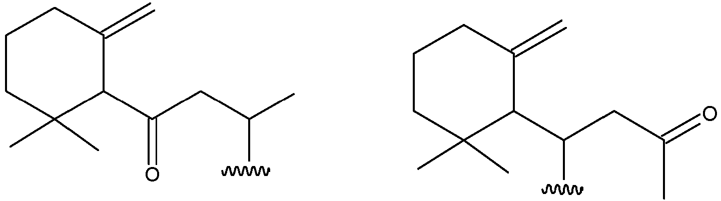 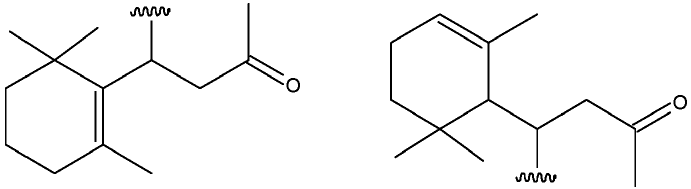 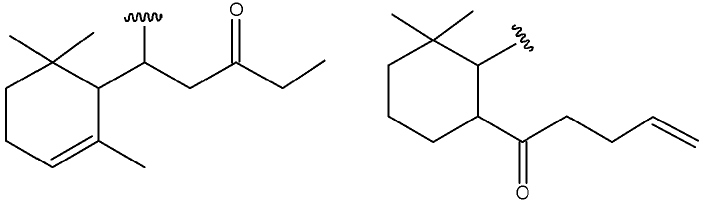 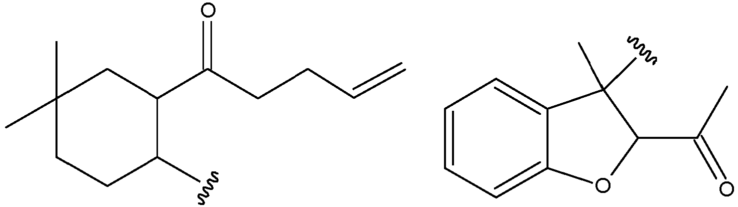 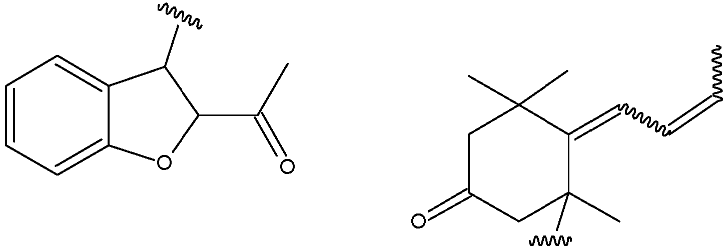 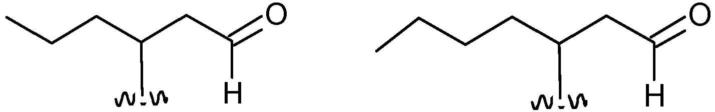 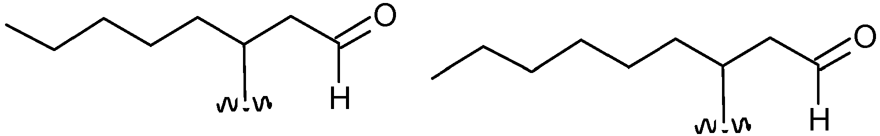 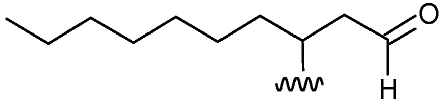 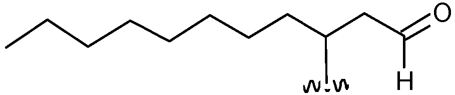 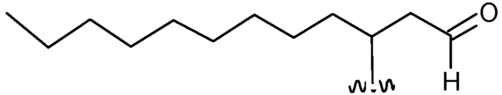 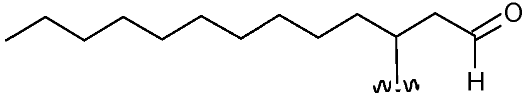 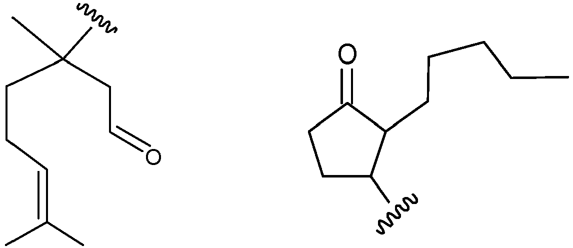 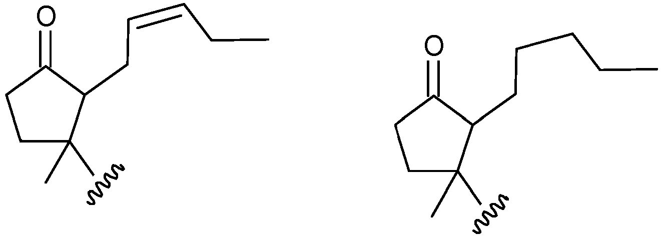
and/or, preferably and, residue R² of the compound of formula (I) is selected from the group consisting of

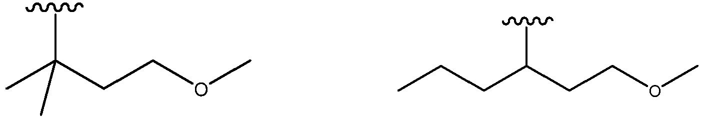

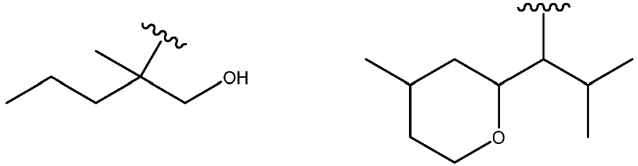

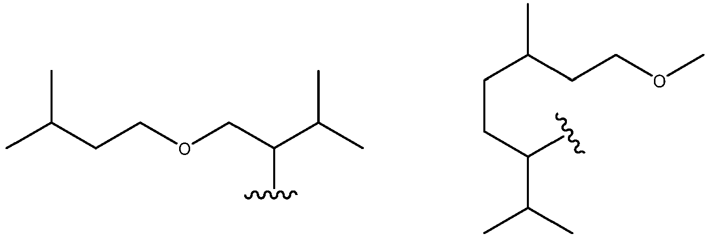

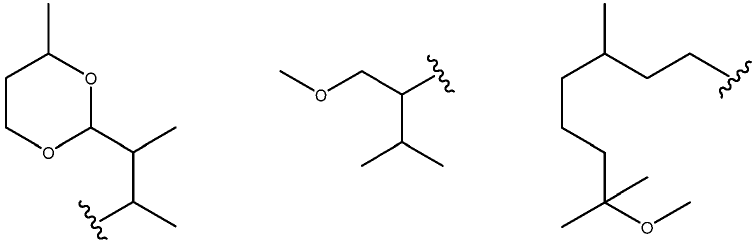

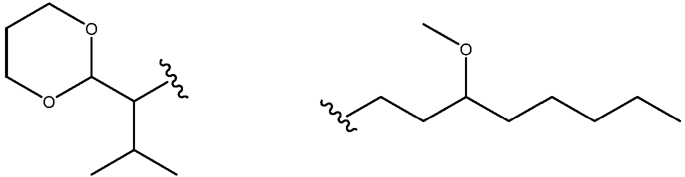

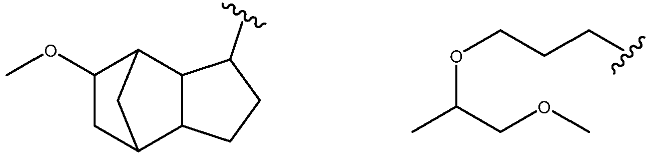

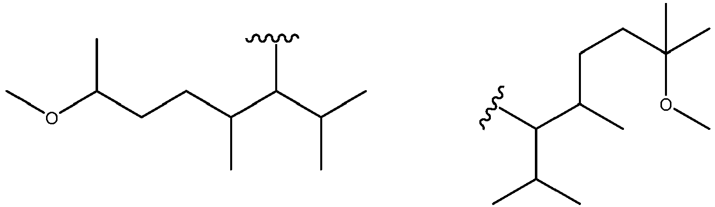

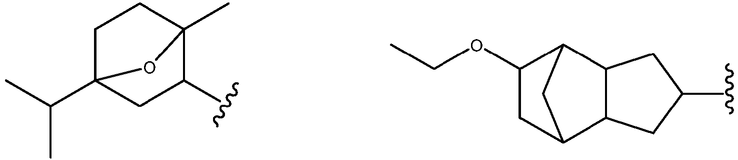

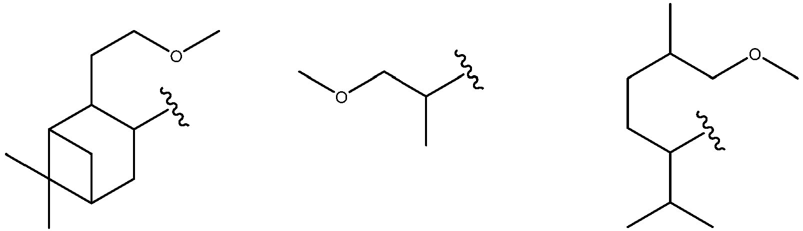

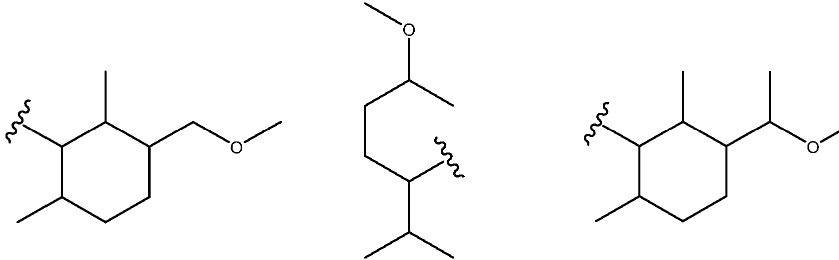

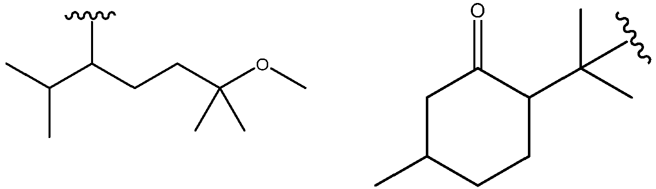

According to a preferred embodiment, the compound of formula (I) according to the invention is obtained or obtainable by a [1,4]-addition reaction between:
- an α,β-unsaturated aldehyde or ketone (Michael acceptor, electrophile) selected from the group consisting of (group of compounds as shown below in the column entitled "Structure" of **Table 1)**

**Table 1**

| **CAS Number(s)** | **Name** | **Structure** |
|---|---|---|
| 102322-83-8 | Filbertone | 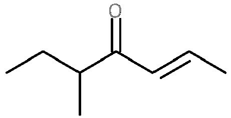 |
| 39872-57-6 | Isodamascone | 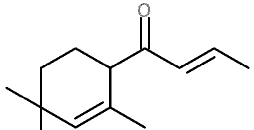 |
| 23726-91-2 | beta-Damascone | 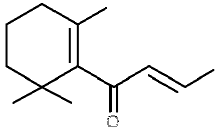 |
| 24720-09-0 | alpha-Damascone | 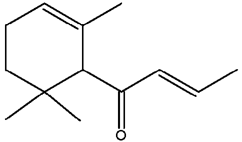 |
| 57378-68-4 | delta-Damascone | 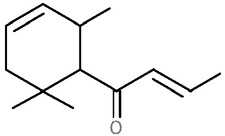 |
| 57350-34-2 | epsilon-Damascone | 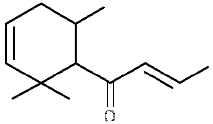 |
| 35076-56-3 | gamma-Damascone | 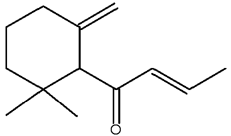 |
| 79-76-5 | gamma-Ionone | 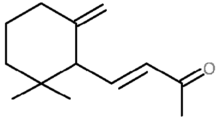 |
| 79-77-6 | beta-lonone | 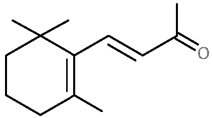 |
| 127-41-3 | alpha-lonone | 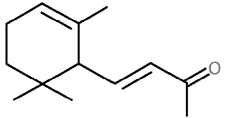 |
| 127-42-4 | Methyliridone | 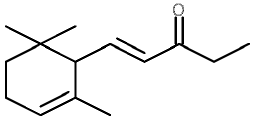 |
| 56973-84-3 | beta-Dynascone | 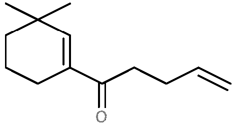 |
| 56973-85-4 | alpha-Dynascone | 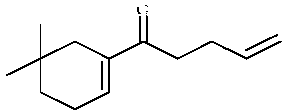 |
| 23911-56-0 | Nerolione | 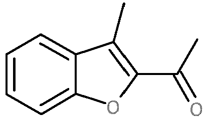 |
| 1646-26-0 | Coumarone | 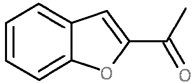 |
| 13215-88-8, 163440-97-9 (mixture of isomers) | Tabanone | 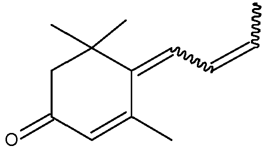 |
| 505-57-7 | 2-Hexenal | 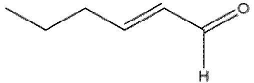 |
| 2463-63-0 | 2-Heptenal | 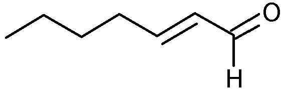 |
| 2363-89-5 | 2-Octenal | 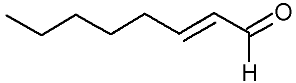 |
| 2463-53-8 | 2-Nonenal | 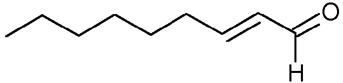 |
| 3913-71-1 | 2-Decenal | 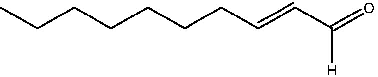 |
| 2463-77-6 | 2-Undecenal | 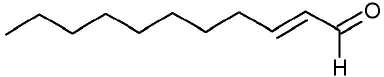 |
| 4826-62-4 | 2-Dodecenal | 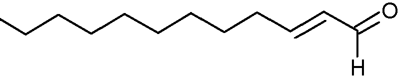 |
| 7774-82-5 | 2-Tridecenal | 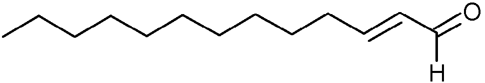 |
| 106-26-3 | Neral | 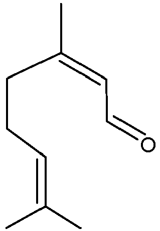 |
| 141-27-5 | Geranial | 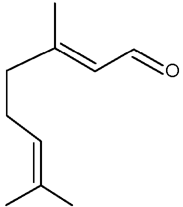 |
| 5392-40-5 (mixture of isomers) | Citral | 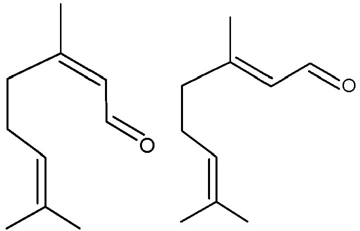 |
| | 2-Pentylcyclopent-2-en-1-one | 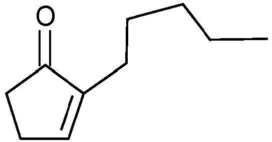 |
| | (Z)-3-Methyl-2-(pent-2-en-1-yl)cyclopent-2-en-1-one | 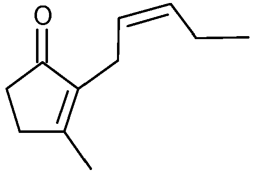 |
| | 3-Methyl-2-pentylcyclopent-2-en-1-one | 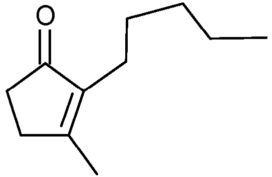 |

and
- a thiol (Michael donor, nucleophile) selected from the group consisting of (group of compounds as shown below in the column entitled "Structure" of **Table 2**)

**Table 2**

| **Chemical Name** | **Odour Description** | **Structure** |
|---|---|---|
| 4-Methoxy-2-methyl-butane-2-thiol | - | 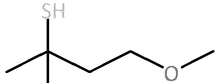 |
| 1-Methoxyhexane-3-thiol | - | |
| | | 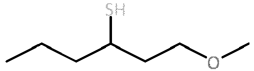 |
| 2-Methyl-2-sulfanyl-pentan-1-ol | Grapefruit, cassis, onion | 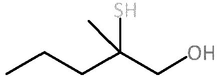 |
| 2-Methyl-1-(4-methyltetrahydropyran-2-yl)propane-1-thiol | Very powerful, more fruity, better quality, exotic fruit, guava | 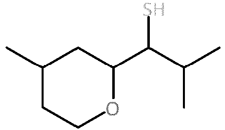 |
| 1-lsopentyloxy-3-methylbutane-2-thiol | breath of smoker | 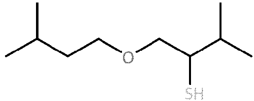 |
| 8-Methoxy-2,6-dimethyl-octane-3-thiol | Sulphur, grapefruit - sulphuric side, more fruity, cassis, plastic, green, rose oxide, green pepper | 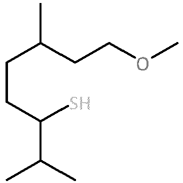 |
| 3-(4-Methyl-1,3-dioxan-2-yl)butane-2-thiol | Liver sausage, burnt plastic, buchu (Asian chives) note, cassis, gasoline | 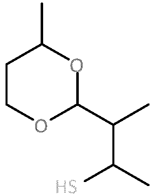 |
| 1-Methoxy-3-methyl-butane-2-thiol | Strong, more fruity, juicy, natural, cat grass, meat broth ("Fleischbrühe"), onion | 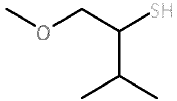 |
| 7-Methoxy-3,7-dimethyl-octane-1-thiol | Cheesy, camembert, cheese in the fridge | 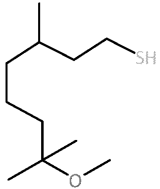 |
| 1-(1,3-Dioxan-2-yl)-2-methylpropane-1-thiol | Fresh, strong, hot rubber, petrol | 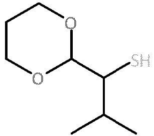 |
| 3-Methoxyoctane-1-thiol | Rose oxide, violet | 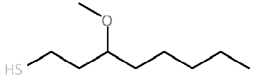 |
| Methoxytricyclo[5.2.1.0^{2,6}]dec ane-3-thiol | Blackcurrant, catnip, natural, fruity, zestral aspect, anisic | 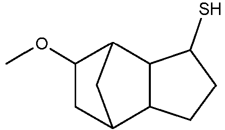 |
| 3-(2-Methoxy-1-methyl-ethoxy)propane-1-thiol | Minty green, catnip grass, mushroom, soup, leek | 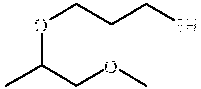 |
| 7-Methoxy-2,4-dimethyl-octane-3-thiol | Sulphur and something well known behind, agrumen, fruity, grapefruit but with natural aspect, odour direction: grapfruit natural | 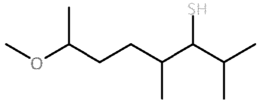 |
| 7-Methoxy-2,4,7-trimethyl-octane-3-thiol | Peach, cassis, meat, sulphur | 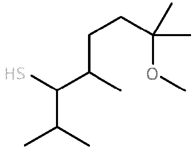 |
| 4-Isopropyl-1-methyl-7-oxabicyclo[2.2.1]heptane-2-thiol | - | 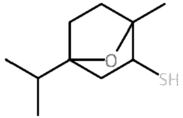 |
| 8-Ethoxy-tricyclo[5.2.1.0^{2,6}]decane-4-thiol | Cassis, sulphur | 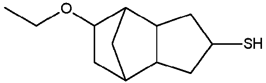 |
| 3-Ethyl-2-(2-methoxyethyl)-4,4-dimethyl-cyclohexanethiol | Grapefruit, pine, meat, natural | 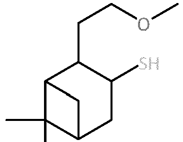 |
| 1-Methoxypropane-2-thiol | Mango, meat | 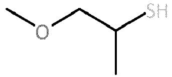 |
| 7-Methoxy-2,6-dimethyl-heptane-3-thiol | Sulphur, durian, jackfruit, artichoke | 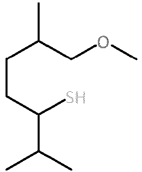 |
| 6-(Methoxymethyl)-2,3-dimethyl-cyclohexanethiol | Cassis, blackcurrant | 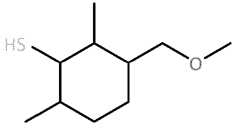 |
| 6-Methoxy-2-methyl-heptane-3-thiol | Sulphur | 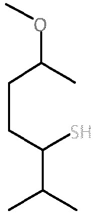 |
| 3-(1-Methoxyethyl)-2,6-dimethyl-cyclohexanethiol | Sulphur | 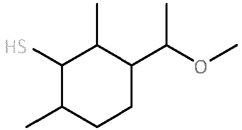 |
| 6-Methoxy-2,6-dimethyl-heptane-3-thiol | Grapefruit, fruity, apricot, peach | 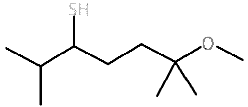 |
| 2-(2-Mercaptopropan-2-yl)-5-methylcyclohexan-1-one | Buchu | 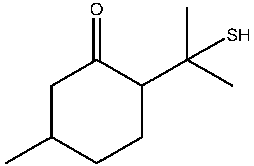 |

According to a preferred embodiment, Citral - i.e. a mixture of Neral and Geranial as shown above in **Table 1** - is used as (a mixture of) α,β-unsaturated aldehyde(s) for the formation of a compound of formula (I).

The compounds of formula (I) according to the invention 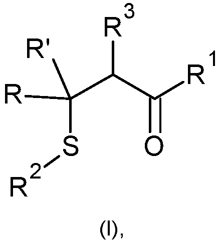 release a (pleasantly smelling, high impact) thiol, which is selected from the group consisting of compounds as shown above in the column entitled "Structure" of **Table 2,** and a (pleasantly smelling, high impact) α,β-unsaturated aldehyde or ketone, which is preferably selected from the group consisting of compounds as shown above in the column entitled "Structure" of **Table 1,** *via* the following decomposition reaction:

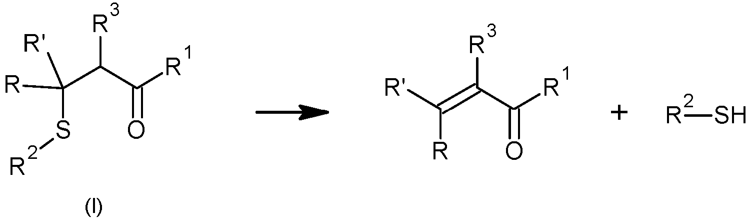

Preferably, said release is triggered by certain pH values and/or temperatures, UV light, water, and/or oxygen.

According to a particularly preferred embodiment, the compound of formula (I) according to the invention is selected from the group consisting of (group of compounds as shown below in the column entitled "Structure" of **Table 3**):

**Table 3**

| **Chemical name** | **Structure** |
|---|---|
| 3-[2-Methyl-1-(4-methyltetrahydropyran-2-yl)propyl]sulfanyl-1-(2,6,6-trimethylcyclohex-3-en-1-yl)butan-1-one | 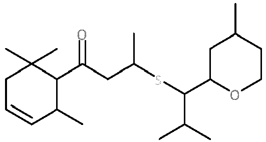 |
| 3-[2-Methyl-1-(4-methyltetrahydropyran-2-yl)propyl]sulfanyldecanal | 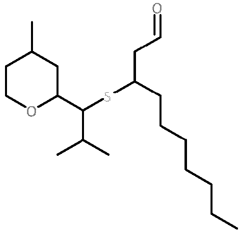 |
| 2-[1-(Methoxymethyl)-2-methylpropyl]sulfanyl-5-methyl-heptan-4-one | 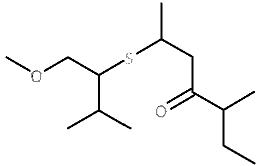 |
| 2-(3-Methoxyoctylsulfanyl)-5-methyl-heptan-4-one | 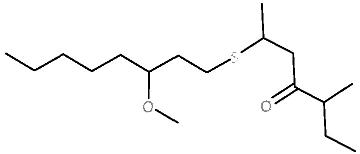 |
| 2-[(8-Ethoxy-4-tricyclo[5.2.1.02,6]decanyl)sulfanyl]-5-methyl-heptan-4-one | 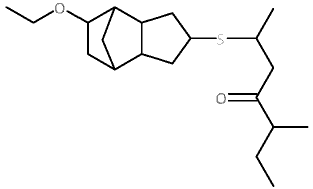 |
| 2-(2-Methoxy-1-methyl-ethyl)sulfanyl-5-methyl-heptan-4-one | 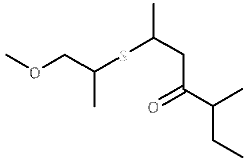 |
| 3-(3-Methoxy-1,1-dimethylpropyl)sulfanyl-1-(2,6,6-trimethylcyclohex-3-en-1-yl)butan-1-one | 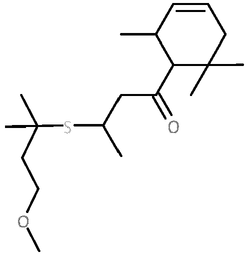 |
| 3-[1-(2-Methoxyethyl)butylsulfanyl]-1-(2,6,6-trimethylcyclohex-3-en-1-yl)butan-1-one | 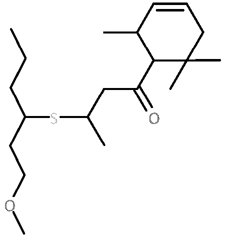 |
| 3-[1-(Hydroxymethyl)-1-methylbutyl]sulfanyl-1-(2,6,6-trimethylcyclohex-3-en-1-yl)butan-1-one | 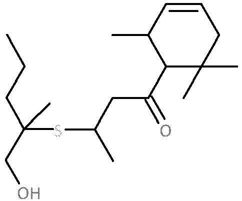 |
| 3-(1-Isopropyl-6-methoxy-4-methylhexyl)sulfanyl-1-(2,6,6-trimethylcyclohex-3-en-1-yl)butan-1-one | 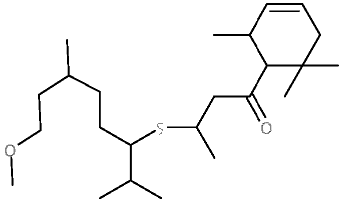 |
| 3-[1-(Methoxymethyl)-2-methylpropyl]sulfanyl-1-(2,6,6-trimethylcyclohex-3-en-1-yl)butan-1-one | 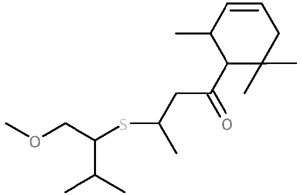 |
| 3-(3-Methoxyoctylsulfanyl)-1-(2,6,6-trimethylcyclohex-3-en-1-yl)butan-1-one | 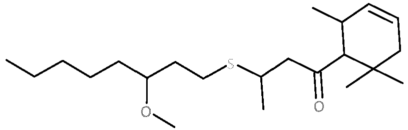 |
| 3-[(8-Ethoxy-4-tricyclo[5.2.1.02,6]decanyl)sulfanyl]-1-(2,6,6-trimethylcyclohex-3-en-1-yl)butan-1-one | 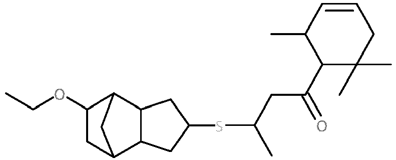 |
| 3-(2-Methoxy-1-methyl-ethyl)sulfanyl-1-(2,6,6-trimethylcyclohex-3-en-1-yl)butan-1-one | 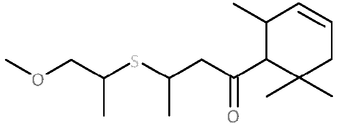 |
| 3-(1-Isopropyl-4-methoxy-4-methylpentyl)sulfanyl-1-(2,6,6-trimethylcyclohex-3-en-1-yl)butan-1-one | 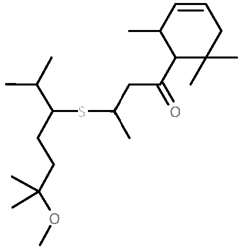 |
| 3-[1-(Methoxymethyl)-2-methylpropyl]sulfanyl-1-(2,4,4-trimethylcyclohex-2-en-1-yl)butan-1-one | 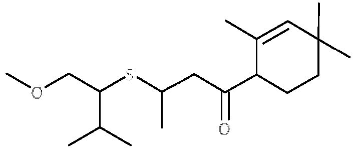 |
| 3-[(8-Ethoxy-4-tricyclo[5.2.1.02,6]decanyl)sulfanyl]-1-(2,4,4-trimethylcyclohex-2-en-1-yl)butan-1-one | 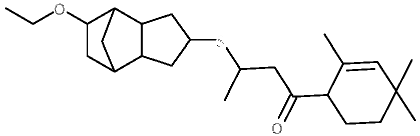 |
| 3-(2-Methoxy-1-methyl-ethyl)sulfanyl-1-(2,4,4-trimethylcyclohex-2-en-1-yl)butan-1-one | 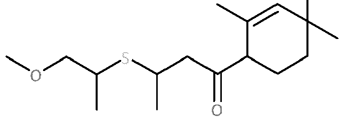 |
| 1-[2-[(8-Ethoxy-4-tricyclo[5.2.1.02,6]decanyl)sulfanyl]-3,3-dimethyl-cyclohexyl]pent-4-en-1-one | 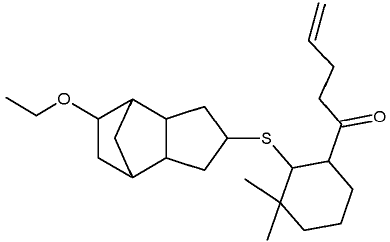 |
| 1-[2-(1-Isopropyl-6-methoxy-4-methylhexyl)sulfanyl-3,3-dimethylcyclohexyl]pent-4-en-1-one | 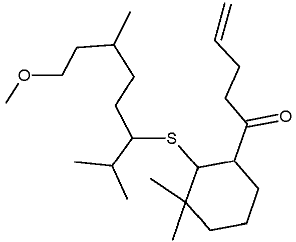 |
| 1-[2-(2-Methoxy-1-methylethyl)sulfanyl-3,3-dimethylcyclohexyl]pent-4-en-1-one | 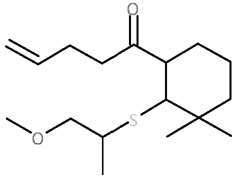 |
| 1-[2-(3-Methoxyoctylsulfanyl)-3,3-dimethyl-cyclohexyl]pent-4-en-1-one | 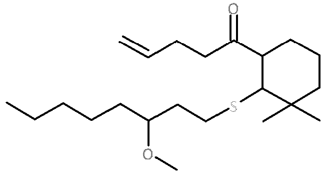 |
| 1-[2-(3-Methoxy-1,1-dimethylpropyl)sulfanyl-5,5-dimethylcyclohexyl]pentan-1-one | 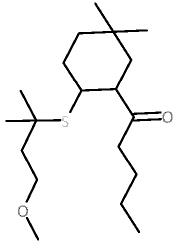 |
| 1-[2-[1-(2-Methoxyethyl)butylsulfanyl]-5,5-dimethyl-cyclohexyl]pentan-1-one | 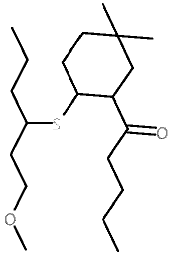 |
| 1-[2-[1-(Hydroxymethyl)-1-methylbutyl]sulfanyl-5,5-dimethylcyclohexyl]pentan-1-one | 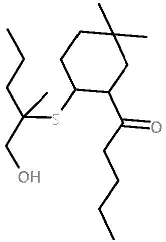 |
| 1-[2-(1-Isopropyl-4-methoxy-4-methylpentyl)sulfanyl-5,5-dimethylcyclohexyl]pentan-1-one | 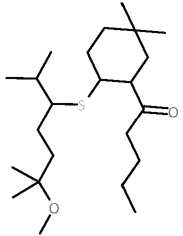 |
| 1-[3-(2-Methoxy-1-methylethyl)sulfanyl-2,3-dihydrobenzofuran-2-yl]ethanone | 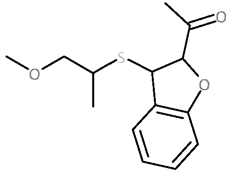 |
| 1-[3-[(8-Ethoxy-4-tricyclo[5.2.1.02,6]decanyl)sulfanyl]-2,3-dihydrobenzofuran-2-yl]ethanone | 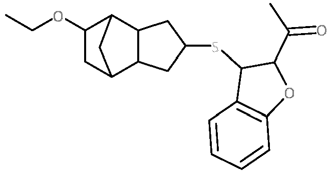 |
| 1-[3-[1-(Methoxymethyl)-2-methylpropyl]sulfanyl-2,3-dihydrobenzofuran-2-yl]ethanone | 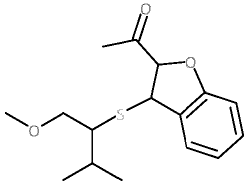 |
| 1-[3-[1-(Hydroxymethyl)-1-methylbutyl]sulfanyl-2,3-dihydrobenzofuran-2-yl]ethanone | 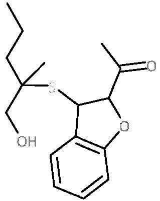 |
| 1-[3-(3-Methoxy-1,1-dimethylpropyl)sulfanyl-2,3-dihydrobenzofuran-2-yl]ethanone | 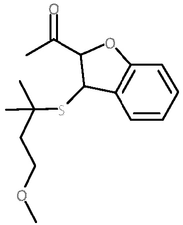 |
| 3-[1-(2-Methoxyethyl)butylsulfanyl]hexanal | 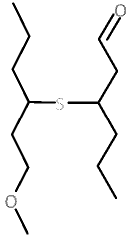 |
| 3-(1-Isopropyl-6-methoxy-4-methylhexyl)sulfanylhexanal | 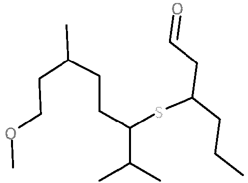 |
| 3-(3-Methoxyoctylsulfanyl)hexanal | 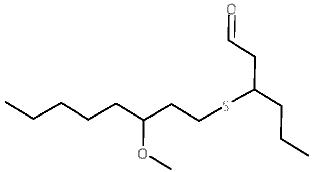 |
| 3-(2-Methoxy-1-methylethyl)sulfanylhexanal | 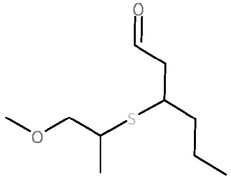 |
| 3-(1-Isopropyl-4-methoxy-4-methylpentyl)sulfanylundecanal | 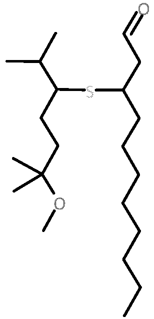 |
| 3-[(8-Ethoxy-4-tricyclo[5.2.1.02,6]decanyl)sulfanyl]un decanal | 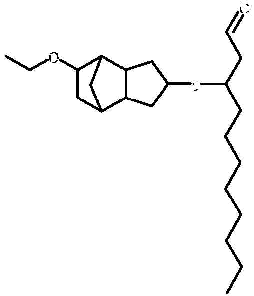 |
| 3-(3-Methoxyoctylsulfanyl)undecanal | 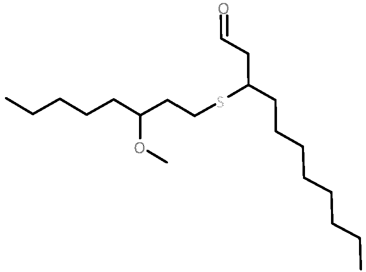 |
| 3-(1-Isopropyl-6-methoxy-4-methylhexyl)sulfanylundecanal | 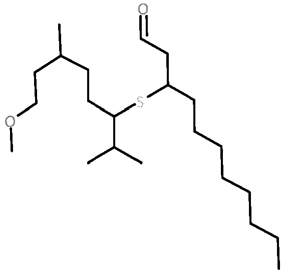 |
| 3-(3-Methoxy-1,1-dimethylpropyl)sulfanylundecanal | 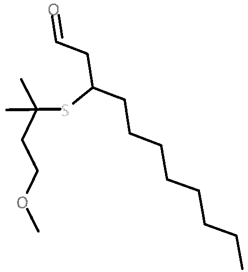 |
| 3-[1-(Hydroxymethyl)-1-methylbutyl]sulfanyltridecanal | 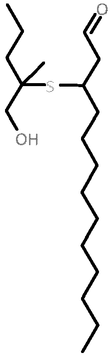 |
| 3-(3-Methoxyoctylsulfanyl)tridecanal | 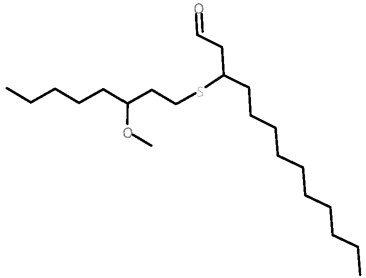 |
| 3-(3-Methoxyoctylsulfanyl)-3,7-dimethyl-oct-6-enal | 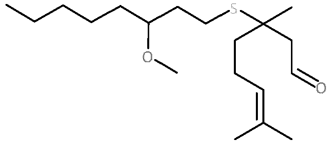 |
| 3-[1-(Methoxymethyl)-2-methylpropyl]sulfanyl-3,7-dimethyl-oct-6-enal | 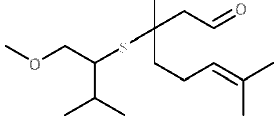 |
| 3-[(8-Ethoxy-4-tricyclo[5.2.1.02,6]decanyl)sulfanyl]-3,7-dimethyl-oct-6-enal | 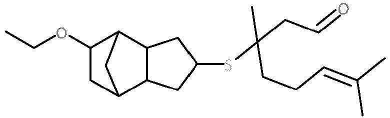 |
| 3-[1-(2-Methoxyethyl)butylsulfanyl]-3,7-dimethyl-oct-6-enal | 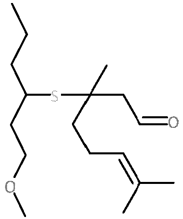 |
| 3-((2-Methyl-1-(4-methyltetrahydro-2H-pyran-2-yl)propyl)thio)-2-pentylcyclopentan-1-one | 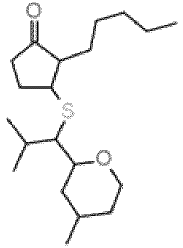 |
| 5-Methyl-2-(2-((3-oxo-2-pentylcyclopentyl)thio)propan-2-yl)cyclohexan-1-one | 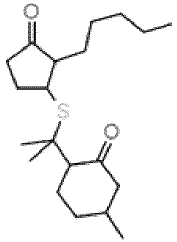 |
| 5-Methyl-2-[1-methyl-1-[1-methyl-3-oxo-3-(2,6,6-trimethylcyclohex-3-en-1-yl)propyl]sulfanyl-ethyl]cyclohexanone | 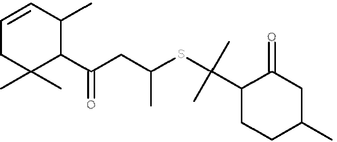 |

According to a particularly preferred embodiment, the compound of formula (I) according to the invention is not

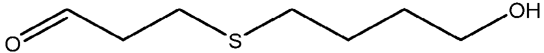

Another aspect of the present invention relates to a fragrance substance mixture, preferably perfume oil, comprising or consisting of the following components:
(i) one or more compound(s) of formula (I) as defined herein,
   and
(ii) one or more further fragrance substances.

The one or more further fragrance substances are different from the compounds of formula (I) according to the invention.

Examples of further fragrance substances that can be advantageously combined with the compounds of formula (I) as defined herein within the scope of the present invention are known to the skilled person and can be found, for example, in S. Arctander, Perfume and Flavor Materials, Vol. I and II, Montclair, N. J. 1969, Eigenverlag, or K. Bauer et al., Common Fragrance and Flavor Materials, 4th Edition, Wiley-VCH, Weinheim 2001.

According to a preferred embodiment of the fragrance substance mixture according to the invention, preferably perfume oil, the proportion of the total amount of compound(s) of formula (I) as defined herein in the fragrance substance mixture is 0.1 to 3 % by weight, preferably 0.5 to 2.5 % by weight, particularly preferably 1 to 2 % by weight, based on the total mass of the fragrance substance mixture.

Another aspect of the present invention relates to a method for producing a fragrance substance mixture, preferably a perfume oil, as defined above, comprising or consisting of the following step:
Mixing of one or more compound(s) of formula (I) as defined herein with one or more further fragrance substances.

Furthermore, fragrance substance mixtures, preferably perfume oils, according to the invention may be adsorbed on a carrier which provides both a fine distribution of the fragrance substance mixture, preferably perfume oil, in the perfumed product and a controlled release during application. Such carriers may be porous inorganic materials such as light sulphate, silica gels, zeolites, gypsums, clays, clay granules, gas concrete, etc. or organic materials such as wood, cellulose-based materials, sugar, dextrins (e.g. maltodextrin) or plastics such as PVC, polyvinyl acetates or polyurethanes. The combination of a fragrance substance mixture, preferably perfume oil, according to the invention and a carrier is also to be understood as a fragrance substance mixture, preferably perfume oil, of the invention or may be presented as a perfumed product according to the invention (as described below).

Fragrance substance mixtures, preferably perfume oils, or perfumed products of the invention (as described below) may also be microencapsulated, spray-dried, or may be provided as inclusion complexes or as extrusion products and, in the case of a fragrance substance mixture, preferably perfume oil, may be added in this form e.g. to a perfumed product to be perfumed (as described below).

If necessary, the properties of such modified mixtures or products can be further optimized by so-called "coating" with suitable materials with a view to a more targeted release of fragrance, preferably using wax-like plastics such as polyvinyl alcohol. The resulting products in turn represent products of the invention.

Microencapsulation can, for example, be achieved by the so-called coacervation method using capsule materials such as polyurethane-like substances or soft gelatin.

Spray-dried products are preferably produced by spray-drying an emulsion or dispersion containing the fragrance substance mixtures, preferably perfume oils, according to the invention, whereby modified starches, proteins, dextrin and vegetable gums can be used as carriers.

Inclusion complexes can be produced e.g. by incorporating dispersions of the fragrance substance mixtures, preferably perfume oils, according to the invention and cyclodextrins or urea derivatives into a suitable solvent, e.g. water.

Extrusion products can be obtained, e.g., by using the fragrance substance mixtures, preferably perfume oils, according to the invention with a suitable waxy substance and by extrusion followed by solidification, if necessary, in a suitable solvent, e.g. isopropanol.

Another aspect of the present invention relates to a perfumed product comprising one or more compound(s) of formula (I) as defined herein or a fragrance substance mixture, preferably a perfume oil, as defined herein, preferably in a sensorially effective amount.

Advantageously, the one or more compound(s) of formula (I) according to the invention positively impart or modify the odour of a perfumed product to which said compound(s) of formula (I) are added.

According to a preferred embodiment, the perfumed product is selected from the group consisting of perfume extracts, eau de parfums, eau de toilettes, aftershaves, eau de colognes, pre-shave products, splash colognes, perfumed refreshing cloths, acidic, alkaline and neutral detergents, textile fresheners, ironing aids, liquid detergents, powdered detergents, laundry pre-treatment agents, fabric softeners, washing soaps, washing tablets, disinfectants, surface disinfectants, air fresheners, aerosol sprays, waxes and polishes, personal care products, hand creams and lotions, foot creams and lotions, depilatory creams and lotions, aftershave creams and lotions, tanning creams and lotions, hair care products, deodorants and antiperspirants, decorative cosmetic products, candles, lamp oils, incense sticks, insecticides, repellents, and fuels, most preferably is a fabric softener.

According to a preferred embodiment of the perfumed product according to the invention, the proportion of the total amount of fragrance substance mixture as defined herein, preferably perfume oil, in the perfumed product is 0.1 to 3 % by weight, preferably 0.5 to 2.5 % by weight, particularly preferably 1 to 2 % by weight, based on the total mass of the perfumed product.

Another aspect of the present invention relates to a method for producing a perfumed product, preferably a perfumed product as defined herein, comprising or consisting of the following steps:
(i) Providing one or more compound(s) of formula (I) as defined herein or a fragrance substance mixture, preferably a perfume oil, as defined herein,
(ii) providing one or more further components of the perfumed product to be produced, and
(iii) contacting or mixing the further components provided in step (ii) with a sensorially effective amount of the components provided in step (i).

Another aspect of the present invention relates to the use of a compound of formula (I) as defined herein as a precursor for the (controlled) release of one, two, three or more different pleasantly smelling fragrance substances, preferably wherein the two or more different fragrance substances are released and/or sensorially perceived at the same or different, preferably different, point(s) in time (if two or more different fragrance substances are released).

Another aspect of the present invention relates to the use of a compound of formula (I) as defined herein as a precursor for the (controlled) release of a pleasantly smelling thiol and of a pleasantly smelling α,β-unsaturated aldehyde or ketone, preferably wherein the thiol and the α,β-unsaturated aldehyde or ketone are released and/or sensorially perceived at the same or different, preferably different, point(s) in time.

Another aspect of the present invention relates to the use of a compound of formula (I) as defined herein as a precursor for the (controlled) release of a (pleasantly smelling) thiol selected from the group consisting of 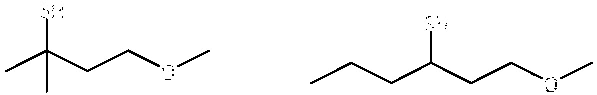 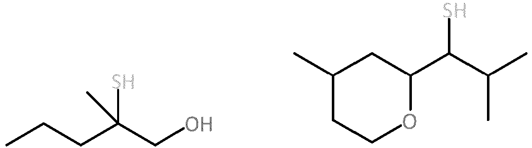 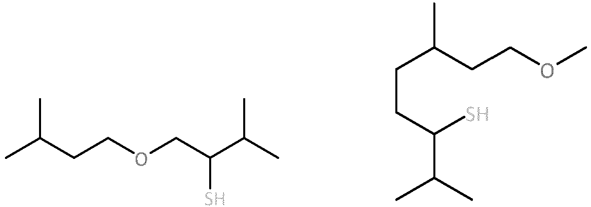 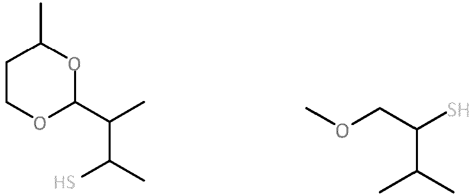 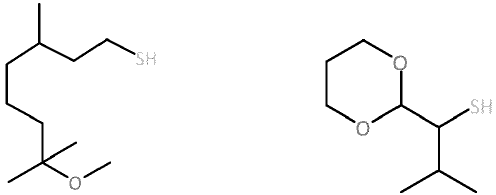 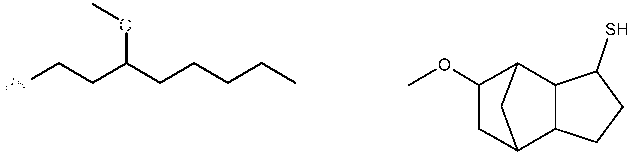 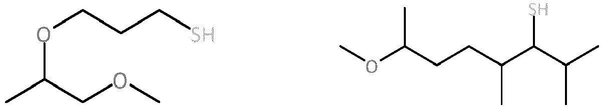 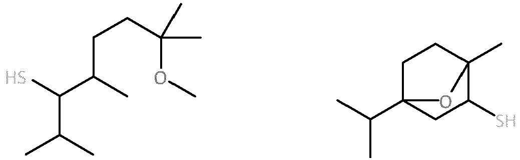 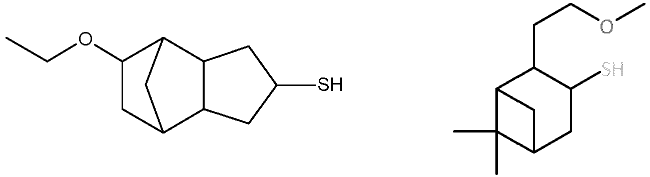 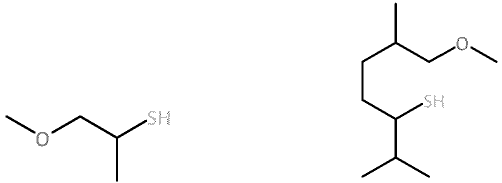 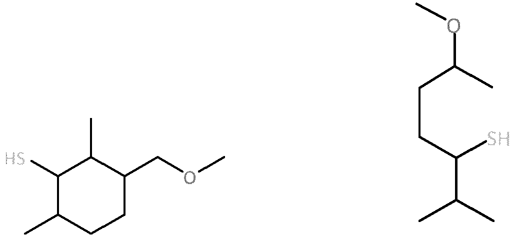 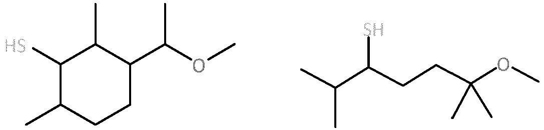 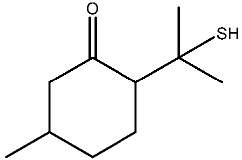 and/or, preferably and, of a (pleasantly smelling) α,β-unsaturated aldehyde or ketone selected from the group consisting of 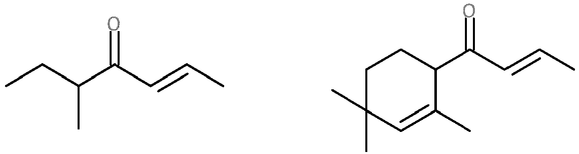 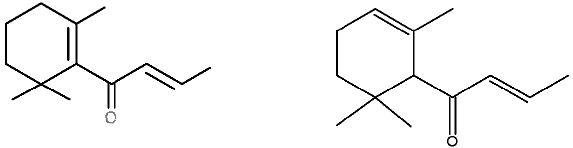 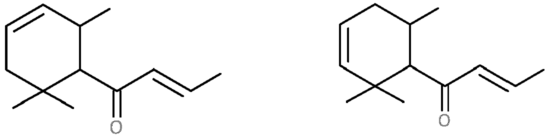 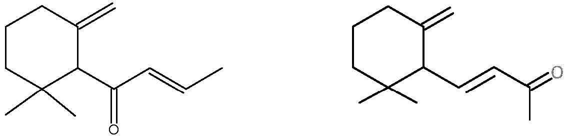 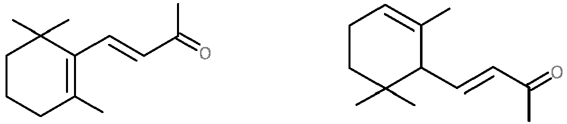 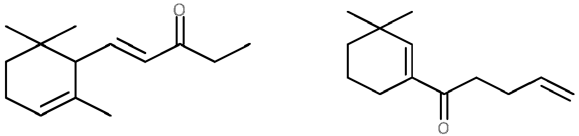 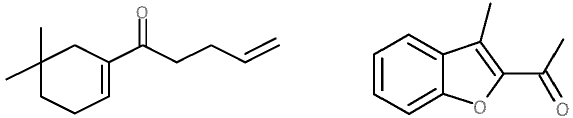 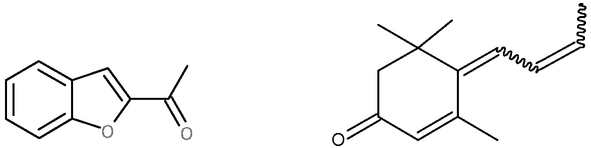 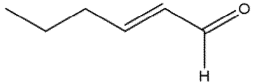 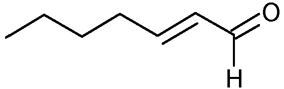 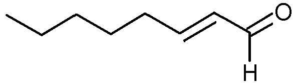 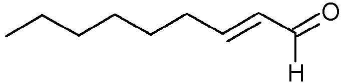 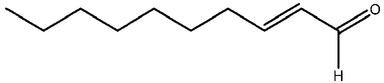 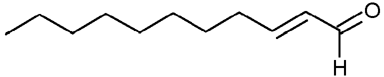 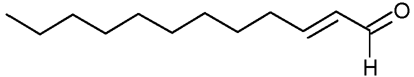 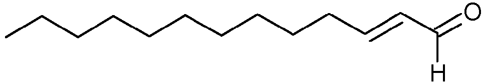 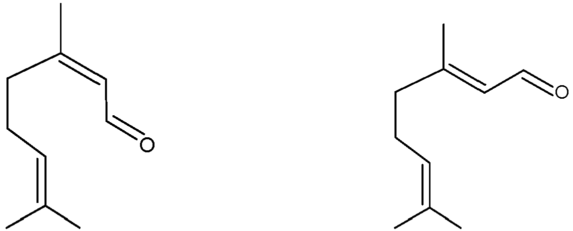 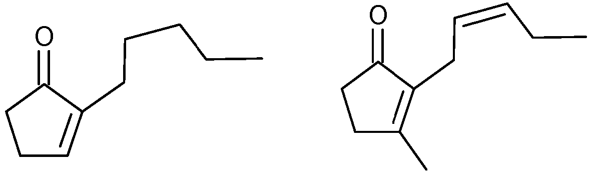 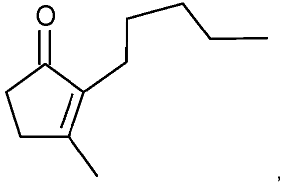 preferably wherein the thiol and the α,β-unsaturated aldehyde or ketone are released and/or sensorially perceived at the same or different, preferably different, point(s) in time.

Another aspect of the present invention relates to the use of a compound of formula (I), wherein residue 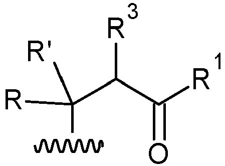 of the compound of formula (I) is selected from the group consisting of 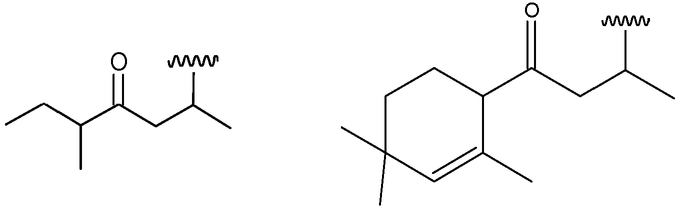 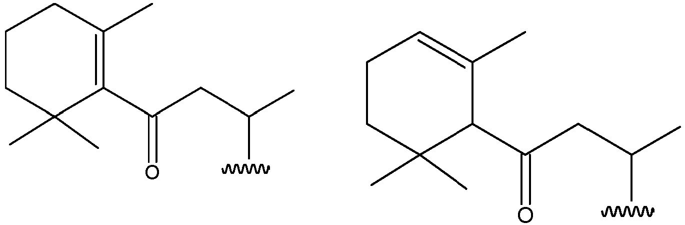 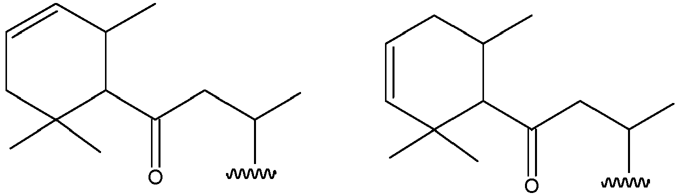 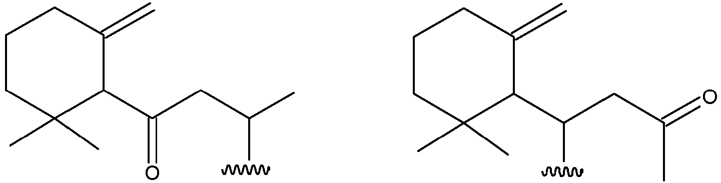 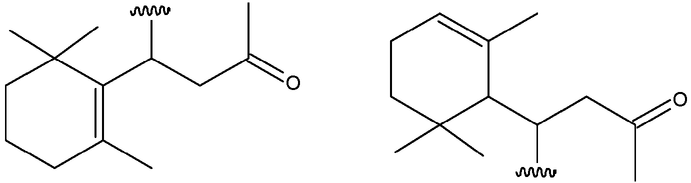 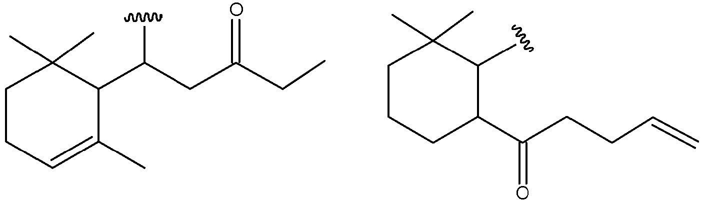 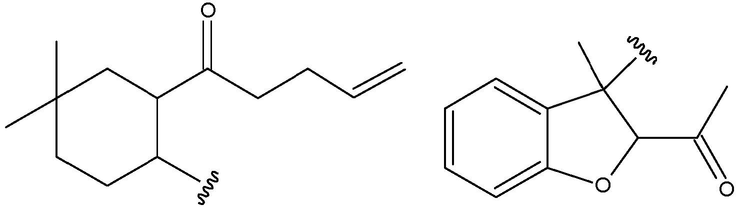 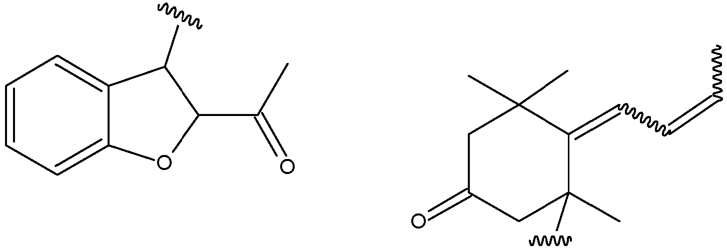 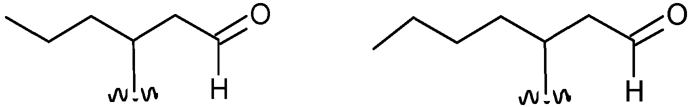 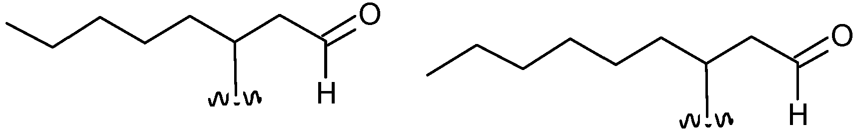 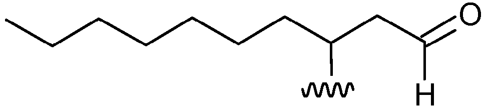 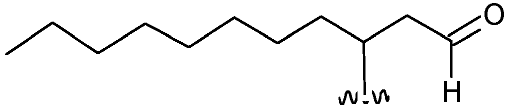 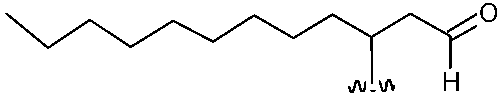 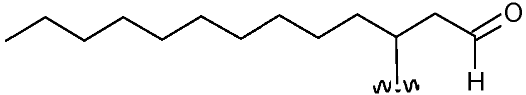 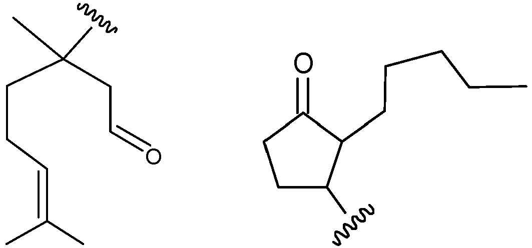 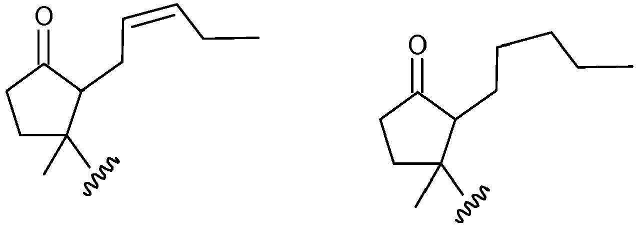 and/or, preferably and, wherein residue R² of the compound of formula (I) is selected from the group consisting of 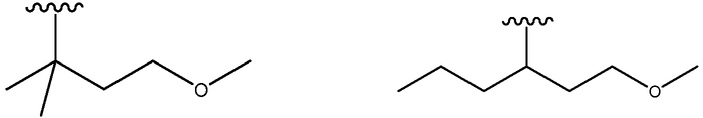 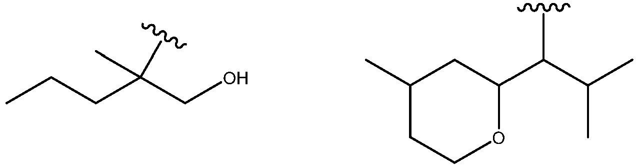 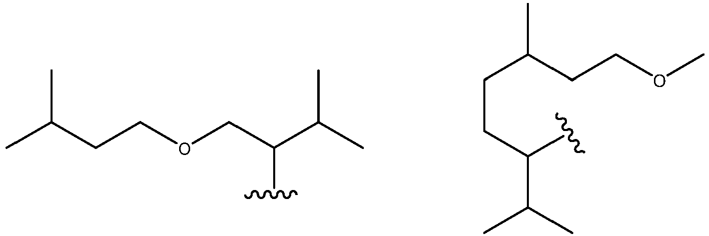 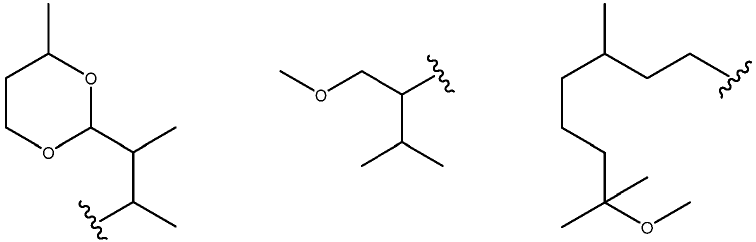 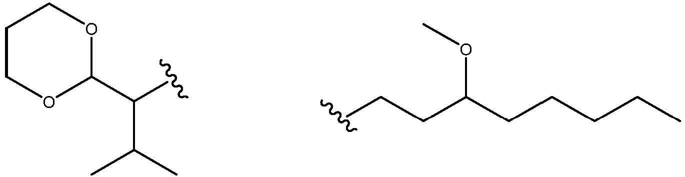 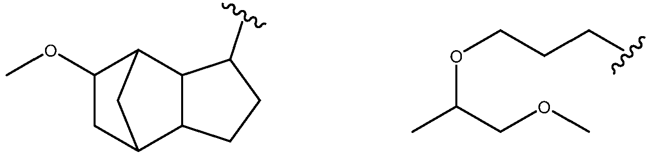 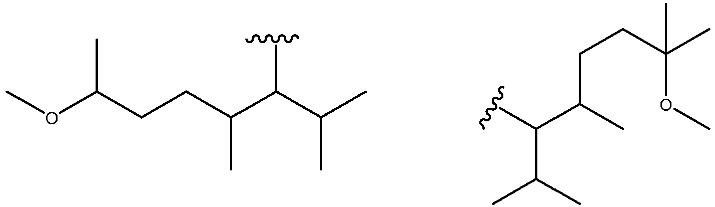 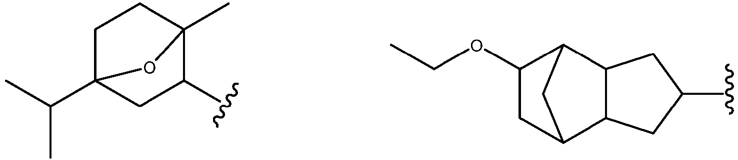 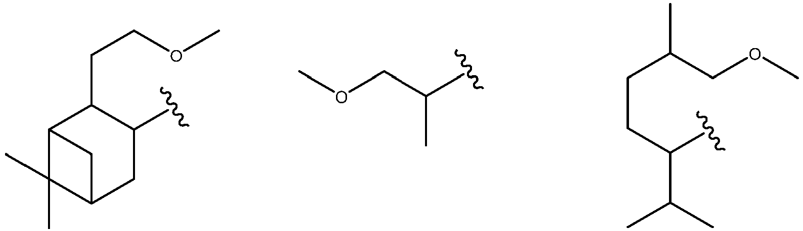 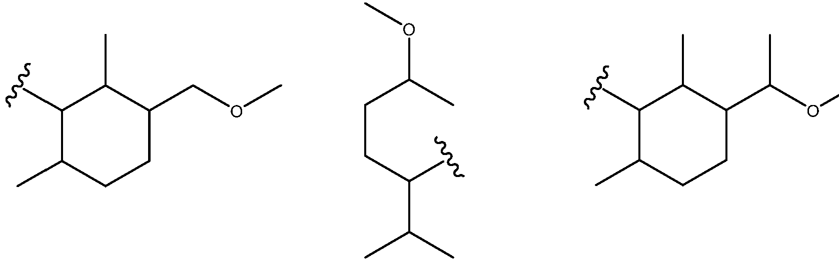 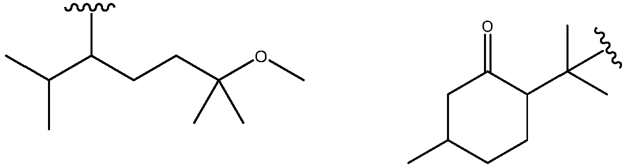 as a precursor for the (controlled) release of a (pleasantly smelling) thiol selected from the group consisting of 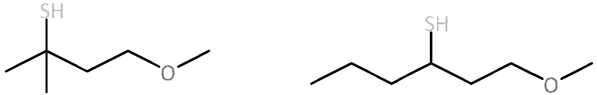 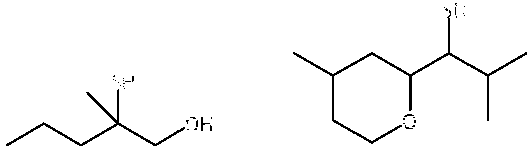 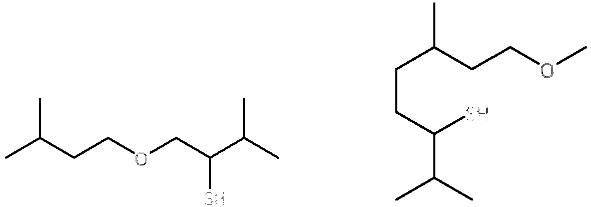 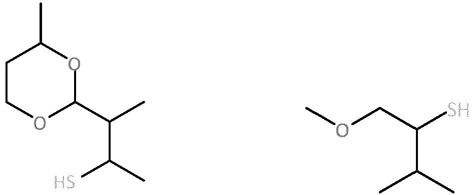 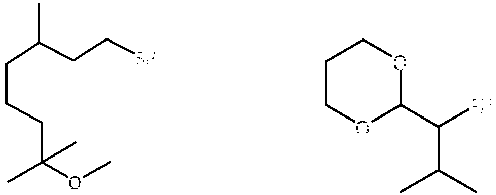 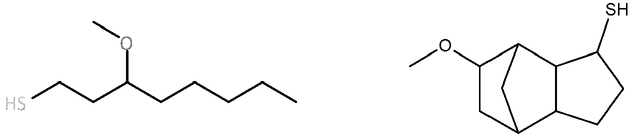 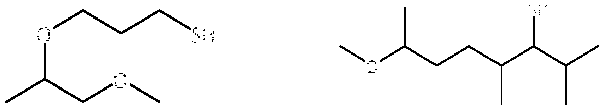 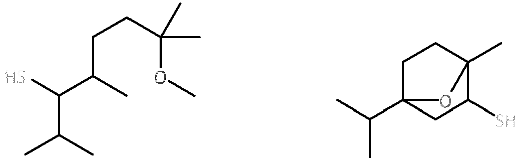 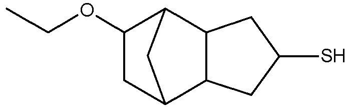 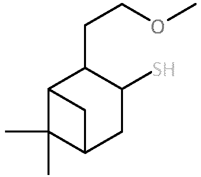 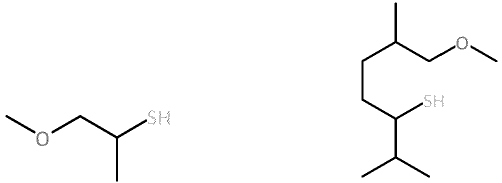 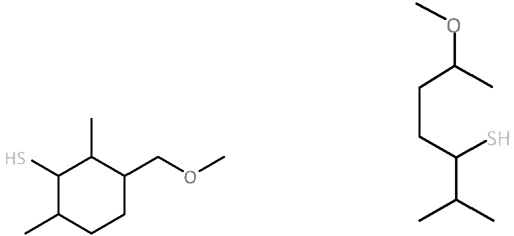 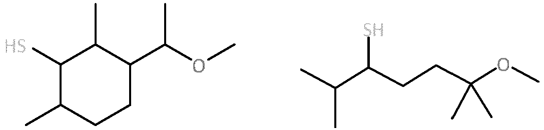 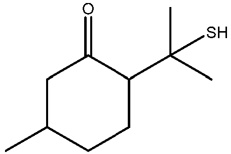 and/or, preferably and, of a (pleasantly smelling) α,β-unsaturated aldehyde or ketone selected from the group consisting of 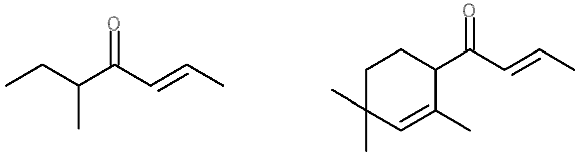 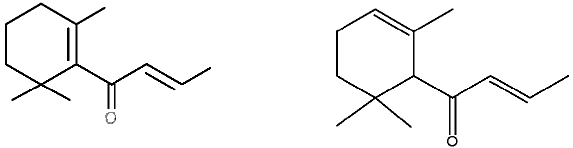 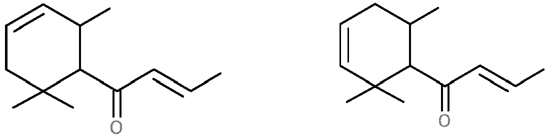 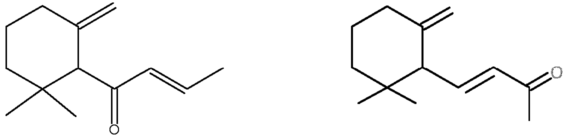 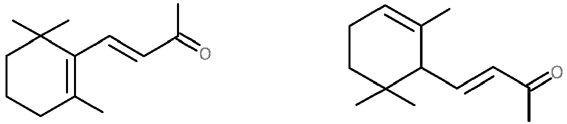 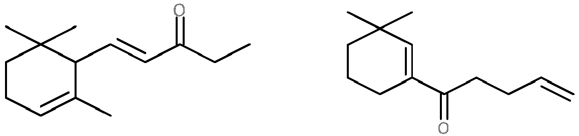 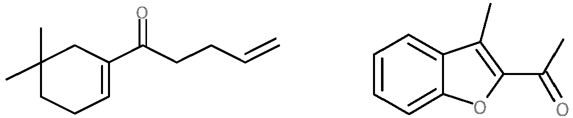 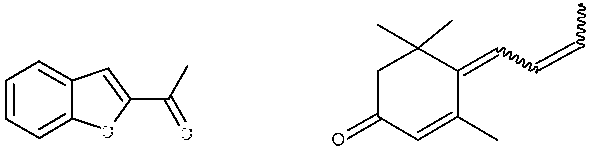 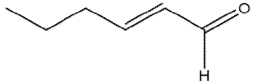 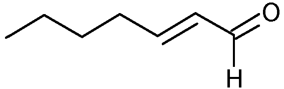 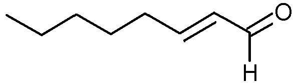 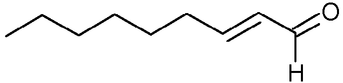 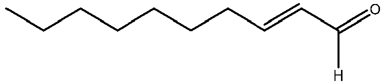 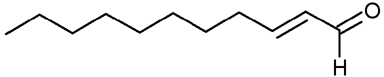 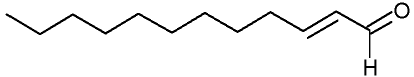 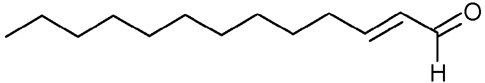 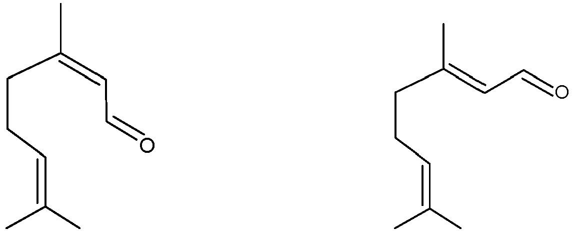 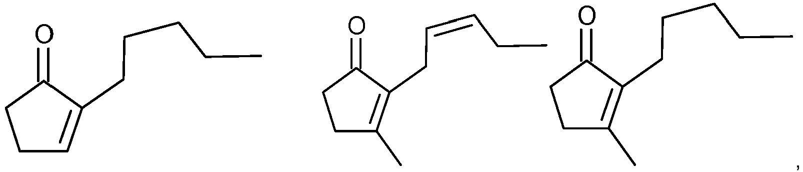 preferably wherein the thiol and the α,β-unsaturated aldehyde or ketone are released and/or sensorially perceived at the same or different, preferably different, point(s) in time.

The thiols and α,β-unsaturated aldehydes or ketones released by the compounds of formula (I) according to the invention and/or used for the formation of a compound of formula (I) according to the invention may be in the form of a pure diastereomer or stereoisomer or in the form of any mixture of said isomers.

According to a preferred embodiment of the use according to the invention, the thiol that is released from the compound of formula (I) as defined herein is released and/or sensorially perceived before the α,β-unsaturated aldehyde or ketone that is released from the (same) compound of formula (I) as defined herein.

According to an alternative embodiment of the use according to the invention, the α,β-unsaturated aldehyde or ketone that is released from the compound of formula (I) as defined herein is released and/or sensorially perceived before the thiol that is released from the (same) compound of formula (I) as defined herein.

According to a further alternative embodiment of the use according to the invention, the thiol that is released from the compound of formula (I) as defined herein and the α,β-unsaturated aldehyde or ketone that is released from the (same) compound of formula (I) as defined herein are released and/or sensorially perceived at the same time.

Preferably, a pleasantly smelling thiol has one or more odour notes selected from the group consisting of grapefruit, cassis, onion, fruity, exotic fruit, guava, breath of smoker, sulphur, (burnt) plastic, (minty) green, rose oxide, green pepper, liver sausage, buchu (Asian chives), gasoline, juicy, natural, cat grass, meat broth ("Fleischbrühe"), cheesy, camembert, cheese in the fridge, fresh, hot rubber, petrol, violet, blackcurrant, catnip (grass), zestral, anisic, mushroom, soup, leek, agrumen, peach, meat, pine, mango, durian, jackfruit, artichoke, and apricot,
more preferably a pleasantly smelling thiol has one or more odour notes selected from the group consisting of grapefruit, cassis, onion, fruity, exotic fruit, guava, sulphur, (minty) green, rose oxide, green pepper, liver sausage, buchu (Asian chives), juicy, natural, cat grass, meat broth ("Fleischbrühe"), cheesy, camembert, fresh, violet, blackcurrant, catnip (grass), zestral, anisic, mushroom, soup, leek, agrumen, peach, meat, pine, mango, durian, jackfruit, artichoke, and apricot.

Another aspect of the present invention relates to a method for the (controlled) release of one, two, three or more different pleasantly smelling fragrance substances comprising or consisting of the following steps:
(a) Providing a compound of formula (I) as defined herein or a fragrance substance mixture as defined herein (comprising one or more compound(s) of formula (I) as defined herein) or a perfumed product as defined herein (comprising one or more compound(s) of formula (I) as defined herein),
(b) optionally, applying or introducing the compound of formula (I) or the fragrance substance mixture or the perfumed product of step (a) to hair or skin or textile fibres or to a surface to be perfumed, or into ambient air to be perfumed, preferably in a sensorially effective amount, and
(c) releasing one, two, three or more different pleasantly smelling fragrance substances from the compound of formula (I), preferably wherein the two or more different pleasantly smelling fragrance substances are released and/or sensorially perceived at the same or different, preferably different, point(s) in time (if two or more different fragrance substances are released).

Another aspect of the present invention relates to a method for the (controlled) release of a pleasantly smelling thiol and of a pleasantly smelling α,β-unsaturated aldehyde or ketone comprising or consisting of the following steps:
(a) Providing a compound of formula (I) as defined herein or a fragrance substance mixture as defined herein (comprising one or more compound(s) of formula (I) as defined herein) or a perfumed product as defined herein (comprising one or more compound(s) of formula (I) as defined herein),
(b) optionally, applying or introducing the compound of formula (I) or the fragrance substance mixture or the perfumed product of step (a) to hair or skin or textile fibres or to a surface to be perfumed, or into ambient air to be perfumed, preferably in a sensorially effective amount,
(c) releasing the pleasantly smelling thiol and the pleasantly smelling α,β-unsaturated aldehyde or ketone from the compound of formula (I), preferably wherein the pleasantly smelling thiol and the pleasantly smelling α,β-unsaturated aldehyde or ketone are released and/or sensorially perceived at the same or different, preferably different, point(s) in time.

According to a preferred embodiment of the method according to the invention, the thiol that is released from the compound of formula (I) as defined herein in step (c) is released and/or sensorially perceived before the α,β-unsaturated aldehyde or ketone that is released from the (same) compound of formula (I) as defined herein in step (c).

According to an alternative embodiment of the method according to the invention, the α,β-unsaturated aldehyde or ketone that is released from the compound of formula (I) as defined herein in step (c) is released and/or sensorially perceived before the thiol that is released from the (same) compound of formula (I) as defined herein in step (c).

According to a further alternative embodiment of the method according to the invention, the thiol that is released from the compound of formula (I) as defined herein in step (c) and the α,β-unsaturated aldehyde or ketone that is released from the (same) compound of formula (I) as defined herein in step (c) are released and/or sensorially perceived at the same time.

Another aspect of the present invention relates to a method for the (controlled) release of a (pleasantly smelling) thiol selected from the group consisting of 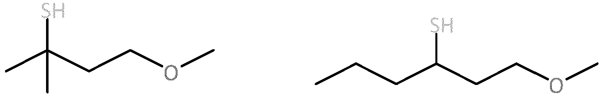 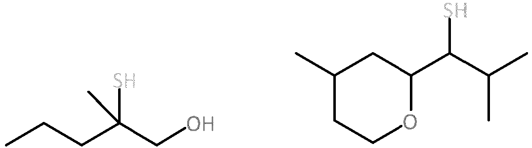 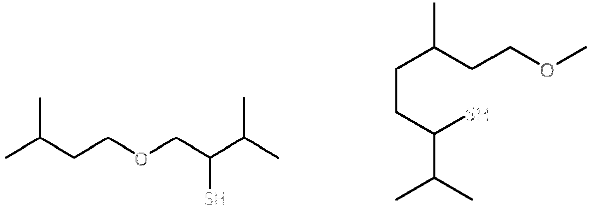 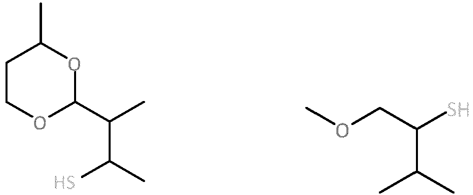 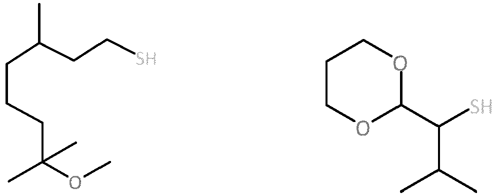 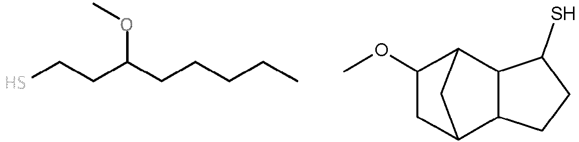 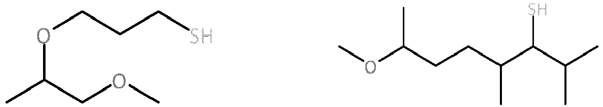 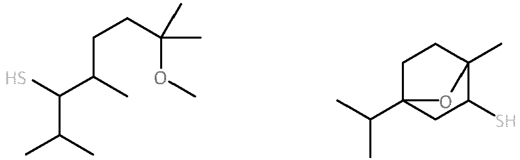 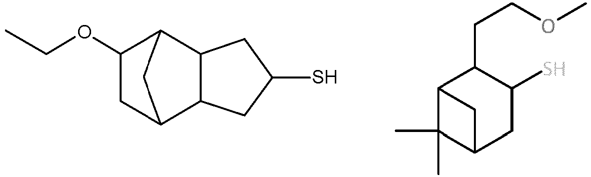 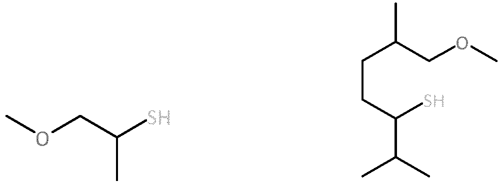 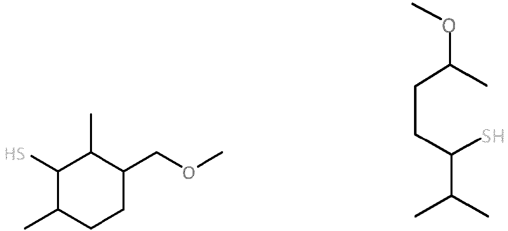 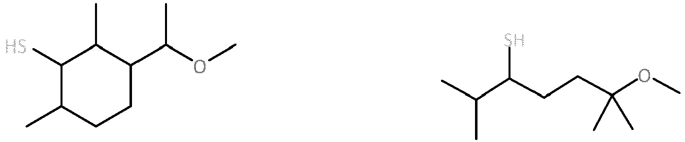 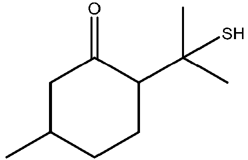 and/or, preferably and, of a (pleasantly smelling) α,β-unsaturated aldehyde or ketone selected from the group consisting of 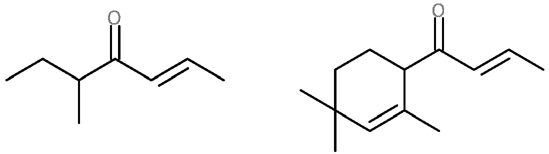 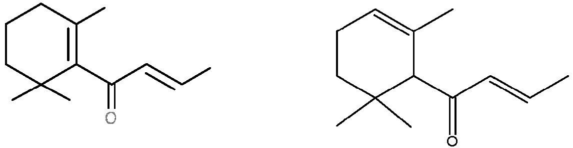 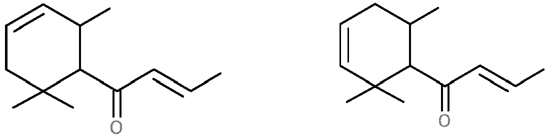 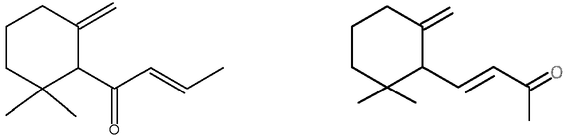 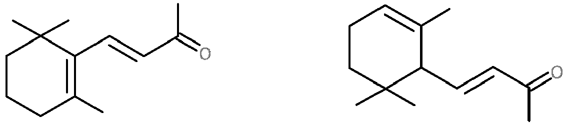 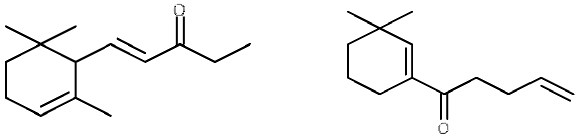 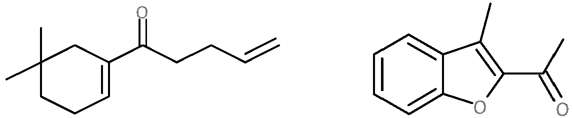 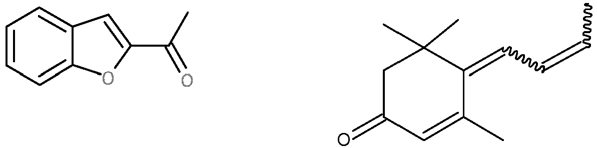 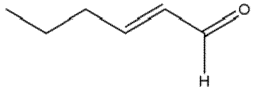 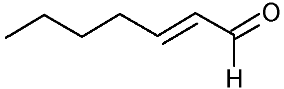 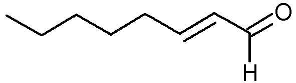 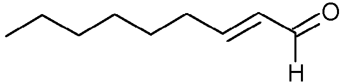 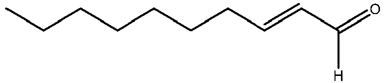 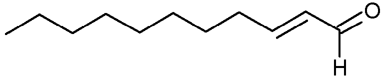 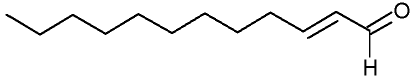 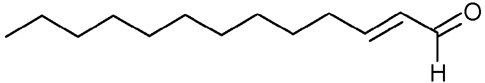 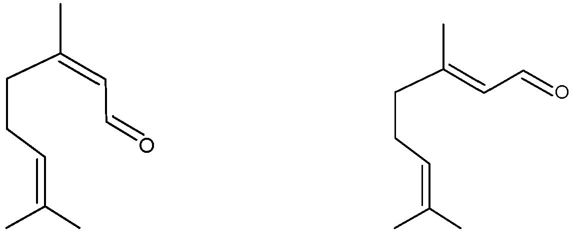 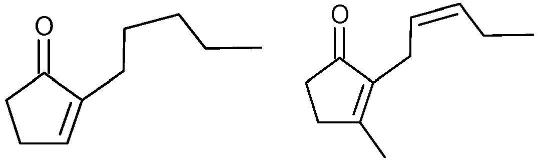 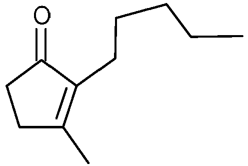 comprising or consisting of the following steps:
(a) Providing a compound of formula (I) (or a fragrance substance mixture or a perfumed product comprising a compound of formula (I) as defined above), wherein residue 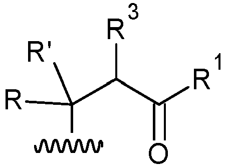 of the compound of formula (I) is selected from the group consisting of 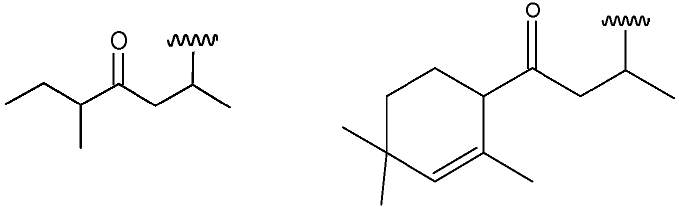 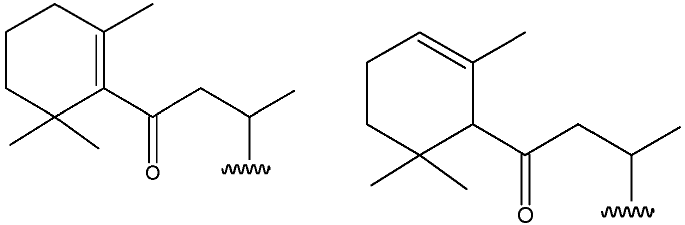 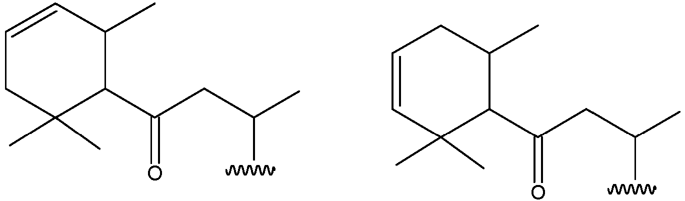 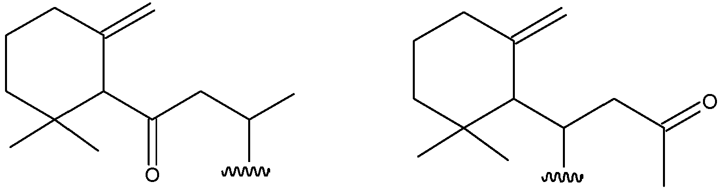 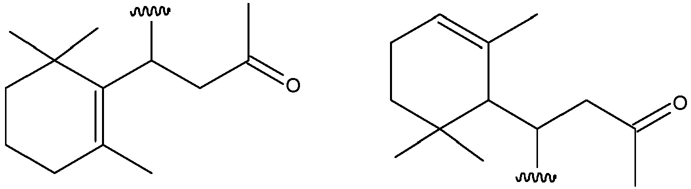 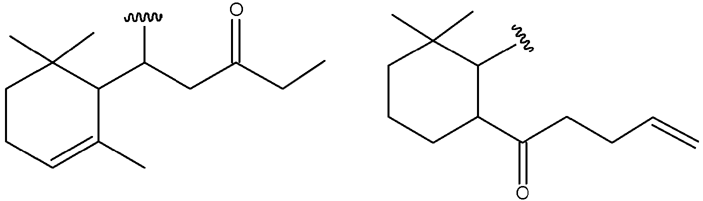 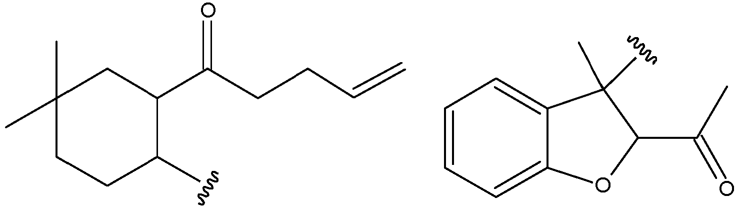 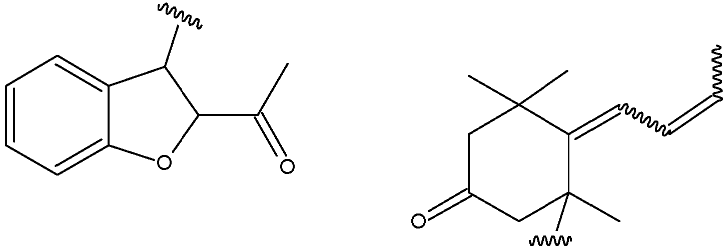 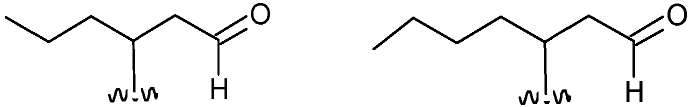 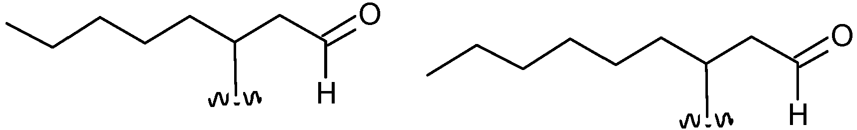 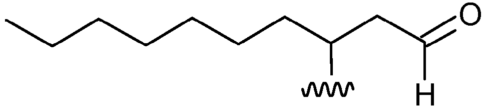 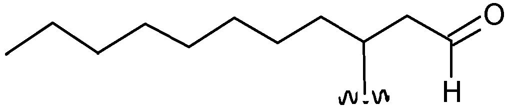 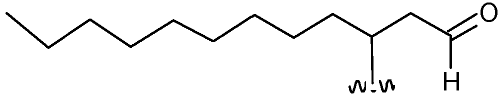 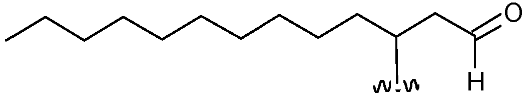 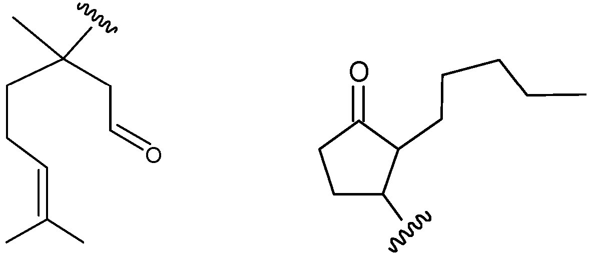 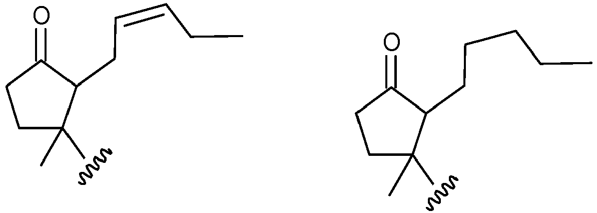 and/or, preferably and, wherein residue R² of the compound of formula (I) is selected from the group consisting of 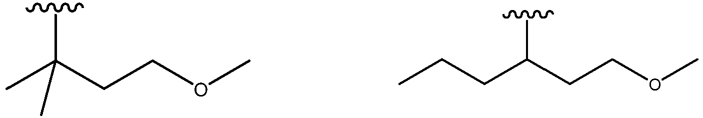 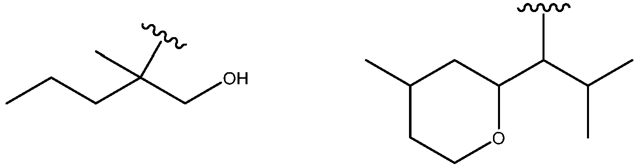 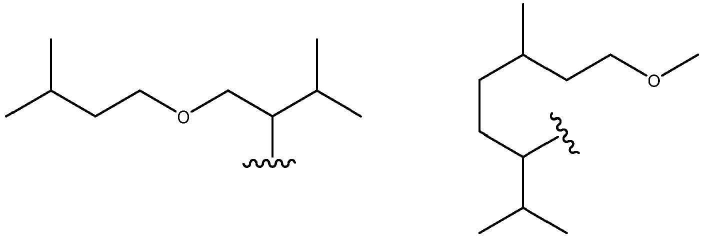 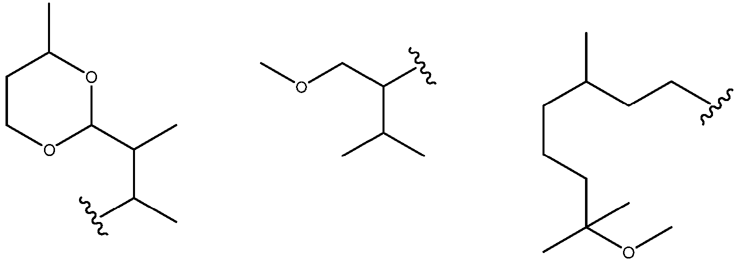 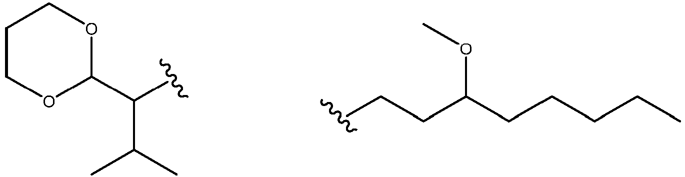 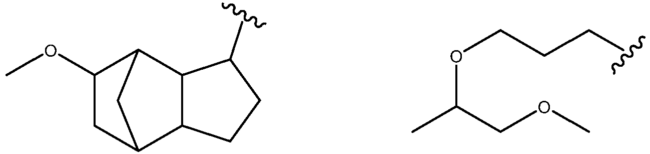 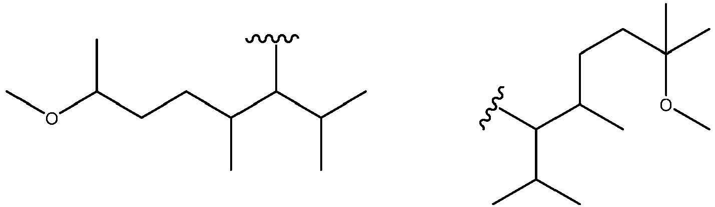 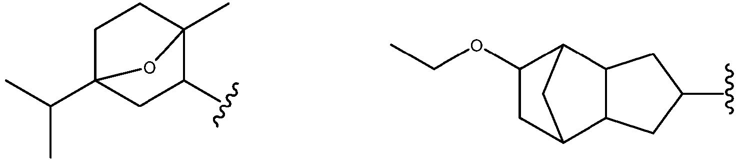 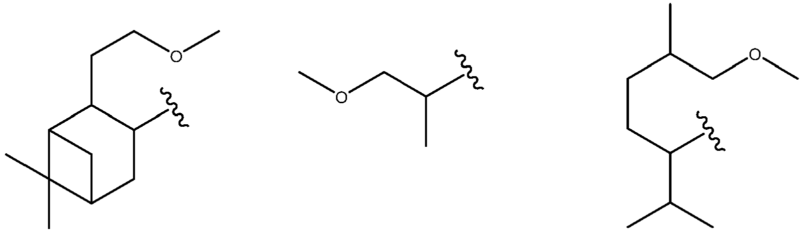 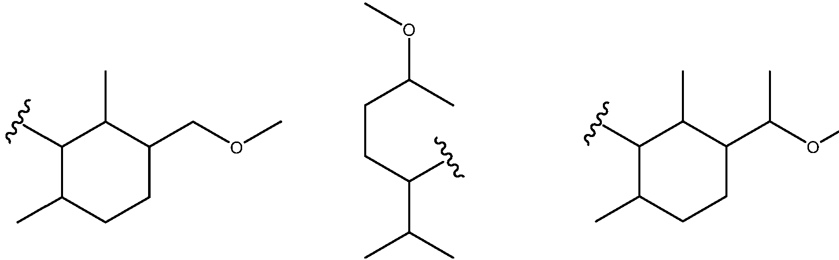 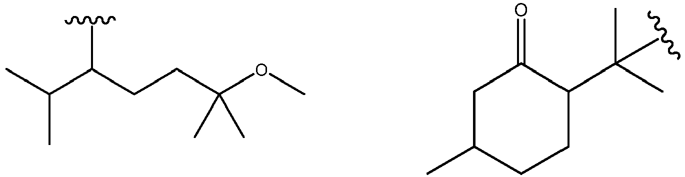
(b) optionally, applying or introducing the compound of formula (I) of step (a) (or the fragrance substance mixture or the perfumed product comprising a compound of formula (I) of step (a)) to hair or skin or textile fibres or to a surface to be perfumed, or into ambient air to be perfumed, preferably in a sensorially effective amount,
(c) releasing the (pleasantly smelling) thiol and/or the (pleasantly smelling) α,β-unsaturated aldehyde or ketone from the compound of formula (I), preferably wherein the (pleasantly smelling) thiol and the (pleasantly smelling) α,β-unsaturated aldehyde or ketone are released and/or sensorially perceived at the same or different, preferably different, point(s) in time.

Another aspect of the present invention relates to a thiol selected from the group consisting of

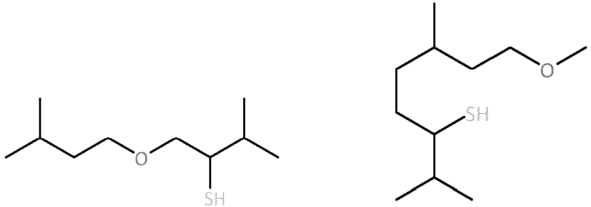

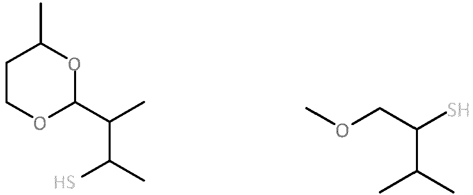

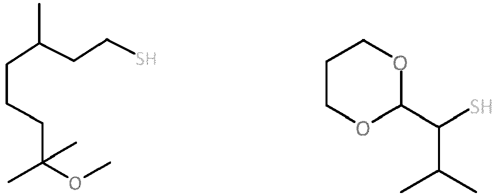

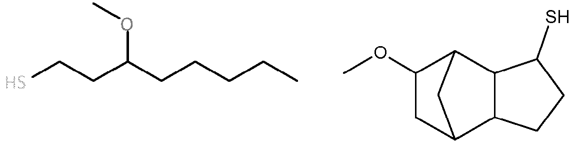

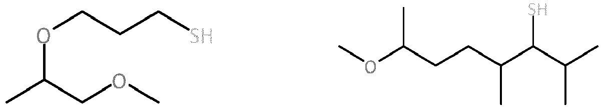

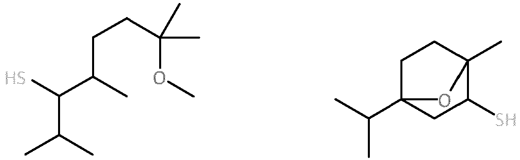

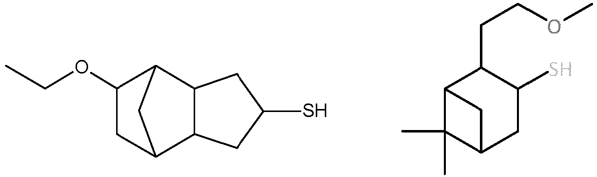

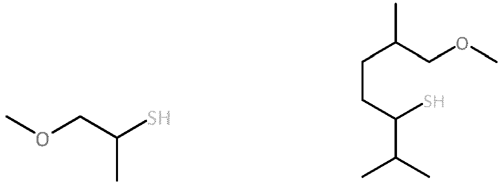

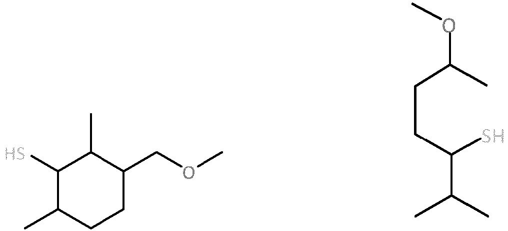

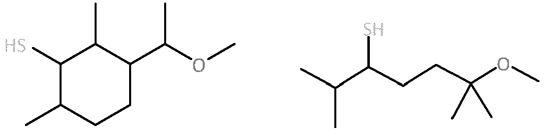

During the studies underlying the present invention, it was surprisingly found that the thiols as defined above can serve as pleasantly smelling, high impact fragrances, preferably after release from a compound of formula (I) according to the invention.

The thiols according to the invention can be obtained by applying the procedures disclosed in WO 2019/037862 A1.

An odour description of the thiols according to the invention can be found in **Table 2** above.

Another aspect of the present invention thus relates to the use of one or more thiol(s) according to the invention as a fragrance substance.

Another aspect of the present invention relates to a method for perfuming hair, skin, textile fibres, surfaces and/or ambient air comprising or consisting of the following steps:
(a) Providing
   (i) one or more thiol(s) as defined herein and preferably also a surfactant or a surfactant mixture, or
   (ii) one or more compound(s) of formula (I) as defined herein and preferably also a surfactant or a surfactant mixture, or
   (iii) a fragrance substance mixture as defined herein, preferably containing a surfactant or a surfactant mixture, or
   (iv) a perfumed product as defined herein, preferably containing a surfactant or a surfactant mixture,
   and
(b) applying or introducing the (i) thiol(s) and preferably also a surfactant or a surfactant mixture or (ii) compound(s) and preferably also a surfactant or a surfactant mixture or the (ii) fragrance substance mixture or the (iii) perfumed product of step (a) to the hair or skin or textile fibres or surface to be perfumed, or into the ambient air to be perfumed, in a sensorially effective amount.

Another aspect of the present invention relates to the use of a compound of formula (I) as defined herein or of a fragrance substance mixture, preferably perfume oil, as defined herein as a perfuming ingredient.

Another aspect of the present invention relates to the use a compound of formula (I) as defined herein or of a fragrance substance mixture, preferably perfume oil, as defined herein for perfuming hair, skin, textile fibres, surfaces and/or ambient air.

Another aspect of the present invention relates to a fragrance substance mixture, preferably perfume oil, as defined herein, wherein the amount of compound(s) of formula (I) is sufficient
(a) to mask or to reduce the or one or several unpleasant odour impressions of another fragrance substance in the fragrance substance mixture,
   and/or
(b) to enhance the or one or several pleasant odour impressions of another fragrance substance in the fragrance substance mixture.

Another aspect of the present invention relates to the use of one or more compound(s) of formula (I) as defined herein or of a fragrance substance mixture, preferably perfume oil, as defined herein
(a) for masking or reducing the or one or several unpleasant odour impressions of one or several unpleasantly smelling substances,
   and/or
(b) for enhancing the or one or several pleasant odour impressions of one or several pleasantly smelling substances.

Another aspect of the present invention relates to a method
(a) for masking or reducing the or one or several unpleasant odour impressions of one or several unpleasantly smelling substances,
   and/or
(b) for enhancing the or one or several pleasant odour impressions of one or several pleasantly smelling substances,
comprising the following step:
Mixing the unpleasantly and/or pleasantly smelling substances with one or more compound(s) of formula (I) as defined herein or with a fragrance substance mixture, preferably perfume oil, as defined herein, wherein the amount of the one or more compound(s) of formula (I) as defined herein or of the fragrance substance mixture, preferably perfume oil, as defined herein is sufficient to (a) reduce or to mask the unpleasant odour impression(s) of the unpleasantly smelling substance(s) and/or (b) enhance the pleasant odour impression(s) of the pleasantly smelling substance(s).

What has been stated herein in connection with the fragrance substance mixtures, perfumed products, methods, uses and thiols according to the invention applies accordingly to preferred embodiments of the compounds of formula (I) according to the invention, and *vice versa.*

In the following, the invention is explained in more detail using examples.

### Examples:

### Synthesis procedures of precursors according to the invention

### 1. Synthesis of precursor 3-[2-methyl-1-(4-methyltetrahydropyran-2-yl)propyl]sulfanyl-1-(2,6,6-trimethylcyclohex-3-en-1-yl)butan-1-one (compound of formula (I) according to the invention)

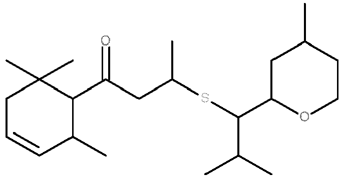

In a 500 ml stirring apparatus with magnetic stirrer, reflux condenser, thermometer, dropping funnel and ice bath, 5.8 g of 1,1,3,3-tetramethylguanidine, 9.4 g of 2-methyl-1-(4-methyltetrahydropyran-2-yl)propanethiol and 200 ml of tetrahydrofuran were added under a nitrogen atmosphere at 0°C. At 0-5°C, 8.1 g of delta-damascone were added dropwise within 1 hour. For post-reaction, stirring was continued for 6 hours at 0-5°C. The mixture was washed twice with saturated sodium bicarbonate solution at room temperature and then the solvents were evaporated on the rotary evaporator. 16.8 g crude yield of 3-[2-methyl-1-(4-methyltetrahydropyran-2-yl)propyl]sulfanyl-1-(2,6,6-trimethyl-cyclohex-3-en-1-yl)butan-1-one, with a GC content of 90%, were obtained. For further purification, the crude yield was chromatographed over a silica gel column with hexane:diethyl ether (10:1) as eluent. 4.0 g of 3-[2-methyl-1-(4-methyltetrahydropyran-2-yl)propyl]sulfanyl-1-(2,6,6-trimethylcyclohex-3-en-1-yl)butan-1-one, with a GC content of 94%, were obtained.

Mass spectrometric analysis of product:
m/z: 99 (100), 123 (35), 81 (35), 43 (33), 69 (27), 115 (24), 55 (18), 225 (17), 41 (17), 192 (16)

### 2. Synthesis of precursor 3-[2-methyl-1-(4-methyltetrahydropyran-2-yl)propyl]-sulfanyldecanal (compound of formula (I) according to the invention)

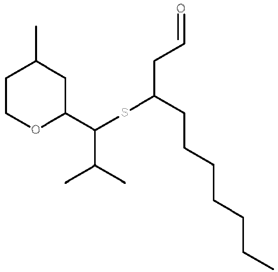

In a 500 ml stirring apparatus with magnetic stirrer, reflux condenser, thermometer, dropping funnel and ice bath, 5.8 g of 1,1,3,3-tetramethylguanidine, 9.4 g of 2-methyl-1-(4-methyltetrahydropyran-2-yl)propanethiol and 200 ml of tetrahydrofuran were placed under a nitrogen atmosphere at 0°C. At 0-5°C, 6.4 g of 2-(E)-decenal were added dropwise within 1 hour. For post-reaction, stirring was continued for 6 hours at 0-5°C. The mixture was washed twice with saturated sodium bicarbonate solution at room temperature and then the solvents were evaporated on the rotary evaporator. 14.6 g crude yield of 3-[2-methyl-1-(4-methyltetrahydropyran-2-yl)propyl]sulfanyldecanal, with a GC content of 76%, were obtained. For further purification, the crude yield was chromatographed over a silica gel column with hexane:diethyl ether (10:1) as eluent. 4.2 g of 3-[2-methyl-1-(4-methyltetrahydropyran-2-yl)propyl]sulfanyldecanal, with a GC content of 90%, were obtained.

Mass spectrometric analysis of product:
m/z: 99 (100), 43 (23), 55 (17), 81 (13), 41 (13), 69 (11), 57 (10), 98 (8), 29 (7), 100 (7)

### 3. Synthesis of precursor 3-[2-methyl-1-(4-methyltetrahydropyran-2-yl)propyl]sulfanylhexanal (compound of formula (I) according to the invention)

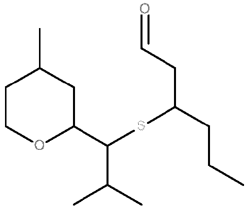

In a 500 mL stirring apparatus with magnetic stirrer, reflux condenser, thermometer, dropping funnel and ice bath, 12.4 g of 1,1,3,3-tetramethylguanidine, 20.4 g of 2-methyl-1-(4-methyltetrahydropyran-2-yl)propane-1-thiol and tetrahydrofuran (400 mL) were added under a nitrogen atmosphere at 0 °C. At 0-5 °C, 12.7 g of (*E*)-hex-2-enal were added dropwise within 1 hour. For post-reaction, stirring was continued for 6 hours at 0-5 °C. The mixture was washed twice with saturated sodium bicarbonate solution at room temperature and then the solvents were evaporated on the rotary evaporator. 30.9 g crude of 3-[2-methyl-1-(4-methyltetrahydropyran-2-yl)propyl]sulfanyl-hexanal were obtained. For further purification, the crude yield was chromatographed over a silica gel column with hexane:diethyl ether (10:1) as eluent. 26.8 g of 3-[2-methyl-1-(4-methyltetrahydropyran-2-yl)propyl]sulfanylhexanal, with a GC content of 90%, were obtained.

Mass spectrometric analysis of product:
m/z: 99 (100), 43 (24), 55 (15), 41 (13), 81 (12), 69 (12), 100 (6), 57 (6), 170 (6), 29 (5), 98 (5).

### 4. Synthesis of precursor 3-[2-methyl-1-(4-methyltetrahydropyran-2-yl)propyl]sulfanyl-1-(2,6,6-trimethyl-1-cyclohexen-1-yl)-3-butan-2-one (compound of formula (I) according to the invention)

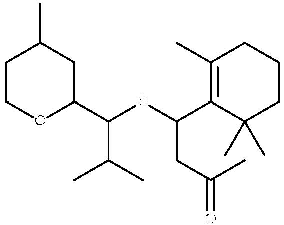

In a 50 mL stirring apparatus with magnetic stirrer, reflux condenser, thermometer, dropping funnel and ice bath, 0.02 g of 1,1,3,3-tetramethylguanidine, 14.1 g of 2-methyl-1-(4-methyltetrahydropyran-2-yl)propane-1-thiol and tetrahydrofuran (40 mL) were added under a nitrogen atmosphere at 0 °C. At 0-5 °C, 14.4 g of (E)-[4]-(2,6,6-trimethylcyclohex-1-en-1-yl)but-3-en-2-one were added dropwise within 1 hour. For post-reaction, stirring was continued for 6 hours at 0-5 °C. The mixture was washed twice with saturated sodium bicarbonate solution at room temperature and then the solvents were evaporated on the rotary evaporator. 28.5 g crude of 3-[2-methyl-1-(4-methyltetrahydropyran-2-yl)propyl]sulfanyl-1-(2,6,6-trimethyl-1-cyclohexen-1-yl)-3-butan-2-one were obtained. For further purification, the crude yield was chromatographed over a silica gel column with hexane:diethyl ether (10:1) as eluent. 18.9 g of 3-[2-methyl-1-(4-methyltetrahydropyran-2-yl)propyl]sulfanyl-1-(2,6,6-trimethyl-1-cyclohexen-1-yl)-3-butan-2-one, with a GC content of 90%, were obtained.

Mass spectrometric analysis of product:
m/z: 43 (100), 99 (78), 177 (35), 175 (33), 133 (29), 41 (29), 135 (27), 55 (24), 81 (19), 69 (18), 193 (15), 149 (15), 192 (12), 93 (12), 29 (11), 91 (11), 79 (10).

### 5. Synthesis of precursor 8-ethoxytricyclo[5.2.1.02,6]decane-4-sulfanyl-1-(2,6,6-trimethylcyclohex-3-en-1-yl)butan-1-one (compound of formula (I) according to the invention)

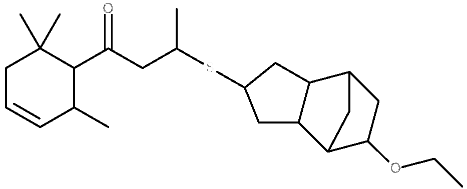

In a 50 mL stirring apparatus with magnetic stirrer, reflux condenser, thermometer, dropping funnel and ice bath, 0.02 g of 1,1,3,3-tetramethylguanidine, 16.4 g of 8-ethoxytricyclo[5.2.1.02,6]decane-4-thiol and tetrahydrofuran (40 mL) were added under a nitrogen atmosphere at 0 °C. At 0-5 °C, 14.4 g of (*E*)-[1]-(2,6,6-trimethylcyclohex-3-en-1-yl)but-2-en-1-one were added dropwise within 1 hours. For post-reaction, stirring was continued for 6 hours at 0-5 °C. The mixture was washed twice with saturated sodium bicarbonate solution at room temperature and then the solvents were evaporated on the rotary evaporator. 30.4 g crude of 8-ethoxytricyclo[5.2.1.02,6]decane-4-sulfanyl-1-(2,6,6-trimethylcyclohex-3-en-1-yl)butan-1-one were obtained.

Mass spectrometric analysis of product:
m/z: 133 (100), 67 (58), 123 (51), 91 (48), 81 (45), 239 (41), 79 (41), 107 (41), 41 (37), 69 (37), 29 (35), 105 (24), 103 (21), 55 (20), 43 (18), 122 (17), 77 (17).

### Synthesis procedures of thiols according to the invention

### 1. 2-Methyl-1-[4-methyltetrahydropyran-2-yl]propane-1-thiol

### 2-Methyl-1-[4-methyltetrahydropyran-2-yl]propane-1-thiol was prepared in accordance to WO 2019/037862 A1:

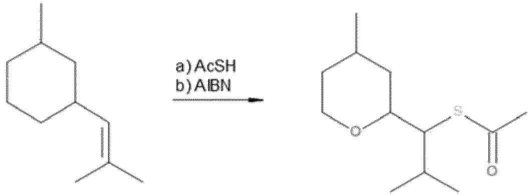

To 463 g 4-methyl-2-(2-methyl-prop-1-enyl)tetrahydropyran and 228 g thioacetic acid was added 2.46 g 2,2-azobis-(2-methyl-propionitril, AIBN) at 5-10 °C and stirred at room temperature for 30 min. Afterwards, the mixture is slowly heated to 60 °C and stirred for 10 h at 60 °C until complete conversion. After cooling to room temperature, the product is obtained by distillation (2.0 mbar, 104-110 °C) to yield 527.69 g (76%) of 2-methyl-(1-4-methyltetrahdyropyran-2-yl)propyl-ethanethioate as a mixture of isomers.

El-MS *m*/*z* (%): 231 (1, [M]⁺), 215 (1), 187 (1), 173 (1), 154 (4), 139 (4), 99 (100), 81 (15), 69 (12), 55 (12), 43 (42), 29 (4).

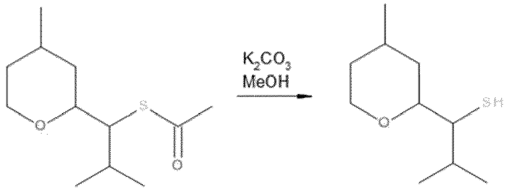

A solution of 501 g 2-methyl-(1-4-methyltetrahdyropyran-2-yl)propyl-ethanethioate in dry methanol (3.3 L) was treated with 300 g potassium carbonate und nitrogen and stirred over night until complete conversion. Afterwards, the mixture is acidified by 2 M hydrochloric acid (1 L) and 1 L H₂O and 1.5L Et₂O are added. The organic phase is separated and the aqueous phase is extracted with 500 mL Et₂O. The combined organic phases are washed with brine (500 mL), dried over sodium sulfate and reduced in vacuum. The crude product is purified by distillation (3.6 mbar, 99-102 °C) to obtain 372 g (91%) 2-methyl-1-[4-methyltetrahydropyran-2-yl]propane-1-thiol as a mixture of isomers.

EI-MS *m*/*z* (%): 188 (1, [M]⁺), 170 (1), 139 (1), 111 (1), 99 (100), 81 (19), 69 (12), 55 (19), 43 (22), 29 (5).

### 2. 8-Ethoxytricyclo[5.2.1.02,6]decane-4-thiol

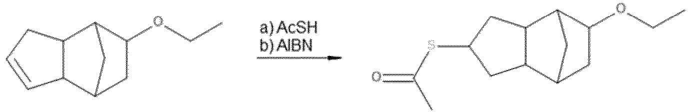

To 583 g 8-methoxytricyclo[5.2.1.02,6]dec-3-ene and 279 g thioacetic acid was added 2.91 g 2,2-azobis-(2-methyl-propionitril, AIBN) at 5-10 °C and stirred at room temperature for 30 min. Afterwards, the mixture is slowly heated to 60 °C and stirred for 10 h at 60 °C until complete conversion. After cooling to room temperature, the product is obtained by distillation (2.0 mbar, 104-110 °C) to yield 773 g (91%) of (8-methoxytricyclo-4-[5.2.1.02,6]decanyl)ethanethioate as a mixture of isomers.

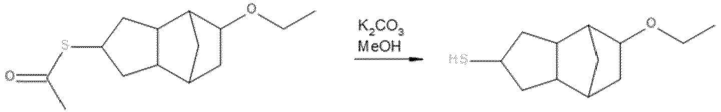

A solution of 538 g (8-methoxytricyclo-4-[5.2.1.02,6]decanyl)ethanethioate in dry methanol (3.3 L) was treated with 300 g potassium carbonate und nitrogen and stirred over night until complete conversion. The solvent is evaporated under reduced pressure and 400 mL MTBE is added. The organic phase is separated and the aqueous phase is extracted with 200 mL MTBE. The combined organic phases are washed with saturated sodium hydrogencarbonate solution, sodium sulfite solution and brine, dried over sodium sulfate and reduced under reduced pressure. The crude product is purified by distillation (3.6 mbar, 99-102 °C) to obtain 376 g (90%) 8-ethoxytricyclo[5.2.1.02,6]decane-4-thiol as a mixture of isomers.

Mass spectrometric analysis of product:
m/z: 166 (100), 91 (75), 92 (75), 133 (72), 79 (57), 132 (48), 105 (47), 117 (45), 93 (44), 67 (37), 106 (35), 212 (28), 77 (26), 29 (25), 41 (25), 66 (24), 137 (24).

### Products according to the invention

### 1. Fabric softener

**Table 4: Fabric softener formulation according to the invention**

| **Ingredients** | **Supplier** | **INCI Name / description** | **(w/w)** |
|---|---|---|---|
| Water, demin. | | Aqua | 86.60 |
| Rewoquat WE 18 | Evonik | Di-(tallow carboxyethyl) hydroxyethyl methylammonium methosulfate | 12.00 |
| Parmetol N 20 | Hoesch Julius | | 0.10 |
| Calcium Chloride 25 % solution | | Calcium Chloride | 0.40 |
| 3-[2-methyl-1-(4-methyltetrahydropyran-2-yl)propyl]sulfanyl-1-(2,6,6-trimethylcyclohex-3-en-1-yl)butan-1-one | | | 0.02 |
| Dipropylengylcol | Symrise | Dipropylengylcol, mixture of isomers | 0.88 |

### 2. Deodorant

**Table 5: Deodorant formulation according to the invention**

| **Ingredients** | **Supplier** | **INCI Name/description** | **(w/w)** |
|---|---|---|---|
| Propylenglycol 1-2 | Symrise | Propan-1,2-diol | 7.00 |
| Ethanol 96% | Symrise | Ethanol | 50.00 |
| Klucel HF | Uniqema | Hydrocxlpropyl cellulose | 0.80 |
| Water, demin | Symrise | Aqua | 39.65 |
| Deolite | Symrise | | 0.50 |
| Trilon B Liquid | BTC Europe GmbH | Na4-EDTA | 0.05 |
| Solubilizer | Symrise | PEG-40 hydrogenated Castor oil, Trideceth-9, Propyleneglycol, Water | 1.00 |
| 3-[2-methyl-1-(4-methyltetrahydropyran-2-yl)propyl]sulfanyl-1-(2,6,6-trimethylcyclohex-3-en-1-yl)butan-1-one | | | 0.02 |
| Dipropylengylcol | Symrise | Dipropylengylcol, mixture of isomers | 0.98 |

### 3. Shampoo

**Table 6: Shampoo formulation according to the invention**

| **Ingredients** | **Supplier** | **INCI Name/description** | **(w/w)** |
|---|---|---|---|
| Water, demin | | Aqua | 76.84 |
| Plantacare PS 10 | Kraemer & Martin GmbH | Sodium Lauryl Sulphate, Lauryl glucoside | 20.00 |
| Sodium Chloride | Symrise | Sodium Chloride | 2.00 |
| Citric Acid E 330 | Symrise | Citric Acid | 0.164 |
| Preservative Complex | Symrise | | 0.50 |
| 3-[2-methyl-1-(4-methyltetrahydropyran-2-yl)propyl]sulfanyl-1-(2,6,6-trimethylcyclohex-3-en-1-yl)butan-1-one | | | 0.08 |
| Dipropylengylcol | Symrise | Dipropylengylcol, mixture of isomers | 0.416 |

### Sensorial analyses

### 1. Headspace gas chromatographic / mass spectrometric analysis

### Application of precursor in fabric softener (hand wash):

1.0 g of a solution containing 1.0% compound of formula (I) according to the present invention in fabric softener (cf. **Table 7** below for composition of the fabric softener) was added to 1 I H₂O in a 2 I beaker and mixed for a short period of time to form a homogeneous solution.

**Table 7: Composition of the used fabric softener**

| **Ingredients** | **Supplier** | **INCI Name / description** | **(w/w)** |
|---|---|---|---|
| Water, demin. | | Aqua | 87.50 |
| Rewoquat WE 18 | Evonik | Di-(tallow carboxyethyl) hydroxyethyl methylammonium methosulfate | 12.00 |
| Parmetol N 20 | Hoesch Julius | Mixture of Bronopol and 2-octyl-2H-isothiazol-3-one | 0.10 |
| Calcium Chloride 25 % solution | | Calcium Chloride | 0.40 |

Five pieces of cotton fabric (16 x 17 cm, 25 g each) were added and stirred using a magnetic stirrer for 30 min at room temperature. Afterwards, the pieces of cotton fabric were individually wrung (approx. 60 g each) and dried on a laundry rack in a self-made closed drying chamber (143 l) at a constant air exchange and temperature of 25 °C. At defined points in time, volatiles were adsorbed on a Tenax^{®} cartridge for 2 min at a continuous flow of 500 ml/min by means of a Gerstel gas sampling system.

### Additional measurements were carried out in a self-made glass tube using two pieces of fabric after 1 hour of drying in the drying chamber. Two pieces of fabric were taken from the laundry rack and introduced into the glass tube (0.44 I) at room temperature. The volatiles were adsorbed at a continuous flow of air (500 ml/min) using a Gerstel gas sampling system, initially for 58 min on a waste Tenax^{®} cartridge and then for 2 min on a fresh Tenax^{®} cartridge. Sampling on a waste Tenax^{®} cartridge was then continued until, at defined points in time, samplings on fresh Tenax^{®} cartridges were carried out for 2 mins each after 1h, 2h, 3h, 4h, 5h, 6h, 7h, 8h, 24h, 48h, 72h. These cartridges were then desorbed on a Gerstel Thermal Desorption Unit at 260°C. The volatiles were injected into an Agilent 6890 gas chromatograph with a DB-1 MS column (30 m, 0.25 mm, 0.25 µm) coupled to an Agilent mass spectrometer 5973 N. The volatiles were eluted with a flow of He at 1.4 ml/min using a temperature gradient moving from 50°C (for 4 min) to 260 °C at 8 °C/min.

Headspace concentrations were calculated by external standard calibrations using different concentrations of the corresponding fragrance materials (0.1, 0.25, 0.5, 1.0, 2.5, 5.0 ng/l) in EtOH. The corresponding fragrance materials are the fragrance materials that are released from a specific precursor / compound of formula (I), i.e. the corresponding α,β-unsaturated carbonyl compounds and thiol compounds. Each calibration solution (1 µl) was injected onto a clean, empty cartridge which was desorbed and analyzed using the same conditions as described above.

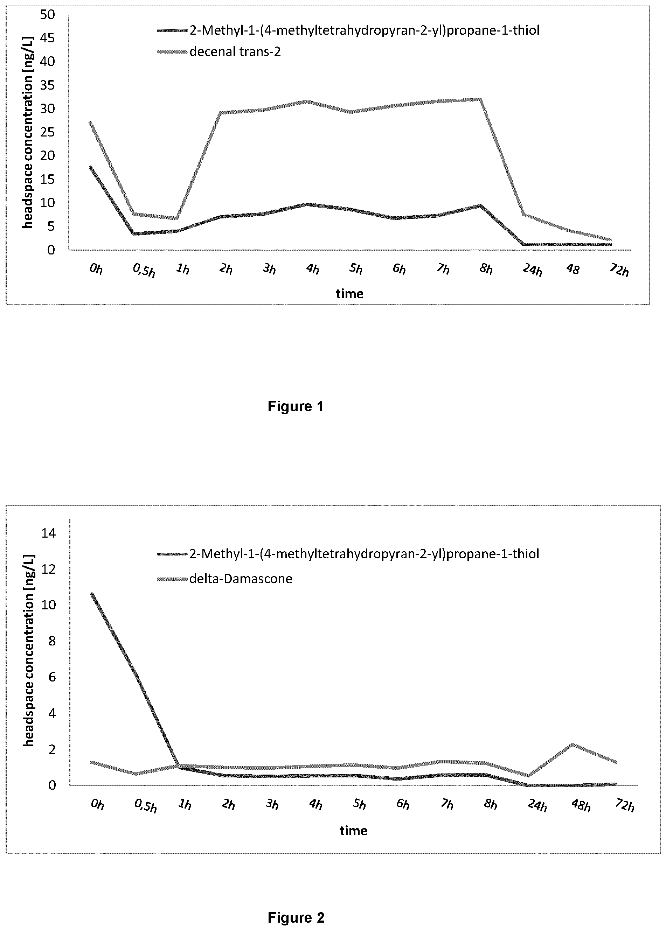

**Figure 1****:** Fragrance release curve for precursor 3-[2-methyl-1-(4-methyltetrahydropyran-2-yl)propyl]sulfanyldecanal (obtained according to procedure 2 as described above under synthesis procedures of precursors) as determined by procedure 1 as described above under sensorial analyses.

**Figure 2****:** Fragrance release curve for precursor 3-[2-methyl-1-(4-methyltetrahydropyran-2-yl)propyl]sulfanyl-1-(2,6,6-trimethylcyclohex-3-en-1-yl)butan-1-one (obtained according to procedure 1 as described above under synthesis procedures of precursors) as determined by procedure 1 as described above under sensorial analyses.

**Figures 1** and **2** show the release of 2-methyl-1-(4-methyltetrahydropyran-2-yl)propanethiol and the respective α,β-unsaturated carbonyl compound from the compounds of formula (I) obtained according to procedures 1 and 2 as described above under synthesis procedures of precursors. The x axis of the Figures shows the period of time over which the measurement was conducted. On the y axis, the headspace concentrations (determined according to procedure 1 as described above under sensorial analyses) of the two components released from the compound of formula (I) are shown, respectively. From the Figures it can be seen that on the freshly washed/wet laundry a strong release of the thiol compound occurs, which is also sensory perceptible. On the other hand, a noticeable release of the α,β-unsaturated carbonyl compound occurs with a time delay on dried laundry after several hours (2-decenal: 2 - 48 h, delta-damascone: 24 - 72 h). These analytical results demonstrate the (controlled) decomposition of the compounds of formula (I) according to the invention with simultaneous release of the α,β-unsaturated aldehyde or ketone over time and are in line with the sensory evaluation of the washing tests (cf. below). The controlled decomposition of the compound of formula (I) advantageously leads to a long-lasting perception of the α,β-unsaturated aldehyde or ketone on the dried laundry. Additionally, the high impact thiol compound improves the overall scent of the freshly washed laundry without unwanted or unpleasant off notes.

For comparison, the experiment was repeated, however instead of using the compounds of formula (I) according to the invention, 1.0 g of a mixture of α,β-unsaturated carbonyl compound (hexenal) and thiol (2-methyl-1-(4-methyltetrahydropyran-2-yl)propane-1-thiol) was employed.

**Table 8: Fragrance release for precursor 3-[2-methyl-1-(4-methyltetrahydropyran-2-yl)propyl]sulfanylhexanal (compound of formula (I) according to the invention) and a mixture of 34% hexenal and 66% 2-methyl-1-(4-methyltetrahydropyran-2-yl)propane-1-thiol.**

| Time | *c*(Hexenal) from compound of formula (I) [ng/L] | *c*(Hexenal) [ng/L] | *c*(Thiol) from compound of formula (I) [ng/L] | *c*(Thiol) [ng/L] |
|---|---|---|---|---|
| 0 h | 10.7 | 285.0 | 12.1 | 215.7 |
| 0.5 h | 12.6 | 0.5 | 19.1 | 7.5 |
| 1 h | 46.0 | 5.3 | 5.0 | 3.5 |
| 2 h | 67.1 | 13.5 | 3.2 | 2.2 |
| 3 h | 58.9 | 11.9 | 3.1 | 2.6 |
| 4 h | 69.5 | 11.4 | 3.3 | 2.1 |
| 5 h | 75.6 | 11.1 | 3.4 | 2.1 |
| 6 h | 70.2 | 11.7 | 3.6 | 2.1 |
| 7 h | 74.8 | 10.0 | 3.3 | 2.1 |
| 8 h | 62.1 | 10.2 | 3.1 | 2.1 |
| 24 h | 43.9 | 1 | 2.1 | 2.1 |
| 48 h | 13.9 | - | 2.2 | - |
| 72 h | 3.9 | 0.3 | 2.1 | 2.1 |

It can be seen that the fragrance release from the compound of formula (I) according to the invention is substantially different to the release from the mixture of α,β-unsaturated carbonyl compound and thiol, underlying that released fragrance materials are delivered from the partial degradation of the compound of formula (I) according to the invention over time. Unexpectedly, it is observed that the peak of thiol release is between 0-1 h, while the peak of release of hexenal is reached at 4-7 h, surprisingly revealing that the compounds of formula (I) according to the invention can deliver two pleasantly smelling fragrance materials at different points in time.

### 2. Washing tests

A sensory evaluation of the compounds of formula (I) according to the invention (obtained according to procedures 1 and 2 as described above under synthesis procedures of precursors) after a washing application was carried out as described below and delivered the test results as shown in **Table 9** below.
- Application of the respective compound of formula (I) according to the invention in perfume oil in a realistic in-use test
- Perfume oil dosage: 1% by weight in fabric softener (cf. **Table 7** above for composition of the fabric softener)
- Precursor dosage in perfume oil: 2% by weight
- Cotton fabric cloths were washed at 30°C in a washing machine using the short cycle program
- The sensory evaluation was carried out by a panel of 5 persons. The intensities given in **Table 9** on a scale of 0 (no odour) to 9 (very strong odour) are an average of three evaluations by three panelists, respectively. The odour descriptions given in **Table 9** are an average of five evaluations by five panelists, respectively.

**Table 9**

| | Benchmark: Perfume oil with 2% dipropylene glycol | Perfume oil with 2% 3-[2-methyl-1-(4-methyltetrahydropyran-2-yl)propyl]-sulfanyldecanal (releases 2-decenal as carbonyl compound) | Perfume oil with 2% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|---|
| Wet fabric - fresh | Floral, transparent, slight musky **5** | Strongest effect, strong sulfuric-fruity odour **7-8** | Pleasant, soft, slight sulfuric-fruity note **5-6** |
| 4 h | **3** | Same odour profile as benchmark **3** | Same odour profile as benchmark **3** |
| 24 h | **2** | Intense decenal (for 24 - 48 h) **4** | Intense damascone (for 24 - 120 h) **4-5** |
| 48 h | **2** | **3** | **5** |
| 120 h | **2** | **2** | **4** |

### 3. Fabric Softener

Perfumed fabric softener samples were prepared by mixing the appropriate amount of compound of formula (I) and dipropylene glycol (DPG) to the perfume oil and adding 1% of the resulting fragrance mixture to unperfumed fabric softener.

2 kg of cotton towels were washed in a European washing machine (Express 20 program, 20 °C, 800 rpm) using 30 g of perfumed fabric softener (cf. Table 7 for composition of the fabric softener). The fabrics were lined dried over night and put on a table for evaluation after 24 h, 48 h, and 72 h. The fabrics were evaluated by 12 trained panelists. Perfume intensities are evaluated on a scale from no odor (0) to strongest imaginable (10). Panelists were also asked to choose which sample is perceived in higher intensity.

It is concluded that the fragrance perception is significantly stronger using compound of formula (I) in comparison to an equivalent amount of delta-damascone. To underline this, one piece of cotton towel was analyzed by headspace GC/MS according to procedure 1 as described above under sensorial analyses, revealing a significantly higher headspace concentration of delta-damascone of the cotton towel washed with perfume oil including compound of formula (I).

**Table 10: Mean intensities**

| | Perfume oil containing 1% delta-damascone and 1% DPG | Perfume oil with 2% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 24 h | 2.14 | 2.94 |
| 48 h | 1.46 | 2.53 |
| 72 h | 1.03 | 2.37 |
| 168 h | 1.04 | 1.51 |

**Table 11: Probability of strongest sample**

| | Perfume oil containing 1% delta-damascone and 1% DPG | Perfume oil with 2% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 24 h | 0.31 | 0.62 |
| 48 h | 0.08 | 0.92 |
| 72 h | 0.15 | 0.85 |
| 168 h | 0.36 | 0.64 |

**Table 12: Composition of used perfume oil**

| | |
|---|---|
| AGRUMEX LC | 40 |
| ALDEHYD C14 SOG | 5 |
| ALLYLCYCLOHEXYLPROPIONAT | 5 |
| ALLYLHEPTYLAT | 5 |
| AMBROXIDE | 5 |
| CITRONELLOL 950 | 10 |
| CYCLAMENALDEHYD | 10 |
| CYCLOGALBANAT^{®} | 1 |
| DECALACTON GAMMA | 3 |
| DIHYDROMYRCENOL | 300 |
| DIMETHYLBENZYLCARBINYLACETAT | 10 |
| DYNASCONE 10% DPG | 5 |
| ETHYLENBRASSYLAT | 45 |
| ETHYLVANILLIN 10% DPG | 5 |
| FLORAZON | 1 |
| FLOWERPOOL 0.1% DPG | 5 |
| FREESIOL / CORPS 119 | 50 |
| GLOBALIDE^{®} | 60 |
| HERBAFLORAT | 40 |
| HERBYLPROPIONAT | 25 |
| HEXENOL CIS-3 10% DPG | 35 |
| HEXYLZIMTALDEHYD ALPHA | 140 |
| KRAUSEMINZOEL 65% AMERIK. | 5 |
| MANZANATE 10% DPG | 5 |
| MELONAL | 15 |
| NEROLIN YARA YARA KRIST. | 10 |
| ORANGENOEL TERPENE | 40 |
| PHENIRAT^{®} | 20 |
| STYROLYLACETAT | 5 |
| TERPINEOL ALPHA | 10 |
| TIMBEROL^{®} | 10 |
| VANILLE EXTRAKT BOURBON 10X FAIR 10% DPG | 15 |
| VELOUTONE 10% DPG | 15 |
| VERTOCITRAL | 5 |
| YSAMBER^{®} K | 20 |

Fragrance release for compound of formula (I) 3-[2-methyl-1-(4-methyltetrahydropyran-2-yl)propyl]-sulfanyl-1-(2,6,6-trimethylcyclohex-3-en-1-yl)butan-1-one and a mixture of 50% delta-damascone and 50% DPG:

**Table 13**

| Time | *c*(damascone) from compound of formula (I) [ng/L] | *c*(damascone) [ng/L] |
|---|---|---|
| 24 h | 1.2 | 0.2 |
| 48 h | 4.8 | 0.2 |
| 72 h | 6.7 | 0.2 |
| 168 h | 0.3 | 0.2 |

### 4. Deodorant

An artificial skin model was prepared using synthetic skin (Vitro-Skin^{®}, approx. 2.5 cm radius), fat, cellulose, and agar.

Perfumed deo samples were prepared by mixing the appropriate amount of compound of formula (I) and DPG to the perfume oil and adding 1% of the resulting fragrance mixture to unperfumed deo base.

100 mg of perfumed deo is added on artificial skin and evenly distributed for 15 s. Afterwards, the model is placed on a heating plate (43 °C) and evaluated after 0 h, 4 h, 24 h, and 48 h by 6 trained panelists. Perfume intensities are evaluated on a scale from no odor (0) to strongest imaginable (9).

It is concluded that the intensity of the fragrance is significantly stronger when applying a perfume oil including a compound of formula (I). Unexpectedly, it is revealed the perception of the individual precursor components (thiol, α,β-unsaturated carbonyl) occurs nonsimultaneously, as the thiol component is perceived at 0 h, giving a richer fruity profile, while delta-damascone is perceived after 4-48h without any perception of thiol notes.

**Table 14: Mean intensities**

| | Perfume oil containing 2% DPG | Perfume oil with 0.5% 3-[2-methyl-1-(4-methyltetrahydropyr an-2-yl)propyl]sulfanyl-1-(2,6,6-trimethylcyclohex-3-en-1-yl)butan-1-one (releases delta-damascone as carbonyl compound) | Perfume oil with 1% 3-[2-methyl-1-(4-methyltetrahydropyra n-2-yl)propyl]sulfanyl-1-(2,6,6-trimethylcyclohex-3-en-1-yl)butan-1-one (releases delta-damascone as carbonyl compound) | Perfume oil with 2% 3-[2-methyl-1-(4-methyltetrahydropyr an-2-yl)propyl]sulfanyl-1-(2,6,6-trimethylcyclohex-3-en-1-yl)butan-1-one (releases delta-damascone as carbonyl compound) |
|---|---|---|---|---|
| 0 h | 4.2 | 4.9 | 4.8 | 4.8 |
| 4 h | 3.2 | 4.3 | 4.6 | 4.7 |
| 24 h | 1.5 | 2.5 | 3.3 | 4 |
| 48 h | 1.6 | 2 | 3 | 3.3 |

Panelists were asked to describe the hedonic differences between the different samples.

**Table 15: Sensory descriptions by panelists**

| | Perfume oil containing 2% DPG | Perfume oil with 0.5% 3-[2-methyl-1-(4-methyltetrahydropyr an-2-yl)propyl]sulfanyl-1-(2,6,6-trimethylcyclohex-3-en-1-yl)butan-1-one (releases delta-damascone as carbonyl compound) | Perfume oil with 1% 3-[2-methyl-1-(4-methyltetrahydropyra n-2-yl)propyl]sulfanyl-1-(2,6,6-trimethylcyclohex-3-en-1-yl)butan-1-one (releases delta-damascone as carbonyl compound) | Perfume oil with 2% 3-[2-methyl-1-(4-methyltetrahydropyra n-2-yl)propyl]sulfanyl-1-(2,6,6-trimethylcyclohex-3-en-1-yl)butan-1-one (releases delta-damascone as carbonyl compound) |
|---|---|---|---|---|
| 0 h | | More floral-creamy, slight thiol note (not unpleasant) | More floral, fruity, slight thiol note (not unpleasant | More floral, fruity, slight thiol note (not unpleasant |
| 4 h | | Stronger, more floral, apple, fruitier, damascone | Stronger, more floral, apple, fruitier, damascone | Stronger, more floral, apple, fruitier, damascone |
| 24 h | | Fruity, apple, damascone | Fruity, apple, damascone | Fruity, apple, damascone |
| 48 h | | Slight damascone, slight plum | Damascone, fruity, plum | Damascone, fruity, dried plum |

**Table 16: Composition of the used perfume oil**

| | |
|---|---|
| AGRUMEX LC | 20 |
| ALDEHYD C 8 | 1.5 |
| ALDEHYD C11 UNDECYLENIC 10% DPG | 2 |
| ALDEHYD C12 LAURIN | 1 |
| ALDEHYD C18 SOG. | 3 |
| ALLYLCYCLOHEXYLPROPIONAT | 4 |
| AMAROCIT^{®} | 10 |
| BENZYLACETAT | 8 |
| BENZYLSALICYLAT | 15 |
| CITRAL 95 | 4 |
| CITRONELLOL 950 | 20 |
| CUMARIN | 3 |
| DECALACTON GAMMA | 13 |
| DIHYDROMYRCENYLACETAT | 23 |
| DIMETHYLBENZYLCARBINYLACETAT | 2 |
| DIPROPYLENGLYCOL | 183 |
| ETHYLENBRASSYLAT | 40 |
| ETHYLLINALOOL | 8 |
| EVERNYL 10% DPG | 5 |
| Ecomusk R | 10 |
| FLOROPAL | 3 |
| FLOROSA BM / PYRANOL | 22 |
| GERANIOL SUPRA | 15 |
| GERANYLACETAT PUR | 5 |
| GLOBALIDE^{®} | 15 |
| GRAPEFRUITOEL TERPENE | 80 |
| HELIONAL | 8 |
| HELIOTROPIN/PIPERONAL | 3 |
| HEXENOL CIS-3 | 0.5 |
| HEXENYLACETAT CIS-3 | 1 |
| HEXENYLSALICYLAT CIS-3 | 4 |
| HEXYLSALICYLAT | 25 |
| HEXYLZIMTALDEHYD ALPHA | 45 |
| INDOFLOR^{®} KRIST. 10% DPG | 2 |
| IONON ALPHA | 2 |
| ISO E SUPER | 35 |
| ISOPROPYLMETHYLBUTYRAT-2 | 1 |
| LILYBELLE^{®} 10% DPG | 3 |
| LINALOOL | 20 |
| LINALYLACETAT | 15 |
| MAJANTOL^{®} | 2 |
| METHYL IONONE 70 | 25 |
| ORANGENOEL ITAL.SUESS ENTF. | 60 |
| PETITGRAINOEL PARAG. BOLEADOR | 1 |
| PHENYLETHYLALKOHOL | 30 |
| PHENYLETHYLPHENYLACETAT | 3 |
| ROSAPHEN^{®} | 8 |
| STYROLYLACETAT | 12 |
| SYMFRESH^{®} NX | 150 |
| TERPINEOL REIN | 7 |
| THIOCINEOL 100P 1% DPG | 4 |
| VERTACETAL^{®} COEUR | 2 |
| VERTOCITRAL 10% DPG | 6 |

**Table 17: Composition of the used deodorant**

| **Ingredients** | **Supplier** | **INCI Name/description** | **(w/w)** |
|---|---|---|---|
| Propylenglycol 1-2 | Symrise | Propan-1,2-diol | 7.00 |
| Ethanol 96% | Symrise | Ethanol | 50.00 |
| Klucel HF | Uniqema | Hydrocxlpropyl cellulose | 0.80 |
| Water, demin | Symrise | Aqua | 39.65 |
| Deolite | Symrise | | 0.50 |
| Trilon B Liquid | BTC Europe GmbH | Na4-EDTA | 0.05 |
| Solubilizer | Symrise | PEG-40 hydrogenated Castor oil, Trideceth-9, Propyleneglycol, Water | 1.00 |

### 5. Shampoo

Perfumed shampoo samples were prepared by mixing the appropriate amount of compound of formula (I) and DPG to the perfume oil and adding 1% of the resulting fragrance mixture to unperfumed shampoo base.

One slightly wet hair swatch (2 g Caucasian hair) is treated with 1 g of perfumed shampoo and carefully washed for 15s. Afterwards, the swatch is left to stand for 30 s and rinsed with warm water (30 °C) for 15 s. Excess water is removed by passing the swatch between the fingers.

The hair swatches were lined dried and evaluated after 0 h, 4 h, and 24 h by 5 trained panelists. Perfume intensities are evaluated on a scale from no odor (0) to strongest imaginable (9).

**Table 18: Mean intensities**

| | Perfume oil containing 2% DPG | Perfume oil with 1% 3-[2-methyl-1-(4-methyltetrahydropyran-2-yl)propyl]sulfanyl-1- (2,6,6-trimethylcyclohex-3-en-1- yl)butan-1-one (releases delta-damascone as carbonyl compound) | Perfume oil with 2% 3-[2-methyl-1-(4-methyltetrahydropyran-2-yl)propyl]sulfanyl-1- (2,6,6-trimethylcyclohex-3-en-1- yl)butan-1-one (releases delta-damascone as carbonyl compound) |
|---|---|---|---|
| 0h | 5 | 5 | 4.6 |
| 4 h | 3.6 | 4.2 | 4 |
| 24 h | 0.7 | 1.3 | 2 |

Panelists were asked to describe the hedonic differences between the different samples.

**Table 19: Sensory descriptions by panelists**

| | Perfume oil containing 2% DPG | Perfume oil with 1% 3-[2-methyl-1-(4-methyltetrahydropyran-2-yl)propyl]sulfanyl-1- (2,6,6-trimethylcyclohex-3-en-1-yl)butan-1-one (releases delta-damascone as carbonyl compound) | Perfume oil with 2% 3-[2-methyl-1-(4-methyltetrahydropyran-2-yl)propyl]sulfanyl-1- (2,6,6-trimethylcyclohex-3-en-1-yl)butan-1-one (releases delta-damascone as carbonyl compound) |
|---|---|---|---|
| 0 h | | More creamy-caring | Slightly more fougere |
| 4 h | | Fruity, apple | Fruity, damascone |
| 24 h | | Fruity, apple | Fruity, fresh, creamier, damascone |

**Table 20: Composition of the used perfume oil**

| | |
|---|---|
| AGRUMEX LC | 100 |
| ALLYLAMYLGLYCOLAT | 10 |
| BEIFUSSOEL | 10 |
| BRAHMANOL^{®} | 25 |
| DIHYDROMYRCENOL | 200 |
| DIPROPYLENGLYCOL | 194 |
| ETHYLMALTOL 10% DPG | 12 |
| EVERNYL 10% DPG | 70 |
| FICHTENNADELOEL SIB.TYP | 15 |
| FRAMBINON^{®} 10% DPG | 10 |
| GERANIUMOEL MADAG. | 5 |
| HEXYLSALICYLAT | 119 |
| ISOBORNYLACETAT | 20 |
| LAVANDIN ESPIEUR CENSO | 60 |
| PATCHOULIOEL ENTF. | 50 |

**Table 21: Composition of the used shampoo**

| **Ingredients** | **Supplier** | **INCI Name/description** | **(w/w)** |
|---|---|---|---|
| Water, demin | Symrise | Aqua | 76.84 |
| Plantacare PS 10 | Kraemer & Martin GmbH | Sodium Lauryl Sulphate, Lauryl glucoside | 20.00 |
| Sodium Chloride | Symrise | Sodium Chloride | 2.00 |
| Citric Acid E 330 | Symrise | Citric Acid | 1.64 |
| Preservative Complex | Symrise | | 0.50 |

### 6. Deodorant

An artificial skin model was prepared using synthetic skin (Vitro-Skin^{®}, approx. 2.5 cm radius), fat, cellulose, and agar.

Perfumed deo samples were prepared by mixing the appropriate amount of precursor to the perfume oil and adding 1% of the resulting fragrance mixture to unperfumed deo base. 100 mg of perfumed deo is added on artificial skin and evenly distributed for 15 s. Afterwards, the model is placed on a heating plate (43 °C) and evaluated after 0 h, 4 h, 24 h, and 48 h by 12 trained panelists. Perfume intensities are evaluated on a scale from no odor (0) to strongest imaginable (9).

It is detected that the perfume oil containing compound of formula (I) according to the invention is perceived stronger compared to perfume oil containing precursor of the prior art (WO 2015/032885 A1) at all stages of evaluation, showing a clear benefit of the compounds of formula (I) according to the invention. Surprisingly, both the hedonic profile and the perceived intensity of the fragrance after 0 h are positively influenced by introduction of a pleasantly smelling thiol component, while the superior and long-lasting performance at 6-24 h is likely due to the lower evaporation profile/higher substantivity of the compound of formula (I) according to the invention.

**Table 22: Probability of strongest sample**

| | Perfume oil with 0.8% pentanethiol damascone (releases delta-damascone as carbonyl compound; cf. I-C5 of WO 2015/032885 A1) (not according to invention) | Perfume oil with 1% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 0 h | 0.19 | 0.81 |
| 4 h | 0.27 | 0.73 |
| 6 h | 0.31 | 0.69 |
| 24 h | 0.20 | 0.80 |

**Table 23: Mean intensities**

| | Perfume oil with 0.8% pentanethiol damascone (releases delta-damascone as carbonyl compound; cf. I-C5 of WO 2015/032885 A1) (not according to invention) | Perfume oil with 1% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 0 h | 5.1 | 5.9 |
| 4 h | 3.8 | 4.7 |
| 6 h | 3.2 | 3.8 |
| 24 h | 2.6 | 3.1 |

**Table 24: Probability of strongest sample**

| | Perfume oil with 1% 3-(dodecylthio)-1-(2,6,6-trimethyl-3-cyclohexen-1-yl)-1-butanone (releases delta-damascone as carbonyl compound) (not according to invention) | Perfume oil with 1% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 0 h | 0.46 | 0.54 |
| 4 h | 0.46 | 0.54 |
| 6 h | 0.45 | 0.55 |
| 24 h | 0.29 | 0.71 |
| 48 h | 0.27 | 0.73 |

**Table 25: Mean intensities**

| | Perfume oil with 1% 3-(dodecylthio)-1-(2,6,6-trimethyl-3-cyclohexen-1-yl)-1-butanone (releases delta-damascone as carbonyl compound) (not according to invention) | Perfume oil with 1% 3-[2-methyl-1-(4-methyltetrahydropyran-2-yl)propyl]sulfanyl-1- (2,6,6-trimethylcyclohex-3-en-1-yl)butan-1-one (releases delta-damascone as carbonyl compound) |
|---|---|---|
| 0 h | 5.2 | 5.3 |
| 4 h | 4.1 | 4.2 |
| 6 h | 3.5 | 4.0 |
| 24 h | 3 | 3.3 |
| 48 h | 2.1 | 2.8 |

**Table 26: Composition of used perfume oil**

| | |
|---|---|
| AGRUMEX LC | 20 |
| ALDEHYD C 8 | 1,5 |
| ALDEHYD C11 UNDECYLENIC 10% DPG | 2 |
| ALDEHYD C12 LAURIN | 1 |
| ALDEHYD C18 SOG. | 3 |
| ALLYLCYCLOHEXYLPROPIONAT | 4 |
| AMAROCIT^{®} | 10 |
| BENZYLACETAT | 8 |
| BENZYLSALICYLAT | 15 |
| CITRAL 95 | 4 |
| CITRONELLOL 950 | 20 |
| CUMARIN | 3 |
| DECALACTON GAMMA | 13 |
| DIHYDROMYRCENYLACETAT | 23 |
| DIMETHYLBENZYLCARBINYLACETAT | 2 |
| DIPROPYLENGLYCOL | 183 |
| ETHYLENBRASSYLAT | 40 |
| ETHYLLINALOOL | 8 |
| EVERNYL 10% DPG | 5 |
| Ecomusk R | 10 |
| FLOROPAL | 3 |
| FLOROSA BM / PYRANOL | 22 |
| GERANIOL SUPRA | 15 |
| GERANYLACETAT PUR | 5 |
| GLOBALIDE^{®} | 15 |
| GRAPEFRUITOEL TERPENE | 80 |
| HELIONAL | 8 |
| HELIOTROPIN/PIPERONAL | 3 |
| HEXENOL CIS-3 | 0,5 |
| HEXENYLACETAT CIS-3 | 1 |
| HEXENYLSALICYLAT CIS-3 | 4 |
| HEXYLSALICYLAT | 25 |
| HEXYLZIMTALDEHYD ALPHA | 45 |
| INDOFLOR^{®} KRIST. 10% DPG | 2 |
| IONON ALPHA | 2 |
| ISO E SUPER | 35 |
| ISOPROPYLMETHYLBUTYRAT-2 | 1 |
| LILYBELLE^{®} 10% DPG | 3 |
| LINALOOL | 20 |
| LINALYLACETAT | 15 |
| MAJANTOL^{®} | 2 |
| METHYL IONONE 70 | 25 |
| ORANGENOEL ITAL.SUESS ENTF. | 60 |
| PETITGRAINOEL PARAG. BOLEADOR | 1 |
| PHENYLETHYLALKOHOL | 30 |
| PHENYLETHYLPHENYLACETAT | 3 |
| ROSAPHEN^{®} | 8 |
| STYROLYLACETAT | 12 |
| SYMFRESH^{®} NX | 150 |
| TERPINEOL REIN | 7 |
| THIOCINEOL 100P 1% DPG | 4 |
| VERTACETAL^{®} COEUR | 2 |
| VERTOCITRAL 10% DPG | 6 |

### 7. Fabric Softener

Perfumed fabric softener samples were prepared by mixing the appropriate amount of precursor to the perfume oil and adding 1% of the resulting fragrance mixture to unperfumed fabric softener.

2 kg of cotton towels were washed in a European washing machine (Express 20 program, 20 °C, 800 rpm) using 30 g of perfumed fabric softener (cf. Table 7 for composition of the fabric softener). The fabrics were lined dried over night and put on a table for evaluation after 0 h, 24 h, 48 h, 72 h, and 144 h. The fabrics were evaluated by 15 trained panelists. Perfume intensities are evaluated on a scale from no odor (0) to strongest imaginable (10). Panelists were also asked to choose which sample is perceived in higher intensity.

It is detected that the perfume oil containing compound of formula (I) according to the invention is perceived stronger compared to precursor of the prior art (WO 2015/032885 A1) at all stages of evaluation, showing a clear benefit of the present system. Surprisingly, both the hedonic profile and the perceived intensity of the fragrance after 0 h are positively influenced by introduction of a pleasantly smelling thiol component, while the superior, long-lasting performance at 24-48 h is likely due to the lower evaporation profile/higher substantivity of the compound of formula (I) according to the invention.

**Table 27: Probability of strongest sample**

| | Perfume oil with 0.8% pentanethiol damascone (releases delta-damascone as carbonyl compound; cf. I-C5 of WO 2015/032885 A1) (not according to invention) | Perfume oil with 1% 3-[2-methyl-1-(4-methyltetrahydropyran-2-yl)propyl]sulfanyl-1-(2,6,6-trimethylcyclohex-3-en-1-yl)butan-1-one (releases delta-damascone as carbonyl compound) |
|---|---|---|
| 0 h | 0.46 | 0.54 |
| 24 h | 0.07 | 0.93 |
| 48 h | 0.12 | 0.88 |
| 72 h | 0.45 | 0.55 |
| 144 h | 0.57 | 0.43 |

**Table 28: Mean intensities**

| | Perfume oil with 0.8% pentanethiol damascone (releases delta-damascone as carbonyl compound; cf. I-C5 of WO 2015/032885 A1) (not according to invention) | Perfume oil with 1% 3-[2-methyl-1-(4-methyltetrahydropyran-2-yl)propyl]sulfanyl-1- (2,6,6-trimethylcyclohex-3-en-1-yl)butan-1-one (releases delta-damascone as carbonyl compound) |
|---|---|---|
| 1 h | 4.70 | 5.00 |
| 24 h | 2.1 | 3.2 |
| 48 h | 1.8 | 2.6 |
| 72 h | 1.7 | 1.7 |
| 144 h | 1.5 | 1.7 |

**Table 29: Composition of the used perfume oil**

| | |
|---|---|
| AGRUMEX LC | 20 |
| ALDEHYD C14 SOG. | 10 |
| ALLYLPHENOXYACETAT | 40 |
| BENZYLACETAT | 60 |
| BERGAMOT SYNTH. AFRIC.W/O MYRCENE | 5 |
| CITRONELLOL 950 | 135 |
| CYCLAMENALDEHYD | 100 |
| DAMASCON ALPHA 10% DPG | 5 |
| DIHYDROMYRCENOL | 25 |
| DIMETHYLBENZYLCARBINYLACETAT | 30 |
| DIPROPYLENGLYCOL | 10 |
| FRAMBINON^{®} 10% DPG | 10 |
| HEDION | 10 |
| HERBAFLORAT | 100 |
| HEXYLZIMTALDEHYD ALPHA | 35 |
| ISOBORNYLACETAT | 25 |
| ISOMUSCONE^{®} | 25 |
| LAVANDIN GROSSO RCO | 35 |
| LINALOOL | 40 |
| LINALYLACETAT | 10 |
| MANZANATE | 5 |
| MENTHANYLACETAT | 40 |
| METHYL IONONE 70 | 25 |
| MUSCENONE | 40 |
| PHENYLACETALDEHYD DIMETHYLACETAL | 5 |
| PHENYLETHYLACETAT | 5 |
| PHENYLPROPYLALKOHOL | 5 |
| ROSACETAT | 50 |
| TERPINEOL REIN | 65 |
| TIMBERSILK | 30 |

## Claims

1. Compound of formula (I) 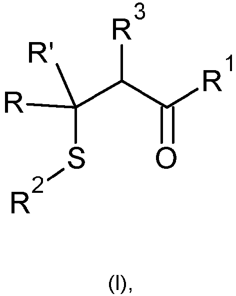
(i) wherein R is selected from the group consisting of hydrogen and C₁ to C₁₀ linear or cyclic alkyl, alkenyl or alkadienyl residues, preferably wherein the residues additionally carry one, two or more C₁ to C₄ linear, cyclic or branched alkyl, alkenyl or alkadienyl substituents, and
wherein R' is selected from the group consisting of hydrogen and C₁ to C₁₀ linear or cyclic alkyl, alkenyl or alkadienyl residues, preferably wherein the residues additionally carry one, two or more C₁ to C₄ linear, cyclic or branched alkyl, alkenyl or alkadienyl substituents, and
wherein R¹ is selected from the group consisting of hydrogen and C₁ to C₁₀ linear or cyclic alkyl, alkenyl or alkadienyl residues, preferably wherein the residues additionally carry one, two or more C₁ to C₄ linear, cyclic or branched alkyl, alkenyl or alkadienyl substituents, and
wherein R² is selected from the group consisting of 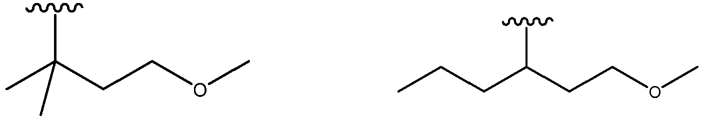 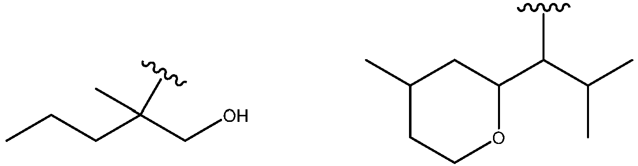 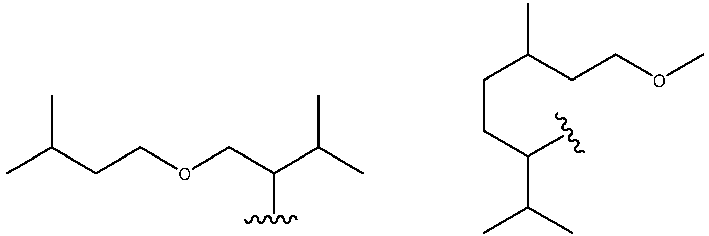 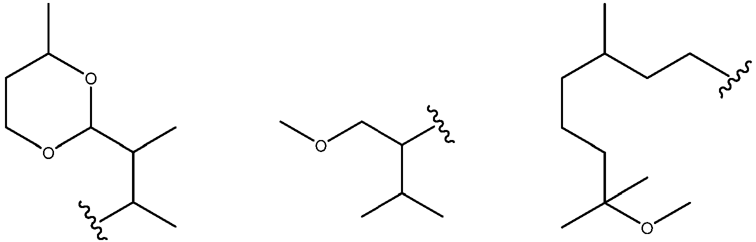 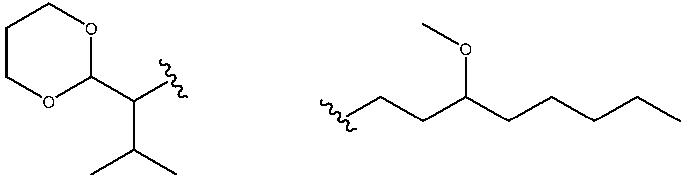 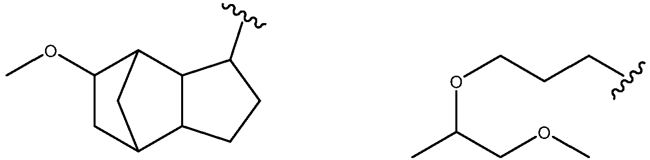 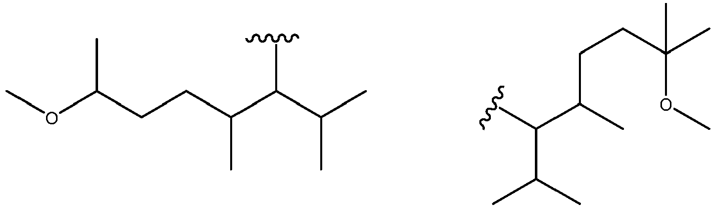 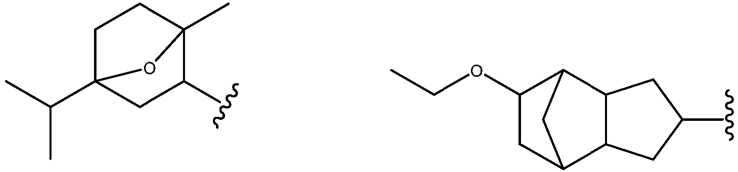 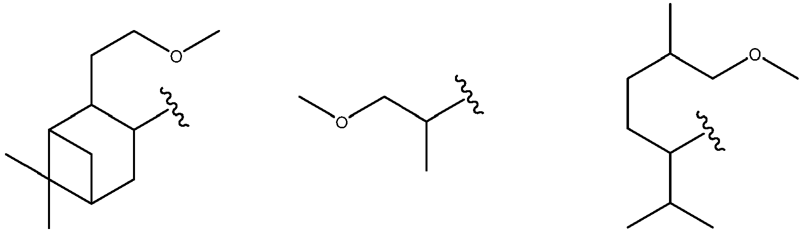 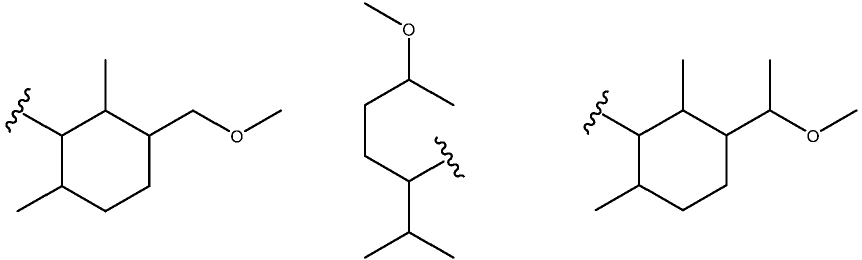 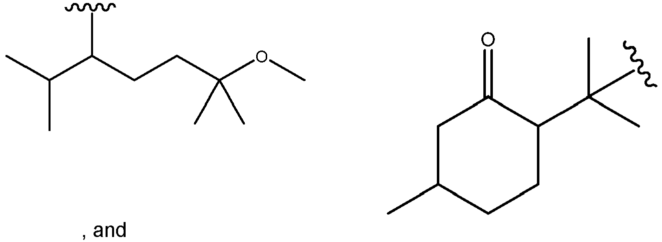
wherein R³ is selected from the group consisting of hydrogen and C₁ to C₁₀ linear or cyclic alkyl, alkenyl or alkadienyl residues, preferably wherein the residues additionally carry one, two or more C₁ to C₄ linear, cyclic or branched alkyl, alkenyl or alkadienyl substituents,
or
(ii) wherein R', R² and R³ are independently selected from the groups as defined in (i), respectively, and R and R¹ are bonded together to form a saturated or unsaturated ring system comprising 5 to 10 carbon atoms, preferably wherein the ring system additionally carries one, two or more C₁ to C₅ linear, branched or cyclic alkyl, alkenyl or alkadienyl substituents,
or
(iii) wherein R', R¹ and R² are independently selected from the groups as defined in (i), respectively, and R and R³ are bonded together to form a saturated or unsaturated, mono or bicyclic, ring system comprising 5 to 10 carbon atoms and optionally 1 or 2 ether functional groups, preferably wherein the ring system additionally carries one, two or more C₁ to C₅ linear, branched or cyclic alkyl, alkenyl or alkadienyl substituents.

2. Compound of formula (I) according to claim 1, wherein R is selected from the group consisting of

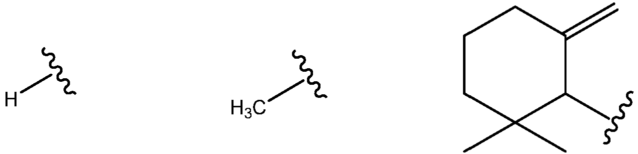

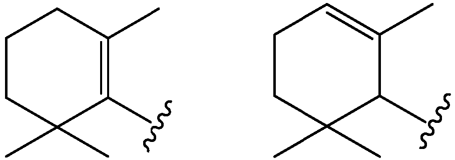

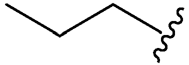

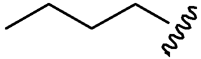

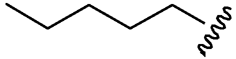

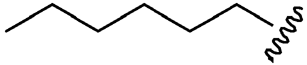

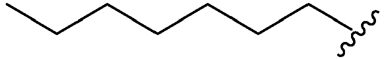

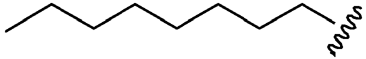

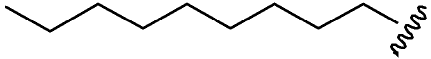

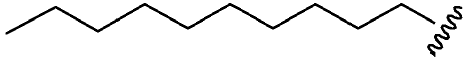

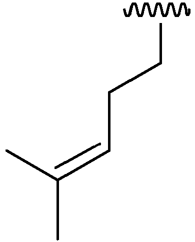

3. Compound of formula (I) according to claim 1 or 2, wherein R' is selected from the group consisting of

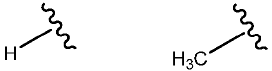

4. Compound of formula (I) according to any of the preceding claims, wherein R¹ is selected from the group consisting of

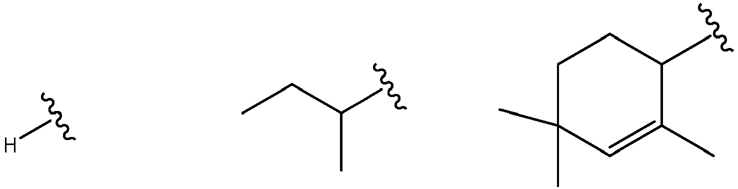

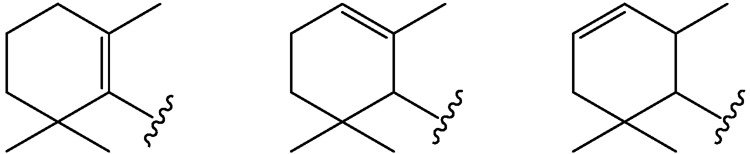

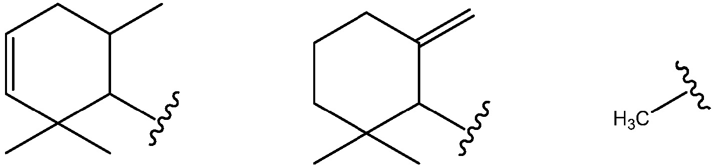

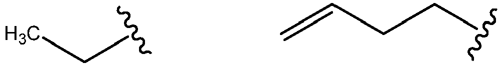

5. Compound of formula (I) according to any of the preceding claims, wherein the compound of formula (I) is selected from the group consisting of

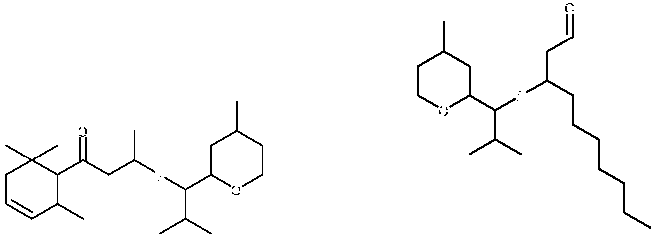

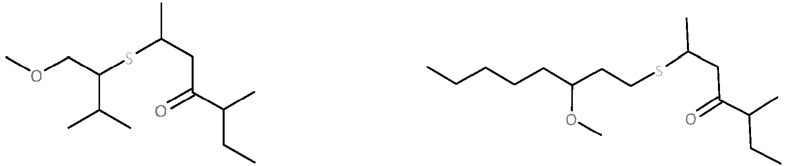

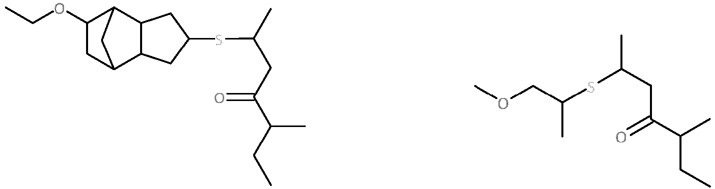

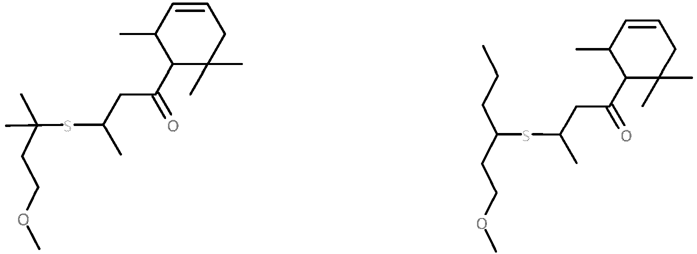

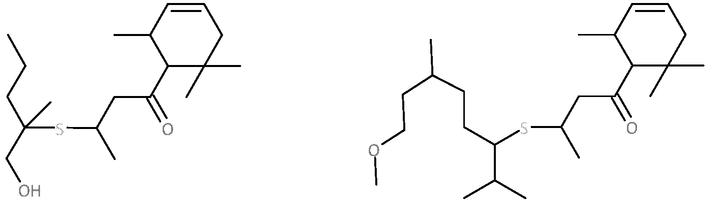

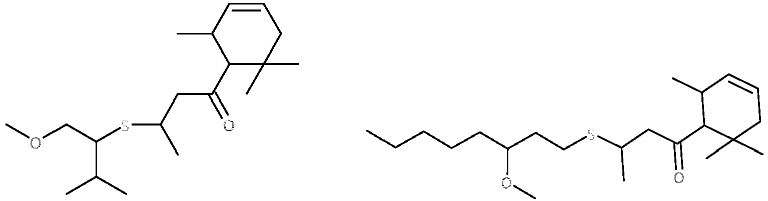

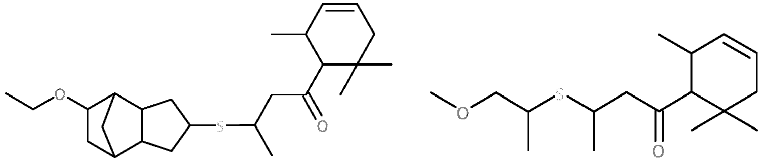

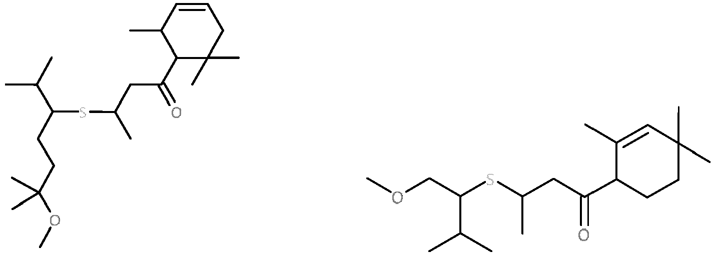

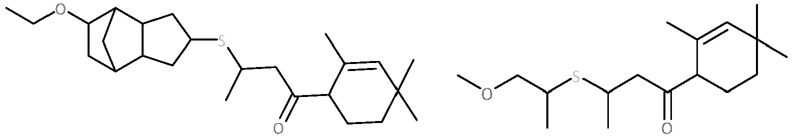

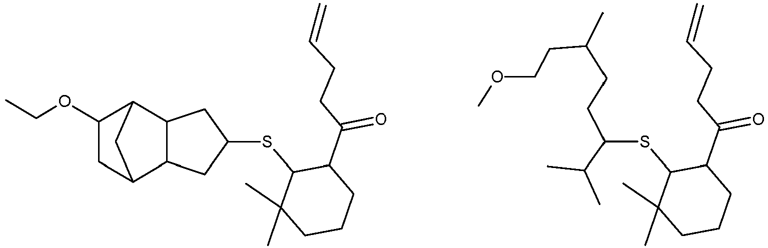

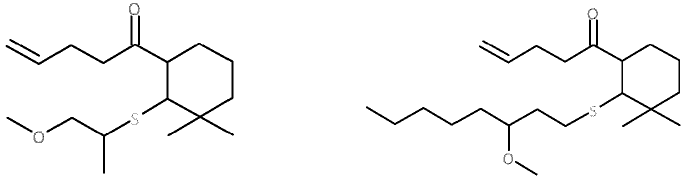

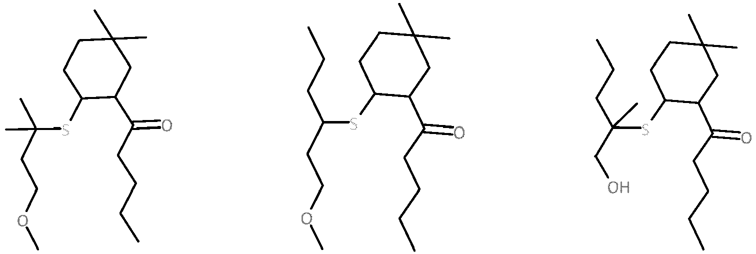

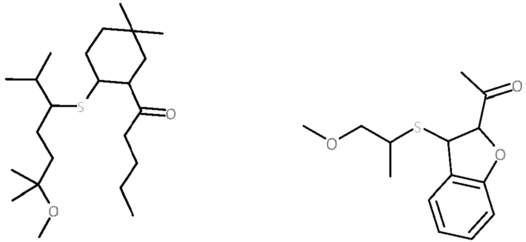

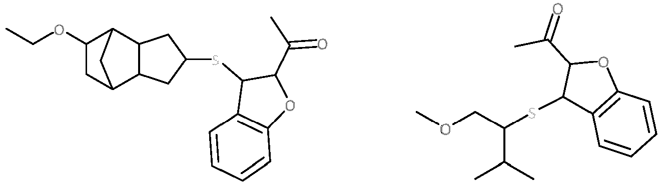

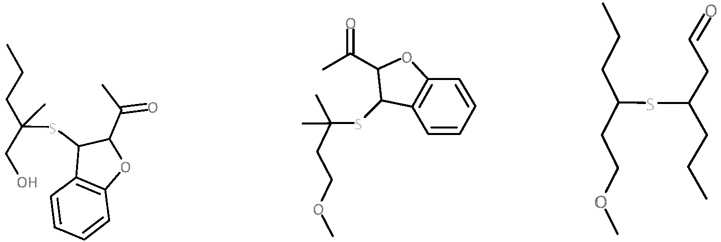

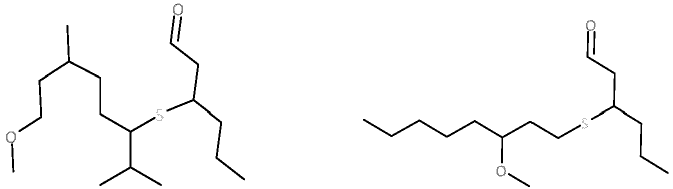

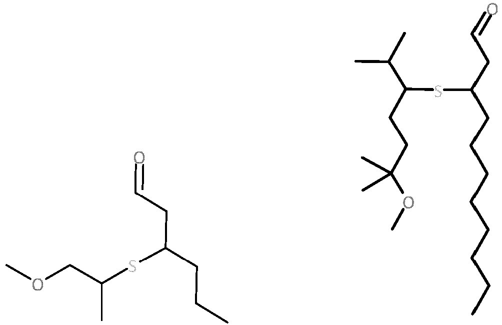

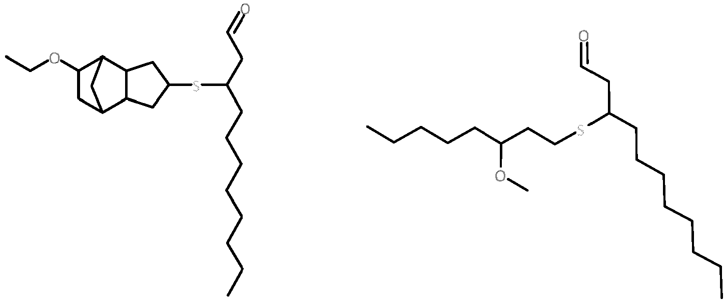

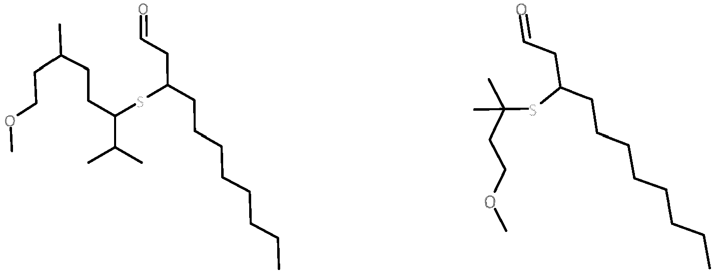

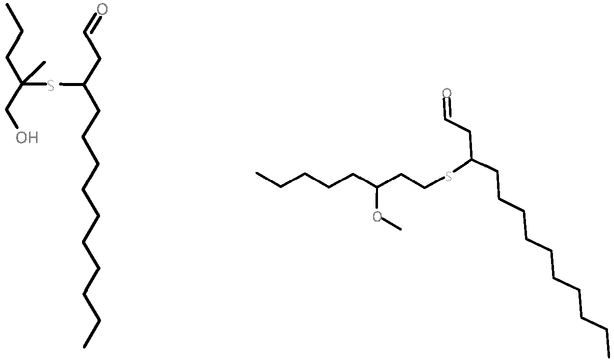

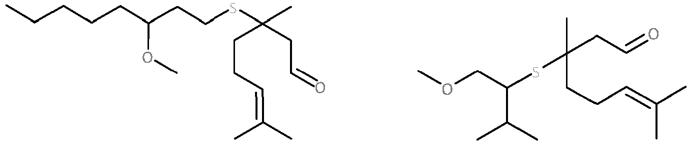

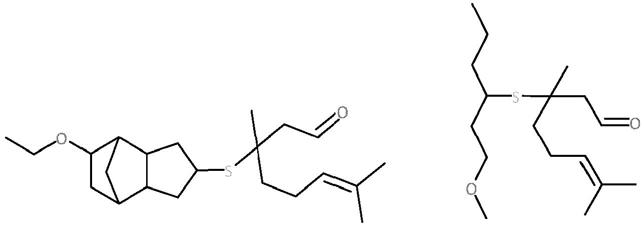

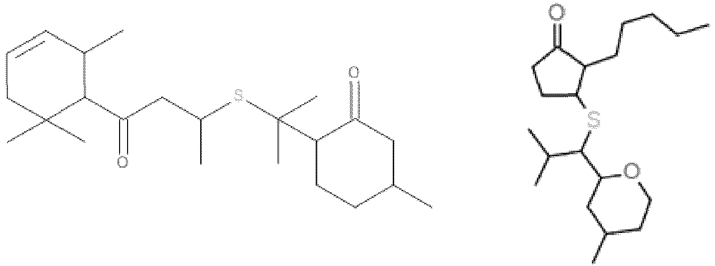

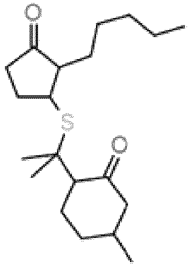

6. Fragrance substance mixture, preferably perfume oil, comprising or consisting of the following components:
(i) one or more compound(s) of formula (I) according to any of the preceding claims, and
(ii) one or more further fragrance substances.

7. Method for producing a fragrance substance mixture, preferably a perfume oil, according to claim 6, comprising or consisting of the following step:
Mixing of one or more compound(s) of formula (I) according to any of the claims 1 to 5 with one or more further fragrance substances.

8. Perfumed product comprising one or more compound(s) of formula (I) according to any of the claims 1 to 5 or a fragrance substance mixture, preferably a perfume oil, according to claim 6, preferably in a sensorially effective amount.

9. Perfumed product according to claim 8, wherein the product is selected from the group consisting of perfume extracts, eau de parfums, eau de toilettes, aftershaves, eau de colognes, pre-shave products, splash colognes, perfumed refreshing cloths, acidic, alkaline and neutral detergents, textile fresheners, ironing aids, liquid detergents, powdered detergents, laundry pre-treatment agents, fabric softeners, washing soaps, washing tablets, disinfectants, surface disinfectants, air fresheners, aerosol sprays, waxes and polishes, personal care products, hand creams and lotions, foot creams and lotions, depilatory creams and lotions, aftershave creams and lotions, tanning creams and lotions, hair care products, deodorants and antiperspirants, decorative cosmetic products, candles, lamp oils, incense sticks, insecticides, repellents, and fuels.

10. Method for producing a perfumed product, preferably a perfumed product according to claim 8 or 9, comprising or consisting of the following steps:
(i) Providing one or more compound(s) of formula (I) according to any of the claims 1 to 5 or a fragrance substance mixture, preferably a perfume oil, according to claim 6,
(ii) providing one or more further components of the perfumed product to be produced, and
(iii) contacting or mixing the further components provided in step (ii) with a sensorially effective amount of the components provided in step (i).

11. Use of a compound of formula (I) according to any of the claims 1 to 5 as a precursor for the release of two or more different pleasantly smelling fragrance substances, preferably wherein the two or more different fragrance substances are released and/or sensorially perceived at the same or different, preferably different, point(s) in time.

12. Method for the release of two or more different pleasantly smelling fragrance substances comprising or consisting of the following steps:
(a) Providing a compound of formula (I) according to any of the claims 1 to 5 or a fragrance substance mixture according to claim 6 or a perfumed product according to claim 8 or 9,
(b) optionally, applying or introducing the compound of formula (I) or the fragrance substance mixture or the perfumed product of step (a) to hair or skin or textile fibres or to a surface to be perfumed, or into ambient air to be perfumed, preferably in a sensorially effective amount, and
(c) releasing two or more different pleasantly smelling fragrance substances from the compound of formula (I), preferably wherein the two or more different pleasantly smelling fragrance substances are released and/or sensorially perceived at the same or different, preferably different, point(s) in time.

13. Thiol selected from the group consisting of

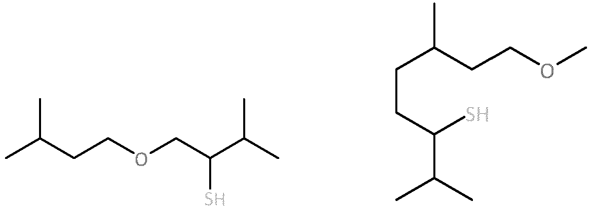

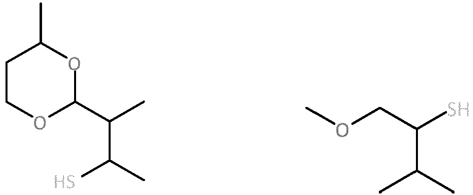

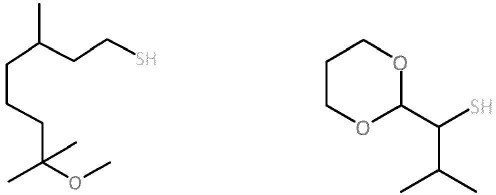

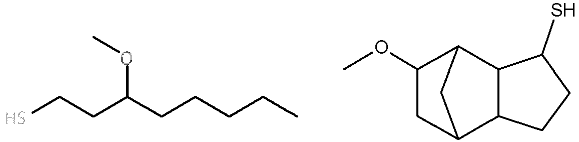

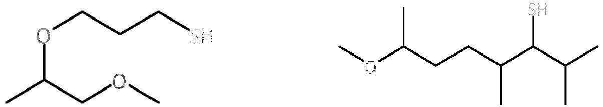

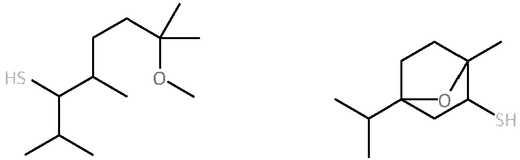

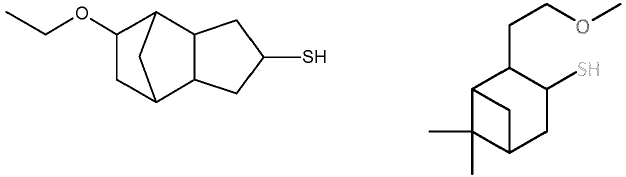

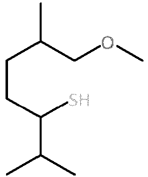

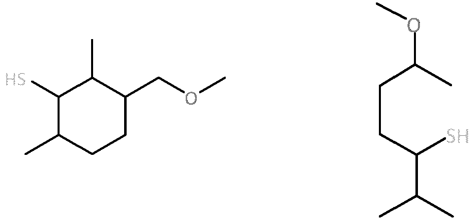

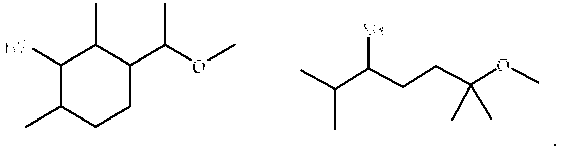

## Patentansprüche

1. Verbindung der Formel (I) 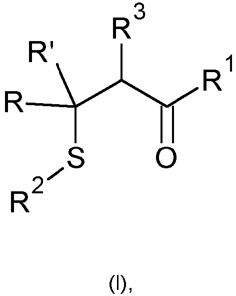
(i) wobei R ausgewählt ist aus der Gruppe bestehend aus Wasserstoff und linearen oder zyklischen C₁ bis C₁₀ Alkyl-, Alkenyl- oder Alkadienylresten, wobei die Reste vorzugsweise zusätzlich einen, zwei oder mehrere lineare, zyklische oder verzweigte C₁ bis C₄ Alkyl-, Alkenyl- oder Alkadienylsubstituenten tragen, und
wobei R' ausgewählt ist aus der Gruppe bestehend aus Wasserstoff und linearen oder zyklischen C₁ bis C₁₀ Alkyl-, Alkenyl- oder Alkadienylresten, wobei die Reste vorzugsweise zusätzlich einen, zwei oder mehrere lineare, zyklische oder verzweigte C₁ bis C₄ Alkyl-, Alkenyl- oder Alkadienylsubstituenten tragen, und
wobei R¹ ausgewählt ist aus der Gruppe bestehend aus Wasserstoff und linearen oder zyklischen C₁ bis C₁₀ Alkyl-, Alkenyl- oder Alkadienylresten, wobei die Reste vorzugsweise zusätzlich einen, zwei oder mehrere lineare, zyklische oder verzweigte C₁ bis C₄ Alkyl-, Alkenyl- oder Alkadienylsubstituenten tragen, und
wobei R² ausgewählt ist aus der Gruppe bestehend aus 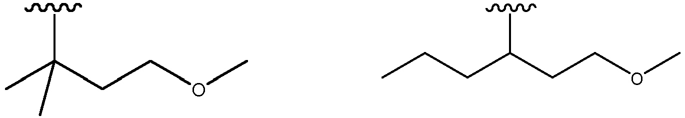 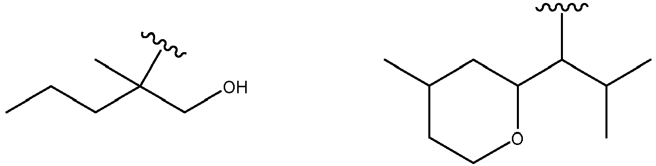 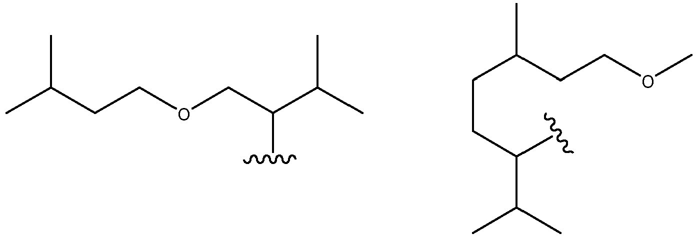 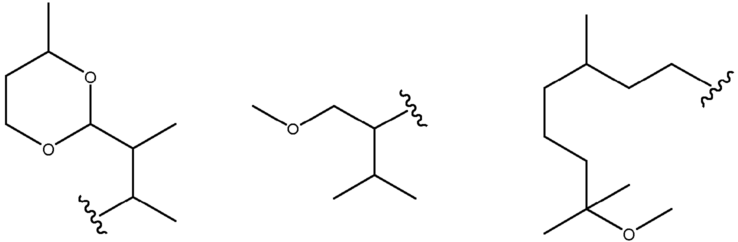 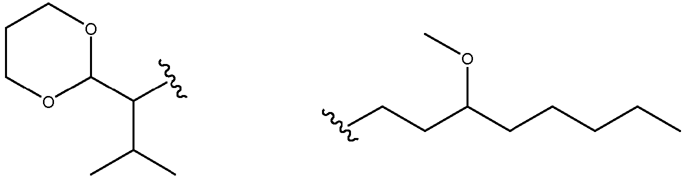 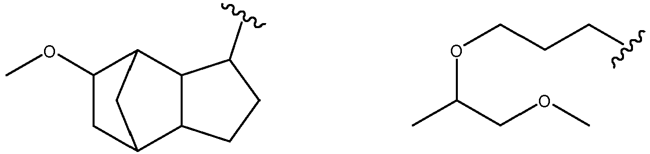 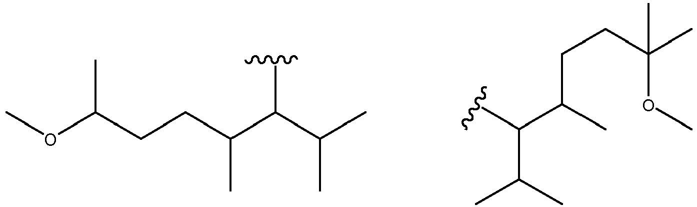 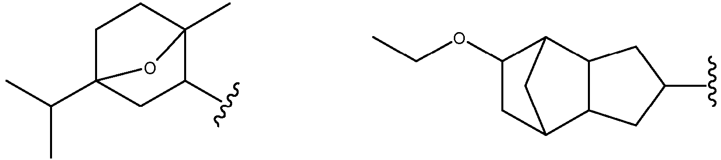 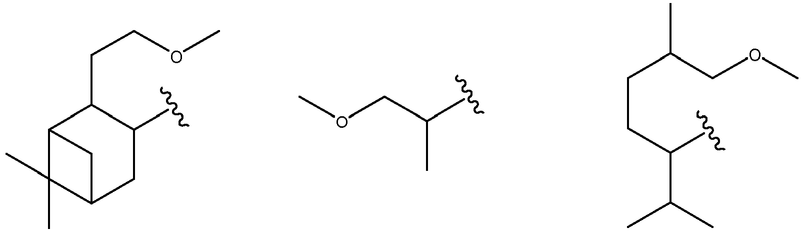 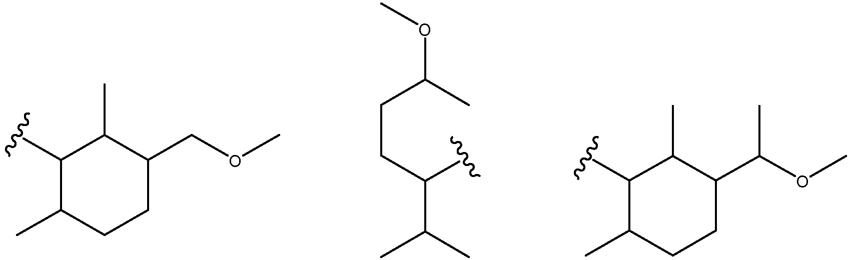 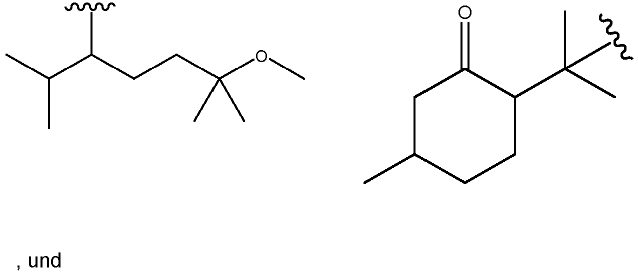 wobei R³ ausgewählt ist aus der Gruppe bestehend aus Wasserstoff und linearen oder zyklischen C₁ bis C₁₀ Alkyl-, Alkenyl- oder Alkadienylresten, wobei die Reste vorzugsweise zusätzlich einen, zwei oder mehrere lineare, zyklische oder verzweigte C₁ bis C₄ Alkyl-, Alkenyl- oder Alkadienylsubstituenten tragen,
oder
(ii) wobei R', R² und R³ unabhängig voneinander aus den in (i) definierten Gruppen ausgewählt sind und R und R¹ miteinander verbunden sind, um ein gesättigtes oder ungesättigtes Ringsystem mit 5 bis 10 Kohlenstoffatomen zu bilden, wobei das Ringsystem vorzugsweise zusätzlich einen, zwei oder mehrere lineare, verzweigte oder zyklische C₁ bis C₅ Alkyl-, Alkenyl- oder Alkadienylsubstituenten trägt,
oder
(iii) wobei R', R¹ und R² unabhängig voneinander aus den in (i) definierten Gruppen ausgewählt sind und R und R³ miteinander verbunden sind, um ein gesättigtes oder ungesättigtes, mono- oder bizyklisches Ringsystem zu bilden, das 5 bis 10 Kohlenstoffatome und gegebenenfalls 1 oder 2 etherfunktionelle Gruppen umfasst, wobei das Ringsystem vorzugsweise zusätzlich einen, zwei oder mehrere lineare, verzweigte oder zyklische C₁ bis C₅ Alkyl-, Alkenyl- oder Alkadienylsubstituenten trägt.

2. Verbindung der Formel (I) nach Anspruch 1, wobei R ausgewählt ist aus der Gruppe bestehend aus

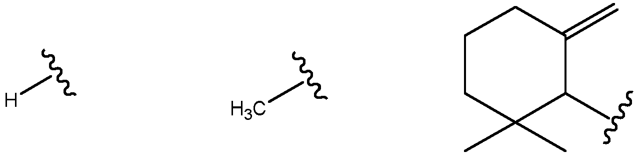

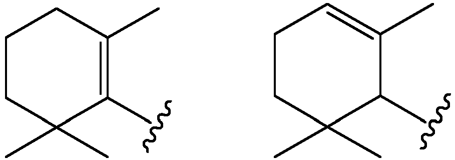

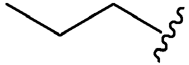

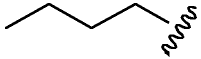

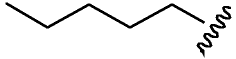

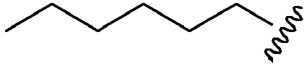

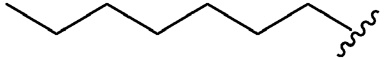

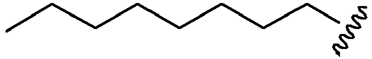

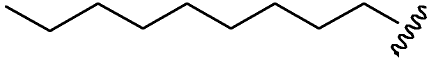

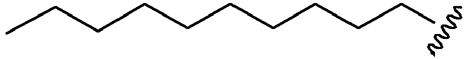

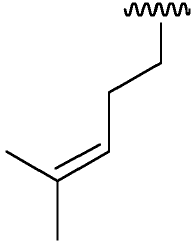

3. Verbindung der Formel (I) nach Anspruch 1 oder 2, wobei R' ausgewählt ist aus der Gruppe bestehend aus

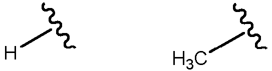

4. Verbindung der Formel (I) nach einem der vorangehenden Ansprüche, wobei R¹ ausgewählt ist aus der Gruppe bestehend aus

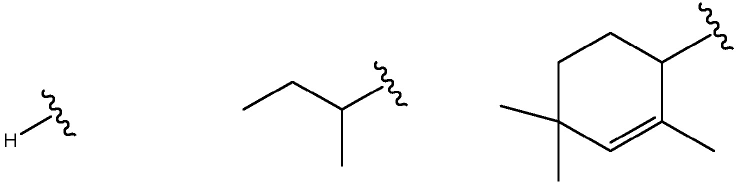

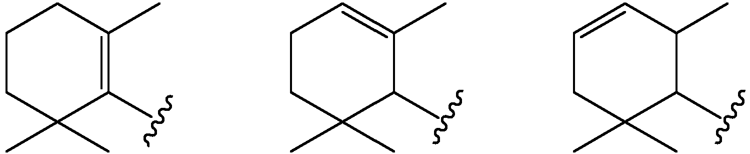

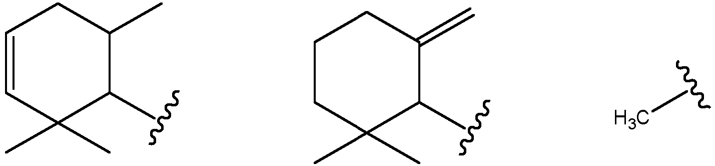

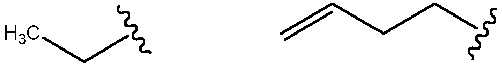

5. Verbindung der Formel (I) nach einem der vorangehenden Ansprüche, wobei die Verbindung der Formel (I) ausgewählt ist aus der Gruppe bestehend aus

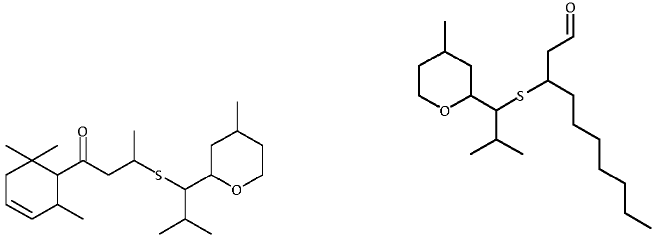

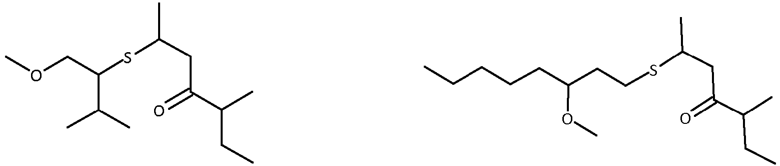

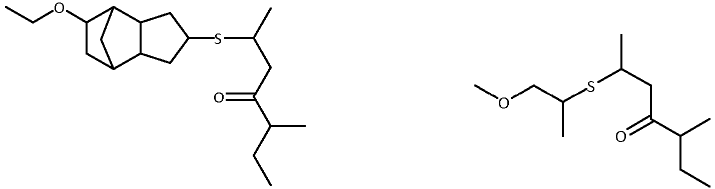

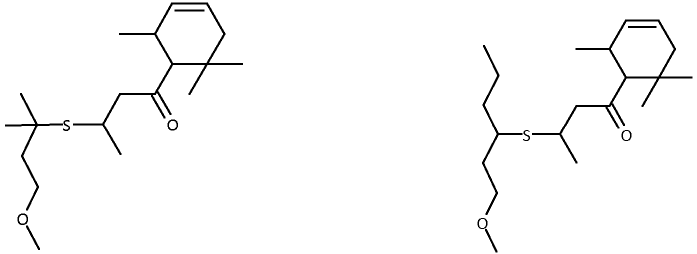

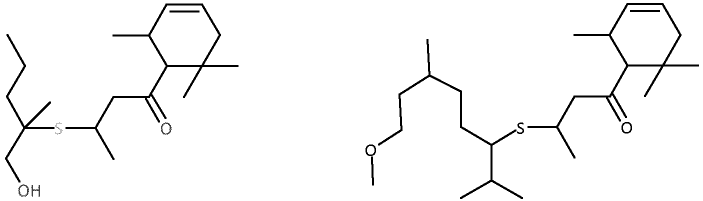

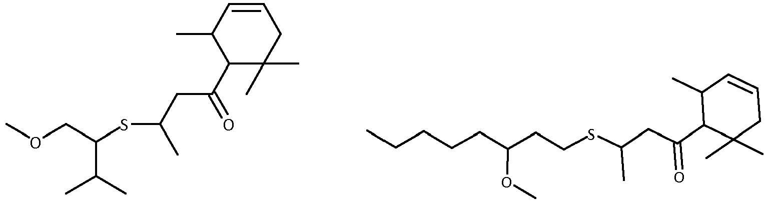

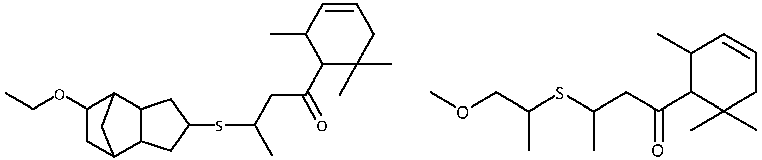

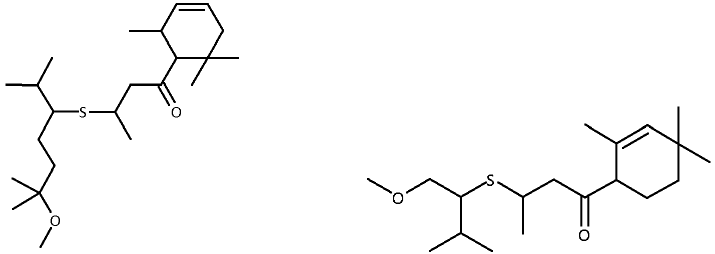

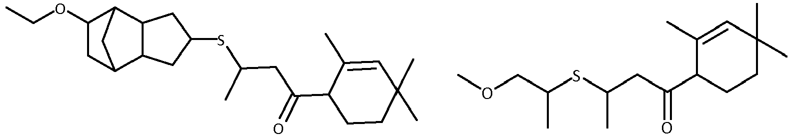

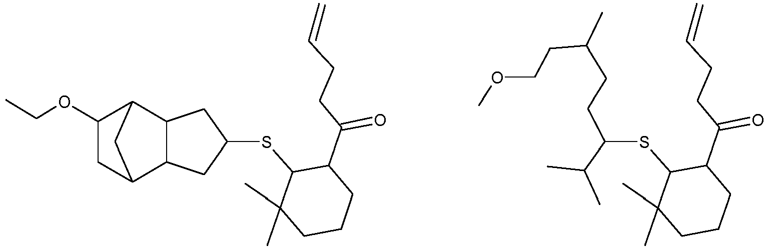

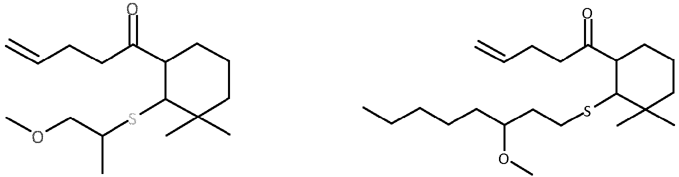

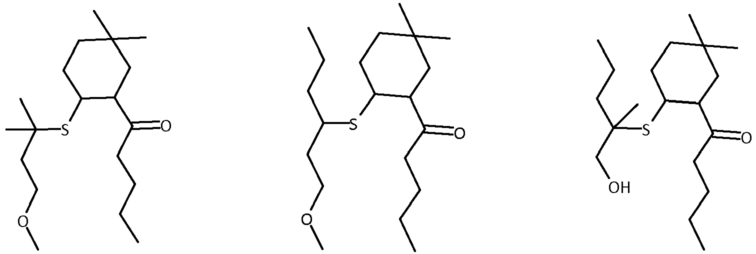

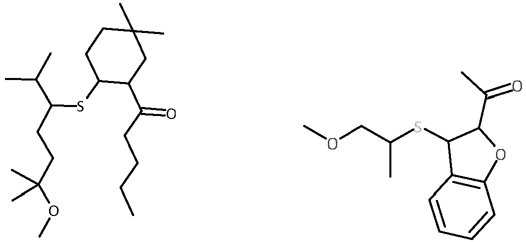

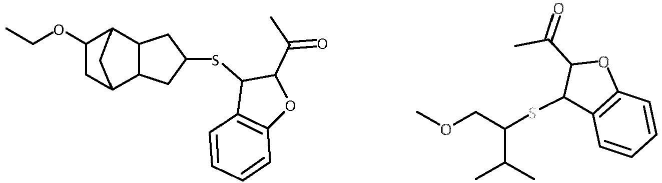

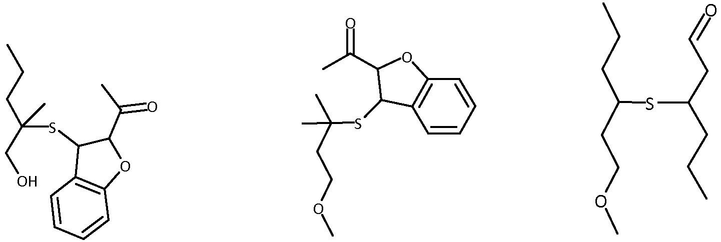

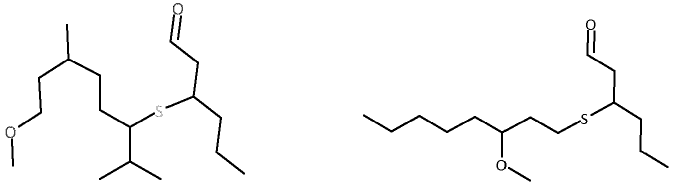

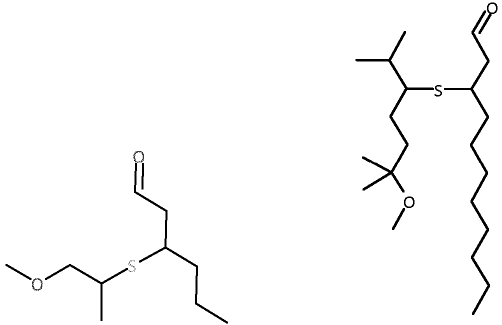

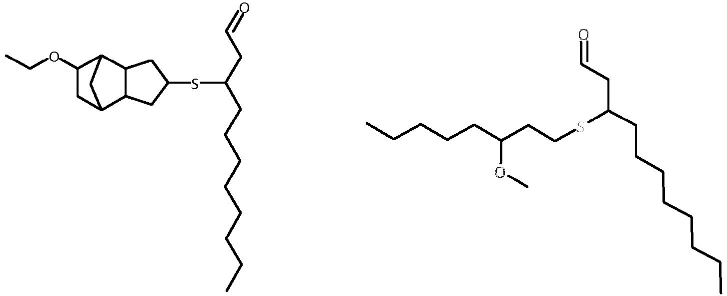

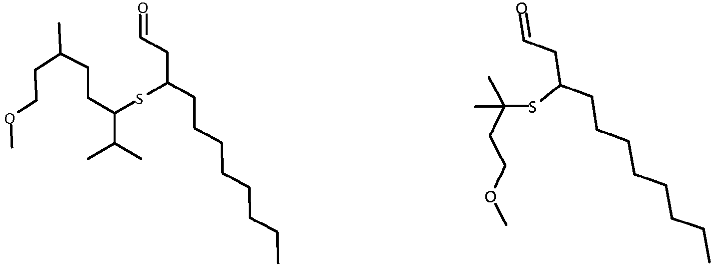

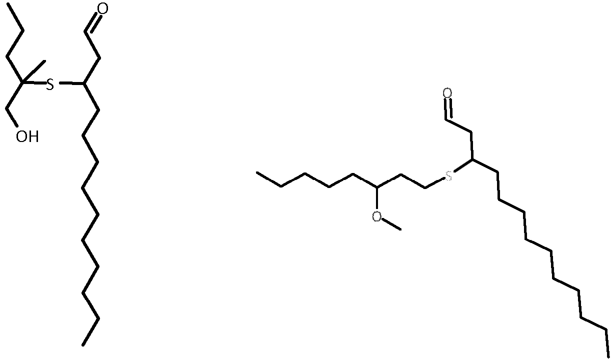

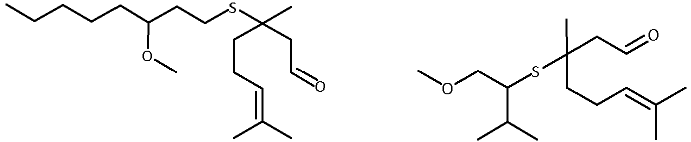

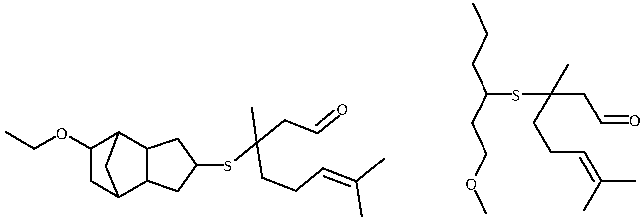

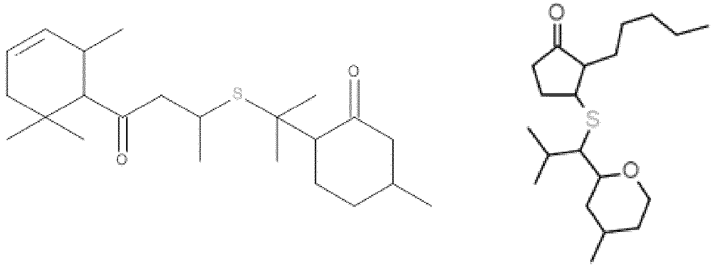

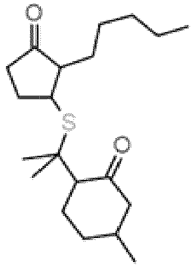

6. Riechstoffmischung, vorzugsweise Parfümöl, umfassend oder bestehend aus den folgenden Bestandteilen:
(i) eine oder mehrere Verbindung(en) der Formel (I) nach einem der vorangehenden Ansprüche, und
(ii) einen oder mehrere weitere Riechstoffe.

7. Verfahren zur Herstellung einer Riechstoffmischung, vorzugsweise eines Parfümöls, nach Anspruch 6, umfassend oder bestehend aus dem folgenden Schritt:
Mischen einer oder mehrerer Verbindung(en) der Formel (I) nach einem der Ansprüche 1 bis 5 mit einem oder mehreren weiteren Riechstoffen.

8. Parfümiertes Produkt umfassend eine oder mehrere Verbindung(en) der Formel (I) nach einem der Ansprüche 1 bis 5 oder eine Riechstoffmischung, vorzugsweise ein Parfümöl, nach Anspruch 6, vorzugsweise in einer sensorisch wirksamen Menge.

9. Parfümiertes Produkt nach Anspruch 8, wobei das Produkt ausgewählt ist aus der Gruppe bestehend aus Parfümextrakten, Eau de Parfums, Eau de Toilettes, Aftershaves, Eau de Colognes, Pre-Shave-Produkten, Splash Colognes, parfümierten Erfrischungstüchern, sauren, alkalischen und neutralen Waschmitteln, Textilerfrischern, Bügelhilfen, Flüssigwaschmitteln, pulverförmigen Waschmitteln, Wäschevorbehandlungsmitteln, Weichspülern, Waschseifen, Waschtabletten, Desinfektionsmittel, Flächendesinfektionsmittel, Lufterfrischer, Aerosolsprays, Wachse und Polituren, Körperpflegemittel, Handcremes und -lotionen, Fußcremes und -lotionen, Enthaarungscremes und -lotionen, Aftershave-Cremes und -lotionen, Bräunungscremes und -lotionen, Haarpflegemittel, Deodorants und Antitranspirantien, dekorative kosmetische Produkte, Kerzen, Lampenöle, Räucherstäbchen, Insektizide, Repellentien und Brennstoffe.

10. Verfahren zur Herstellung eines parfümierten Produkts, vorzugsweise eines parfümierten Produkts nach Anspruch 8 oder 9, umfassend oder bestehend aus den folgenden Schritten:
(i) Bereitstellen einer oder mehrerer Verbindung(en) der Formel (I) nach einem der Ansprüche 1 bis 5 oder einer Riechstoffmischung, vorzugsweise eines Parfümöls, nach Anspruch 6,
(ii) Bereitstellen einer oder mehrerer weiterer Komponenten des herzustellenden parfümierten Produkts, und
(iii) Inkontaktbringen oder Mischen der in Schritt (ii) bereitgestellten weiteren Komponenten mit einer sensorisch wirksamen Menge der in Schritt (i) bereitgestellten Komponenten.

11. Verwendung einer Verbindung der Formel (I) nach einem der Ansprüche 1 bis 5 als Vorläufer für die Freisetzung von zwei oder mehr unterschiedlichen, angenehm riechenden Riechstoffen, wobei die zwei oder mehr unterschiedlichen Riechstoffe vorzugsweise zum gleichen oder zu unterschiedlichen, vorzugsweise unterschiedlichen, Zeitpunkt(en) freigesetzt und/oder sensorisch wahrgenommen werden.

12. Verfahren zur Freisetzung von zwei oder mehr unterschiedlichen, angenehm riechenden Riechstoffen, umfassend oder bestehend aus den folgenden Schritten:
(a) Bereitstellen einer Verbindung der Formel (I) nach einem der Ansprüche 1 bis 5 oder einer Riechstoffmischung nach Anspruch 6 oder eines parfümierten Produkts nach Anspruch 8 oder 9,
(b) gegebenenfalls Auftragen oder Einbringen der Verbindung der Formel (I) oder der Riechstoffmischung oder des parfümierten Produkts aus Schritt (a) auf Haare oder Haut oder Textilfasern oder auf eine zu parfümierende Oberfläche oder in zu parfümierende Umgebungsluft, vorzugsweise in einer sensorisch wirksamen Menge, und
(c) Freisetzen von zwei oder mehr unterschiedlichen, angenehm riechenden Riechstoffen aus der Verbindung der Formel (I), wobei die zwei oder mehr unterschiedlichen, angenehm riechenden Riechstoffe vorzugsweise zum gleichen oder zu unterschiedlichen, vorzugsweise zu unterschiedlichen, Zeitpunkt(en) freigesetzt und/oder sensorisch wahrgenommen werden.

13. Thiol, ausgewählt aus der Gruppe bestehend aus

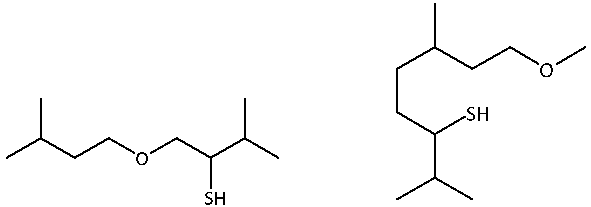

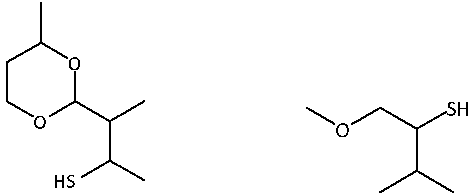

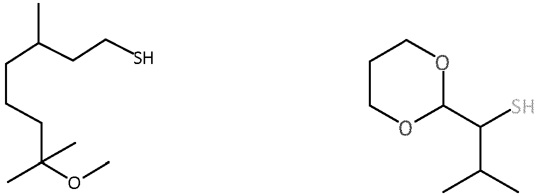

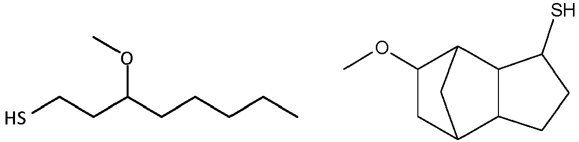

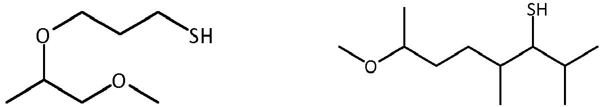

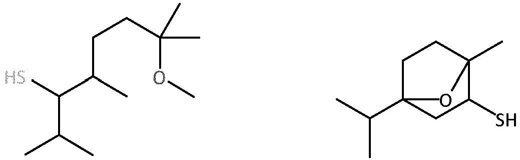

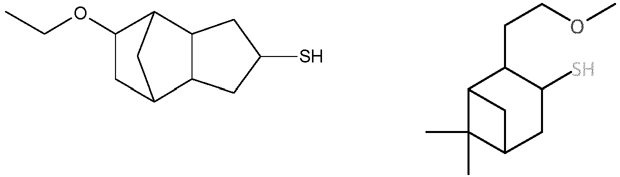

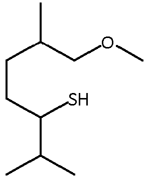

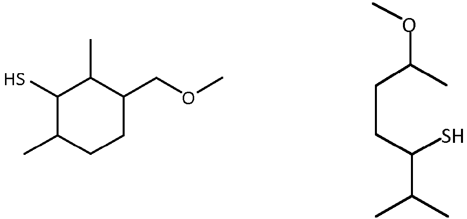

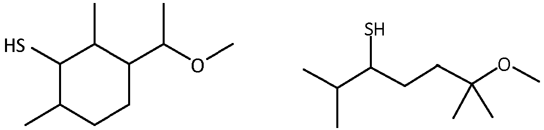

## Revendications

1. Composé de formule (I) 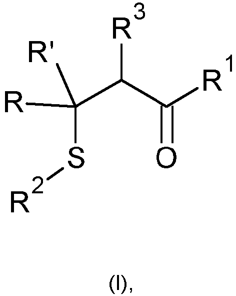
(i) dans laquelle R est choisi dans le groupe constitué par l'hydrogène et des résidus alkyle, alcényle ou alcadiényle linéaires ou cycliques en C₄ à C₁₀ , de préférence dans laquelle les résidus portent en outre un, deux ou plusieurs substituants alkyle, alcényle ou alcadiényle linéaires, cycliques ou ramifiés en C₁ à C₄, et
dans laquelle R' est choisi dans le groupe constitué par l'hydrogène et des résidus alkyle, alcényle ou alcadiényle linéaires ou cycliques en C₄ à C₁₀ , de préférence dans laquelle les résidus portent en outre un, deux ou plusieurs substituants alkyle, alcényle ou alcadiényle linéaires, cycliques ou ramifiés en C₁ à C₄, et
dans laquelle R¹ est choisi dans le groupe constitué par l'hydrogène et des résidus alkyle, alcényle ou alcadiényle linéaires ou cycliques en C₄ à C₁₀, de préférence dans laquelle les résidus portent en outre un, deux ou plusieurs substituants alkyle, alcényle ou alcadiényle linéaires, cycliques ou ramifiés en C₁ à C₄, et
dans laquelle R² est choisi dans le groupe constitué par 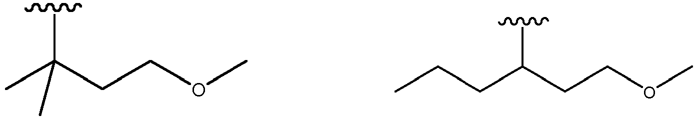 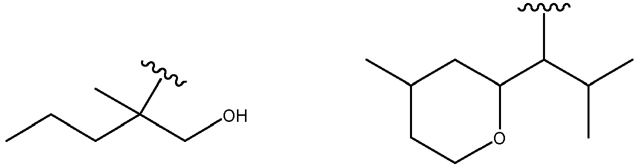 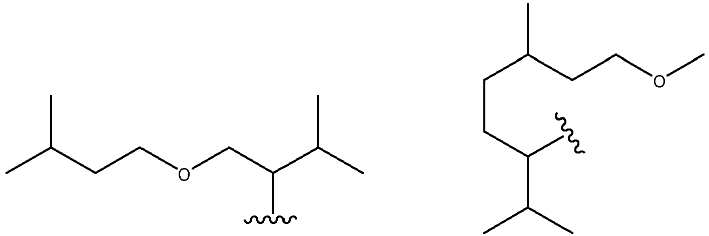 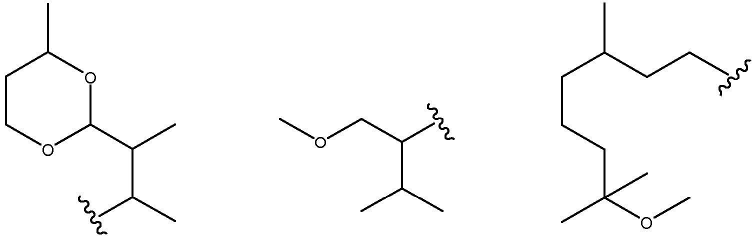 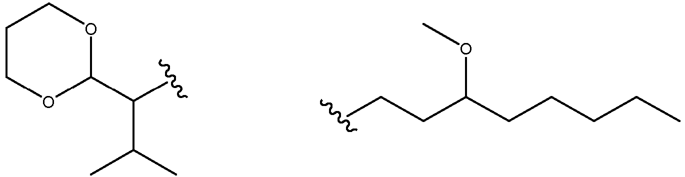 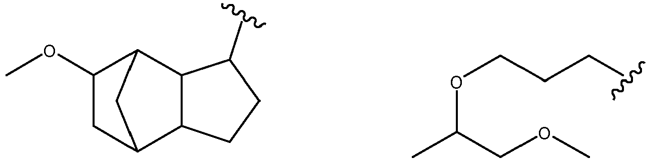 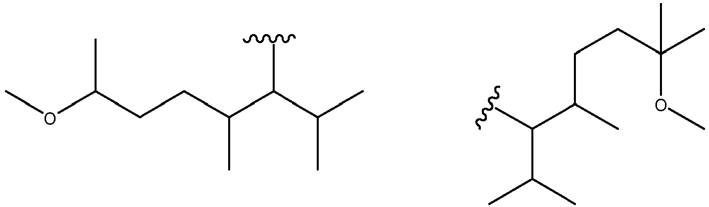 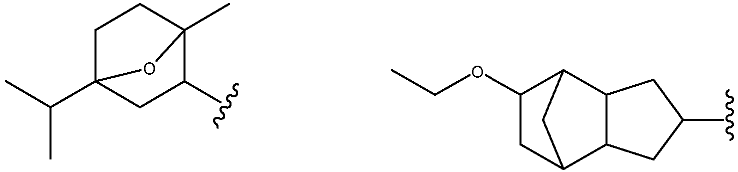 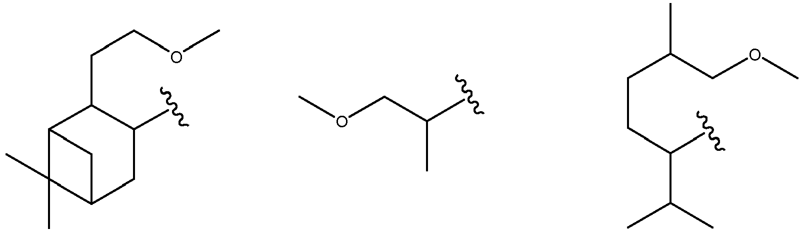 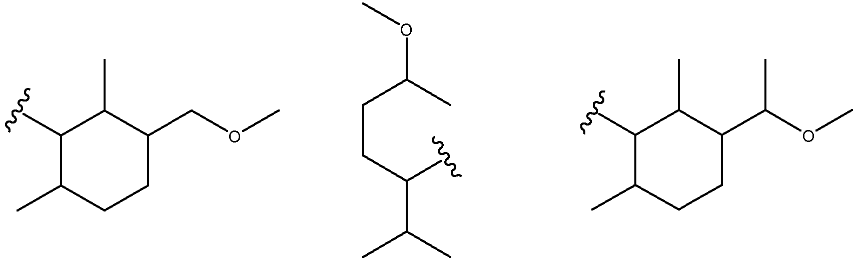 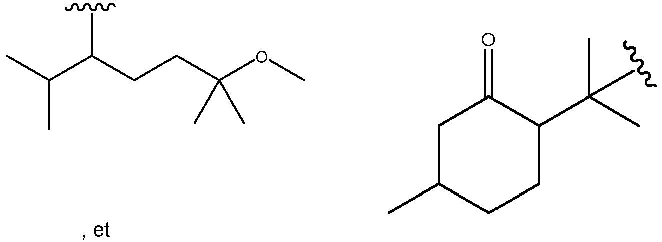 dans laquelle R³ est choisi dans le groupe constitué par l'hydrogène et des résidus alkyle, alcényle ou alcadiényle linéaires ou cycliques en C₄ à C₁₀ , de préférence dans laquelle les résidus portent en outre un, deux ou plusieurs substituants alkyle, alcényle ou alcadiényle linéaires, cycliques ou ramifiés en C₁ à C₄,
ou
(ii) dans laquelle R', R² et R³ sont indépendamment choisis dans les groupes tels que définis dans (i), respectivement, et R et R¹ sont liés ensemble pour former un système cyclique saturé ou insaturé comprenant 5 à 10 atomes de carbone, de préférence dans lequel le système cyclique porte en outre un, deux ou plusieurs substituants alkyle, alcényle ou alcadiényle linéaires, ramifiés ou cycliques en C₁ à C₅,
ou
(iii) dans laquelle R', R¹ et R² sont indépendamment choisis dans les groupes tels que définis dans (i), respectivement, et R et R³ sont liés ensemble pour former un système cyclique saturé ou insaturé, mono ou bicyclique, comprenant 5 à 10 atomes de carbone et le cas échéant 1 ou 2 groupe(s) fonctionnel(s) éther, de préférence dans lequel le système cyclique porte en outre un, deux ou plusieurs substituants alkyle, alcényle ou alcadiényle linéaires, ramifiés ou cycliques en C₁ à C₅ .

2. Composé de formule (I) selon la revendication 1, dans lequel R est choisi dans le groupe constitué par

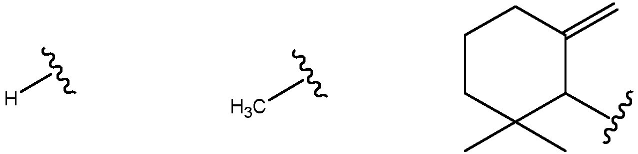

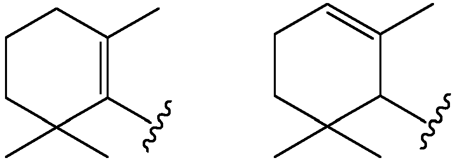

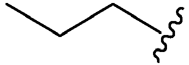

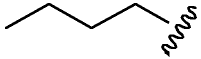

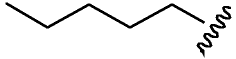

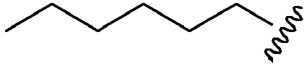

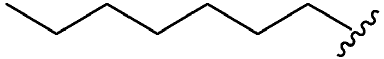

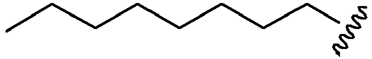

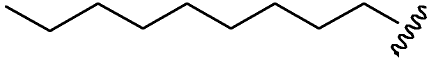

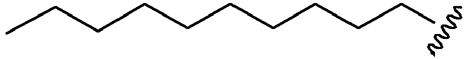

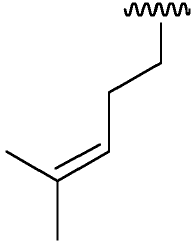

3. Composé de formule (I) selon la revendication 1 ou 2, dans lequel R' est choisi dans le groupe constitué par

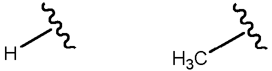

4. Composé de formule (I) selon l'une quelconque des revendications précédentes, dans lequel R¹ est choisi dans le groupe constitué par

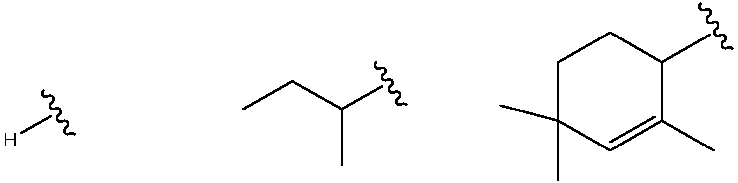

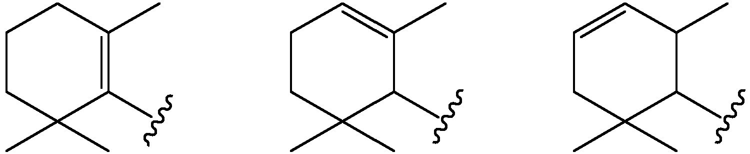

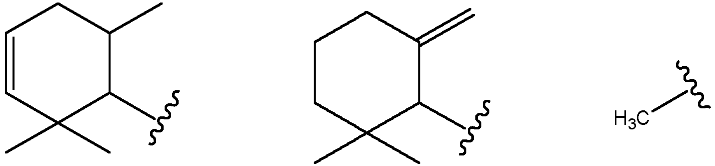

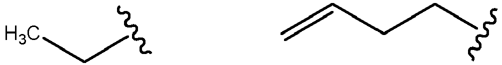

5. Composé de formule (I) selon l'une quelconque des revendications précédentes, dans lequel le composé de formule (I) est choisi dans le groupe constitué par

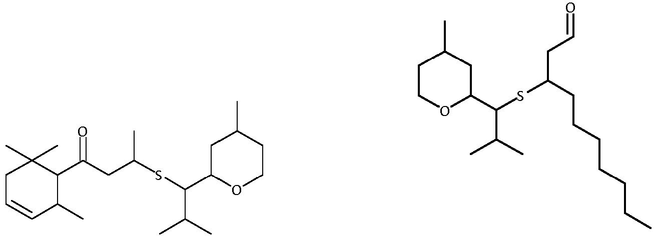

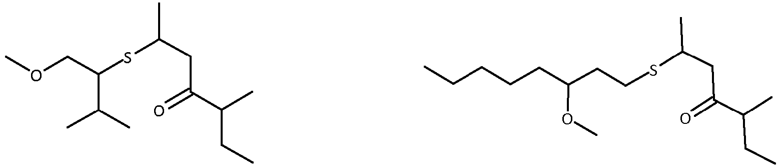

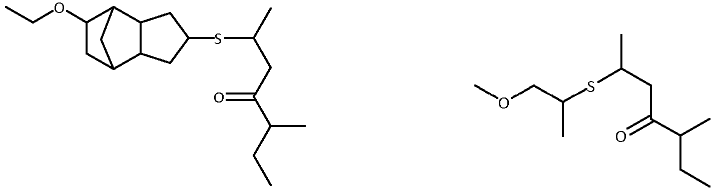

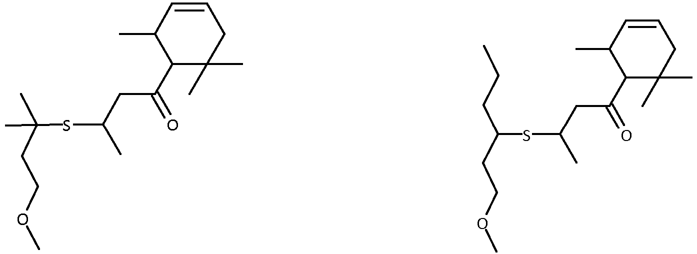

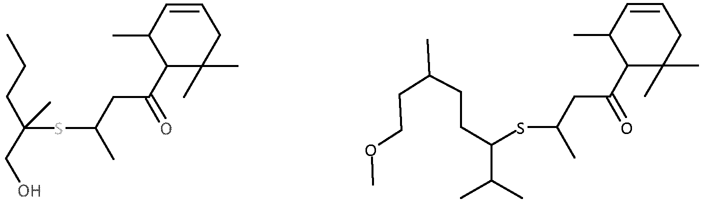

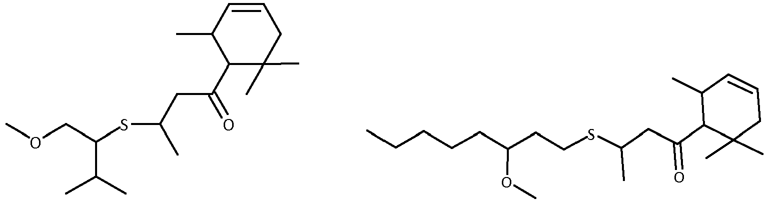

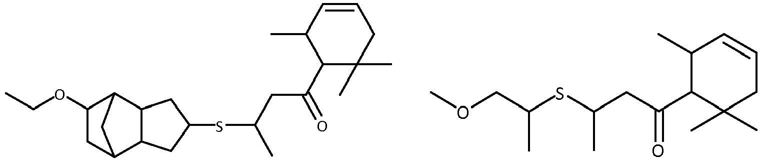

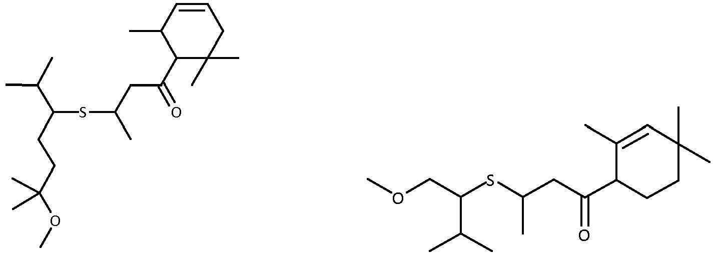

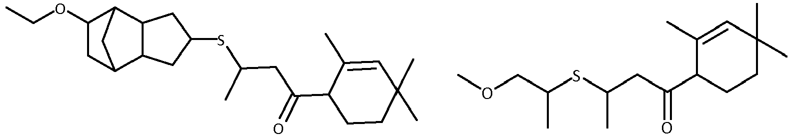

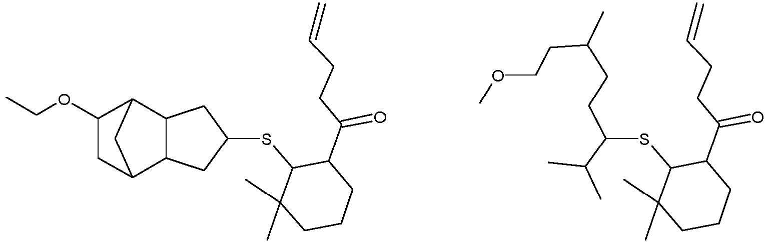

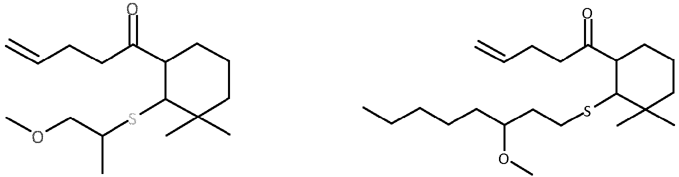

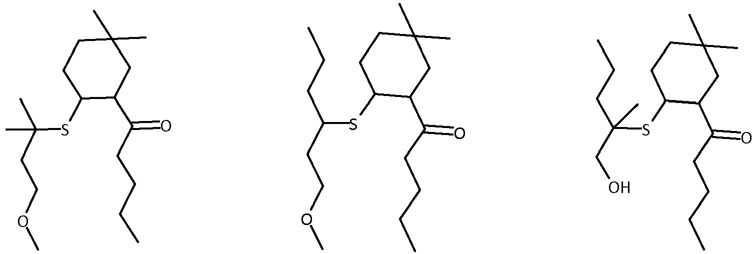

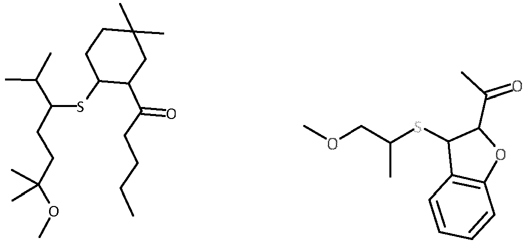

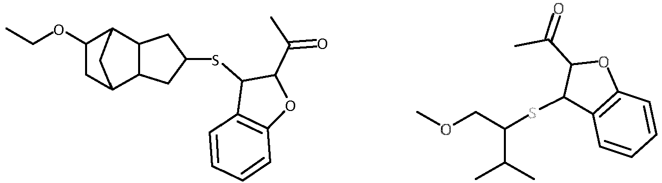

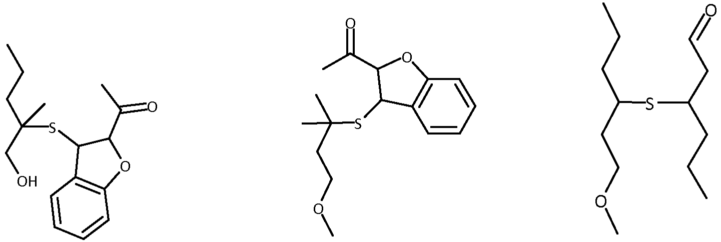

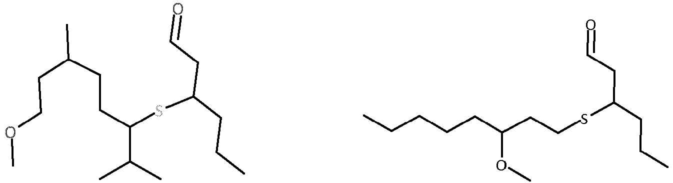

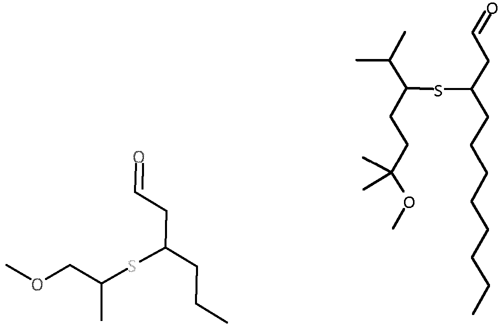

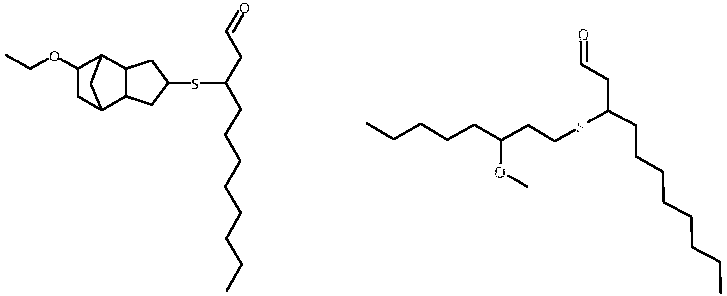

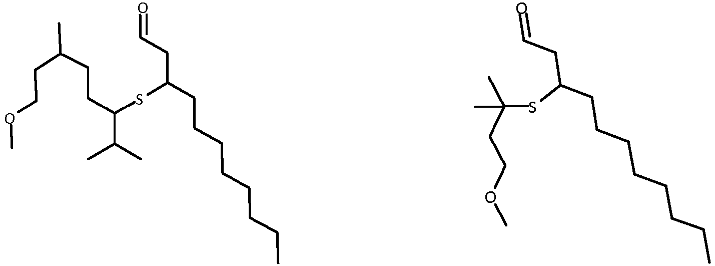

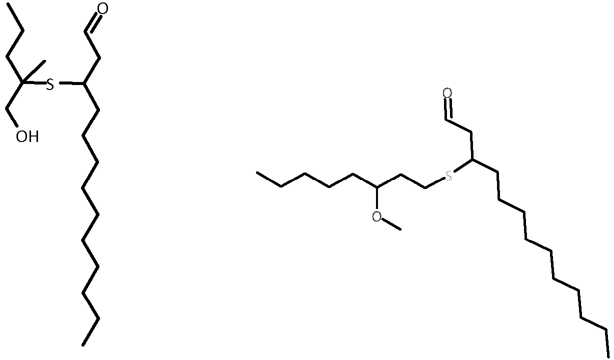

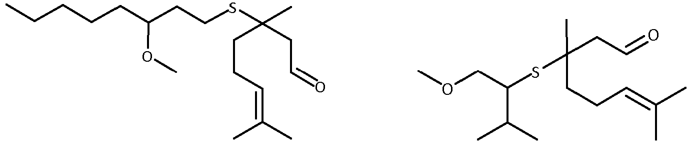

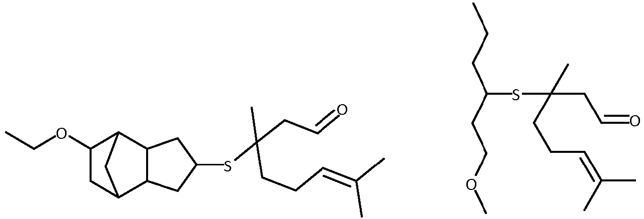

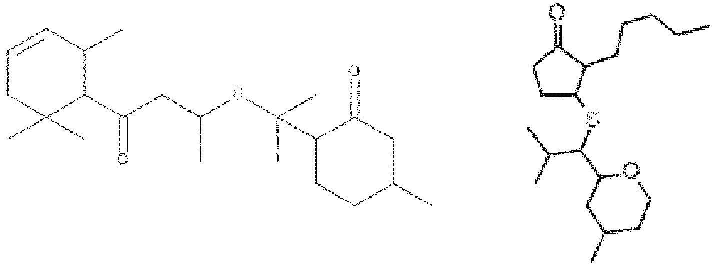

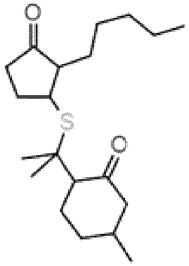

6. Mélange de substances parfumantes, de préférence huile de parfum, comprenant ou constitué des composants suivants:
(i) un ou plusieurs composé(s) de formule (I) selon l'une quelconque des revendications précédentes, et
(ii) une ou plusieurs autres substances parfumantes.

7. Procédé de production d'un mélange de substances parfumantes, de préférence une huile de parfum, selon la revendication 6, comprenant ou constitué de l'étape suivante:
mélanger un ou plusieurs composé(s) de formule (I) selon l'une quelconque des revendications 1 à 5, avec une ou plusieurs autres substances parfumantes.

8. Produit parfumé comprenant un ou plusieurs composé(s) de formule (I) selon l'une quelconque des revendications 1 à 5 ou un mélange de substances parfumantes, de préférence une huile de parfum, selon la revendication 6, de préférence en une quantité sensoriellement efficace.

9. Produit parfumé selon la revendication 8, dans lequel le produit est choisi dans le groupe constitué par les extraits de parfum, les eaux de parfum, les eaux de toilette, les après-rasages, les eaux de Cologne, les produits de pré-rasage, les eaux de Cologne splash, les lingettes rafraîchissantes parfumées, les détergents acides, alcalins et neutres, les assainisseurs de textiles, les aides au repassage, les détergents liquides, les détergents en poudre, les agents de prétraitement du linge, les assouplissants, les savons de lavage, les pastilles de lavage, les désinfectants, les désinfectants de surface, les assainisseurs d'air, les sprays aérosols, les cires et les produits de cirage, les produits de soins personnels, les crèmes et lotions pour les mains, les crèmes et lotions pour les pieds, les crèmes et lotions dépilatoires, les crèmes et lotions après-rasage, les crèmes et lotions de bronzage, les produits de soins capillaires, les déodorants et les antiperspirants, les produits cosmétiques décoratifs, les bougies, les huiles de lampe, les bâtons d'encens, les insecticides, les répulsifs et les carburants.

10. Procédé de production d'un produit parfumé, de préférence d'un produit parfumé selon la revendication 8 ou 9, comprenant ou consistant en les étapes suivantes:
(i) fournir un ou plusieurs composé(s) de formule (I) selon l'une quelconque des revendications 1 à 5 ou un mélange de substances parfumantes, de préférence une huile de parfum, selon la revendication 6,
(ii) fournir un ou plusieurs autres composant(s) du produit parfumé à produire, et
(iii) mettre en contact ou mélanger les autres composants fournis à l'étape (ii) avec une quantité sensoriellement efficace des composants fournis à l'étape (i).

11. Utilisation d'un composé de formule (I) selon l'une quelconque des revendications 1 à 5, comme précurseur pour la libération de deux ou plusieurs substances parfumantes différentes à odeur agréable, de préférence dans laquelle les deux ou plusieurs substances parfumantes différentes sont libérées et/ou perçues sensoriellement au même moment ou à des moments différents, de préférence différents.

12. Procédé de libération de deux ou plusieurs substances parfumantes différentes à odeur agréable, comprenant ou constitué des étapes suivantes:
(a) fournir un composé de formule (I) selon l'une quelconque des revendications 1 à 5 ou un mélange de substances parfumantes selon la revendication 6 ou un produit parfumé selon la revendication 8 ou 9,
(b) le cas échéant, appliquer ou introduire le composé de formule (I) ou le mélange de substances parfumantes ou le produit parfumé de l'étape (a) sur des cheveux ou la peau ou des fibres textiles ou sur une surface à parfumer, ou dans un air ambiant à parfumer, de préférence en une quantité sensoriellement efficace, et
(c) libérer deux ou plusieurs substances parfumantes différentes à odeur agréable à partir du composé de formule (I), de préférence dans lequel les deux ou plusieurs substances parfumantes différentes à odeur agréable sont libérées et/ou perçues sensoriellement au même moment ou à des moments différents, de préférence différents.

13. Thiol choisi dans le groupe constitué par:
